# EUROPEAN PATENT SPECIFICATION

(11) **EP 1 718 665 B1**
(45) Date of publication and mention of the grant of the patent: **10.04.2013**
(21) Application number: 05722892.6
(22) Date of filing: 11.02.2005
(51) Int. Cl.: A61K 38/00, C07K 19/00, C07K 14/575

(54) **HYBRID POLYPEPTIDES WITH SELECTABLE PROPERTIES**
HYBRIDPOLYPEPTIDE MIT AUSWÄHLBAREN EIGENSCHAFTEN
POLYPEPTIDES HYBRIDES PRESENTANT DES PROPRIETES POUVANT ETRE CHOISIES

(30) Priority: 11.02.2004 US 543407 P
(43) Date of publication of application: 08.11.2006
(60) Divisional application: 11176627.5
(73) Proprietor: Amylin Pharmaceuticals, LLC, San Diego, CA 92121 (US); AstraZeneca Pharmaceuticals LP, Wilmington, DE (US)
(72) Inventor: LEVY, Odile Esther, San Diego, California 92130 (US); HANLEY, Michael R., Corte Madera, California 94925 (US); JODKA, Carolyn M., Encinitas, California 92024 (US); LEWIS, Diana Y., San Diego, California 92105 (US); SOARES, Christopher J., La Jolla, California 92037 (US); GHOSH, Soumitra S., San Diego, California 92129 (US); D'SOUZA, Lawrence J., San Diego, California 92130 (US); PARKES, David G., Del Mar, California 92014 (US); MACK, Christine M., San Diego, California 92130 (US)
(74) Representative: Teall, Charlotte
(86) International application number: PCT/US2005/004178
(87) International publication number: WO 2005/077072

(56) References cited:
- WO-A-02/46227
- WO-A-96/40196
- WO-A-97/26321
- US-A- 5 604 203
- US-A1- 2004 053 370
- HALFORD JASON C G ET AL: "THE PSYCHOPHARMACOLOGY OF APPETITE: TARGETS FOR POTENTIAL ANTI-OBESITY AGENTS" CENTRAL NERVOUS SYSTEM AGENTS IN MEDICINAL CHEMISTRY, BENTHAM SCIENCE PUBLISHERS LTD., BUSSUM, NL, vol. 3, no. 4, December 2003 (2003-12), pages 283-310, XP009073953 ISSN: 1871-5249
- HOWL JOHN ET AL: "CHIMERIC STRATEGIES FOR THE RATIONAL DESIGN OF BIOACTIVE ANALOGS OF SMALL PEPTIDE HORMONES" FASEB JOURNAL, FED. OF AMERICAN SOC. FOR EXPERIMENTAL BIOLOGY, BETHESDA, MD, US, vol. 11, no. 7, 1997, pages 582-590, XP002160327 ISSN: 0892-6638
- CHAHRZAD MONTROSE-RAFIZADEH ET AL: "High Potency Antagonists of the Pancreatic Glucagon-like Peptide-1 Receptor" JOURNAL OF BIOLOGICAL CHEMISTRY, AMERICAN SOCIETY OF BIOLOCHEMICAL BIOLOGISTS, BIRMINGHAM,, US, vol. 272, no. 34, 22 August 1997 (1997-08-22), pages 21201-21206, XP002247450 ISSN: 0021-9258

## Description

### FIELD OF THE INVENTION

The present invention relates to peptide chemistry, and more particularly to hybrid polypeptides with selectable properties.

### BACKGROUND OF THE INVENTION

Central to many metabolic diseases and disorders is the regulations of insulin levels and blood glucose levels. Insulin secretion is modulated in part by secretagogue hormones, termed as incretins, which are produced by enteroendocrine cells. The incretin hormone, glucagon-like peptide-1 ("GLP-1") is a peptide hormone secreted by intestinal cells that has been shown in multiple studies to produce an enhancing effect on insulin secretion. GLP-1 is processed from proglucagon in the gut and enhances nutrient-induced insulin release (Krcymann B., et al., Lancet, 2:1300-1303 (1987)). Various truncated forms of GLP-1, are known to stimulate insulin secretion (insulinotropic action) and cAMP formation [see, *e.g.,* Mojsov, S., Int. J. Pep. Pro. Res., 40:333-343 (1992)]. A relationship between various *in vitro* laboratory experiments and mammalian, especially human, insulinotropic responses to exogenous administration of GLP-1, GLP-1(7-36) amide, and GLP-1(7-37) acid has been established (see, *e.g.,* Nauck, M. A., et al., Diabetologia, 36:741-744 (1993); Gutniak, M., et al., New Eng. J. of Med., 326(20):1316-1322 (1992); Nauck, M. A., et al., J. Clin. Invest., 91:301-307 (1993); and Thorens, B., et al., Diabetes, 42:1219-1225 (1993)).

GLP-1(7-36) amide exerts a pronounced antidiabetogenic effect in insulin-dependent diabetics by stimulating insulin sensitivity and by enhancing glucose-induced insulin release at physiological concentrations (Gutniak M., et al., New Eng. J. Med., 326:1316-1322 (1992)). When administered to non-insulin dependent diabetics, GLP-1(7-36) amide stimulates insulin release, lowers glucagon secretion, inhibits gastric emptying and enhances glucose utilization (Nauck, 1993; Gutniak, 1992; Nauck, 1993). However, the use of GLP-1 type molecules for prolonged therapy of diabetes has been complicated because the serum half-life of such peptides is quite short.

More particularly, GLP-1 is a 30-amino acid peptide derived from proglucagon, a 160-amino acid prohormone. Actions of different prohormone convertases in the pancreas and intestine result in the production of glucagons and other ill-defined peptides, whereas cleavage of proglucagon results in the production of GLP-1 and GLP-2 as well as two other peptides. The amino acid sequence of GLP-1 is 100% homologous in all mammals studied so far, implying a critical physiological role. GLP-1 (7-37) acid is C-terminally truncated and amidated to form GLP-1 (7-36) NH₂. The biological effects and metabolic turnover of the free acid GLP-1 (7-37) OH, and the amide, GLP-1 (7-36) NH₂, are indistinguishable. By convention, the numbering of the amino acids is based on the processed GLP-1 (1-37) OH from proglucagon. The biologically active GLP-1 is the result of further processing: GLP-1 (7-36) NH₂. Thus the first amino acid of GLP-1 (7-37) OH or GLP-1 (7-36)NH₂ is ⁷His.

In the gastrointestinal tract, GLP-1 is produced by L-cells of intestinal, colonic and rectal mucosa, in response to stimulation by intraluminal glucose. The plasma half-life of active GLP-1 is <5 minutes, and its metabolic clearance rate is around 12-13 minutes (Holst, 1994). The major protease involved in the metabolism of GLP-1 is dipeptidyl peptidase (DPP) IV (CD26) which cleaves the N-terminal His-Ala dipeptide, thus producing metabolites, GLP-1 (9-37) OH or GLP-1 (9-36) NH₂ which are variously described as inactive, weak agonist or antagonists of GLP-1 receptor. GLP-1 receptor (GLP-1R) is a G protein coupled receptor of 463 amino acid and is localized in pancreatic beta cells, in the lungs and to a lesser extent in the brain, adipose tissue and kidneys. The stimulation of GLP-1R by GLP-1 (7-37) OH or GLP-1 (7-36)NH₂ results in adenylate cyclase activation, cAMP synthesis, membrane depolarization, rise in intracellular calcium and increase in glucose-induced insulin secretion (Holz *et al.,* 1995).

GLP-1 is a potent insulin secretagogue that is secreted from the intestinal mucosa in response to food intake. The profound incretin effect of GLP-1 is underscored by the fact that GLP-1R knockout mice are glucose-intolerant. The incretin response of i.v. infused GLP-1 is preserved in diabetic subjects, though the incretin response to oral glucose in these patients is compromised. GLP-1 administration by infusion or sc injections controls fasting glucose levels in diabetic patients, and maintains the glucose threshold for insulin secretion (Gutniak *et al.,* 1992; Nauck *et al.,* 1986; Nauck *et al.,* 1993). GLP-1 has shown tremendous potential as a therapeutic agent capable of augmenting insulin secretion in a physiological manner, while avoiding hypoglycemia associated with sulfonylurea drugs.

Other important effects of GLP-1 on glucose homeostasis are suppression of glucagon secretion and inhibition of gastric motility. GLP- inhibitory actions on pancreatic alpha cell secretion of glucagon leads to decreases in hepatic glucose production *via* reduction in gluconeogenesis and glycogenolysis. This antiglucagon effect of GLP-1 is preserved in diabetic patients.

The so-called ileal brake effect of GLP-1, in which gastric motility and gastric secretion are inhibited, is effected *via* vagal efferent receptors or by direct action on intestinal smooth muscle. Reduction of gastric acid secretion by GLP-1 contributes to a lag phase in nutrient availability, thus obviating the need for rapid insulin response. In summary, the gastrointestinal effects of GLP-1 contribute significantly to delayed glucose and fatty acid absorption and modulate insulin secretion and glucose homeostasis.

GLP-1 has also been shown to induce beta cell specific genes, such as GLUT-1 transporter, insulin (*via* the interaction of PDX-1 with insulin gene promoter), and hexokinase-1. Thus GLP-1 could potentially reverse glucose intolerance normally associated with aging, as demonstrated by rodent experiments. In addition, GLP-1 may contribute to beta cell neogenesis and increase beta cell mass, in addition to restoring beta cell function during states of beta cell insufficiency.

Central effects of GLP-1 include increases in satiety coupled with decreases in food intake, effected via the action of hypothalamic GLP-1R. A 48 hour continuous SC infusion of GLP-1 in type II diabetic subjects, decreased hunger and food intake and increased satiety. These anorectic effects were absent in GLP-1R knock out mice.

Exendins are another family of peptides implicated in insulin secretion. Exendins are found in the saliva of the Gila-monster, a lizard endogenous to Arizona, and the Mexican Beaded Lizard. Exendin-3 is present in the saliva of Heloderma horridum, and exendin-4 is present in the saliva of Heloderma suspectum (Eng, J., et al., J Biol. Chem., 265:20259-62, 1990; Eng., J., et al., J Biol. Chem., 267:7402-05 (1992)). The exendins have some sequence similarity to several members of the glucagon-like peptide family, with the highest homology, 53%, being to GLP-1 (Goke, et al., J. Biol. Chem., 268:19650-55 (1993)).

Exendin-4 binds the GLP-1 receptors on insulin-secreting TC1 cells, at dispersed acinar cells from guinea pig pancreas, and at parietal cells from stomach; the peptide also stimulates somatostatin release and inhibits gastrin release in isolated stomachs (Goke, et al., J Biol. Chem., 268:19650-55 (1993); Schepp, et al., Eur. J Pharmacol., 69:183-91 (199.4); Eissele, et al., Life Sci., 55:629-34 (1994)). Exendin-3 and exendin-4 were found to bind the GLP-1 receptors on, to stimulating cAMP production in, and amylase release from, pancreatic acinar cells (Malhotra, R., et al., Relulatory Peptides, 41:149-56 (1992); Raufman, et al., J. Biol. Chem., 267:21432-37 (1992); Singh, et al., Regul. Pept., 53:47-59 (1994)). The use of the insulinotropic activities of exendin-3 and exendin-4 for the treatment of diabetes mellitus and the prevention of hyperglycemia has been proposed (Eng, U.S. Pat. No. 5,424,286).

Truncated exendin peptides such as exendin[9-39]-, a carboxyamidated molecule, and fragments 3-39 through 9-39 have been reported to be potent and selective antagonists of GLP-1 *(*Goke, et al., J. Biol. Chem., 268:19650-55 (1993); Raufman, J. P., et al., J. Biol. Chem., 266:2897-902 (1991); Schepp, W., et al., Eur. J Pharm., 269:183-91 (1994); Montrose-Rafizadeh, et al., Diabetes, 45(Suppl. 2):152A (1996)). Exendin[9-39] blocks endogenous GLP-1 *in vivo,* resulting in reduced insulin secretion (Wang, et al., J Clin. Invest., 95:417-21 (1995); D'Alessio, et al., J. Clin. Invest., 97:133-38 (1996)). The receptor apparently responsible for the insulinotropic effect of GLP-1 has been cloned from rat pancreatic islet cells (Thorens, B., Proc. Natl. Acad. Sci. USA 89:8641-8645 (1992)). Exendins and exendin[9-39] bind to the cloned GLP-1 receptor (rat pancreatic - cell GLP-1 receptor: Fehmann HC, et al., Peptides, 15 (3): 453-6 (1994); human GLP-1 receptor: Thorens B, et al., Diabetes, 42 (11): 1678-82 (1993)). In cells transfected with the cloned GLP-1 receptor, exendin-4 is an agonist, *i.e*., it increases cAMP, while exendin[9-39] is an antagonist, *i.e*., it blocks the stimulatory actions of exendin-4 and GLP-1. *Id.*

More particularly, exendin-4 is a 39 amino acid C-terminal amidated peptide found in the saliva of the Gila Monster (Heloderma horridum), with a 53% amino acid sequence homology to the GLP-1 peptide sequence. *See, e.g.,* Eng, J., et al. "Isolation and Characterization of Exendin-4, and Exendin-3 Analogue from Heloderma suspectum Venom," J. Bio. Chem., 267:11, p. 7402-7405 (1992), Young, A. A., et al., "Glucose-Lowering and Insulin-Sensitizing Actions of Exendin-4," Diabetes, Vol. 48, p. 1026-1034, May, 1999. In terms of its activity, exendin-4 is a highly specific agonist for the GLP-1 receptor, and, like GLP-1, is able to stimulate insulin secretion. Therefore, like GLP-1, exendin-4 is regarded as an insulinotropic peptide.

However, unlike GLP-1, exendin-4 has a relatively long half-life in humans, because of its resistance to the dipeptidyl peptidase IV which rapidly degrades the GLP-1 sequence in vivo. Furthermore, it has been shown that, as compared to GLP-1, exendin-4 has a stronger capability to stimulate insulin secretion, and that a lower concentration of exendin-4 may be used to obtain such stimulating activity. See, e.g., U.S. Pat. No. 5,424,286, herein incorporated by reference. Therefore exendin-4 peptides or derivatives thereof (for examples of such derivatives, *see, e.g.,* U.S. Pat. No. 6,528,486, and its corresponding international application WO 01/04156) have a greater potential utility for the treatment of conditions involving the dysregulation of insulin levels (e.g., conditions such as diabetes) than either insulin or GLP-1.

Another family of peptide hormones implicated in metabolic diseases and disorders is the amylin family of peptide hormones, including amylin, calcitonin, calcitonin gene related peptide, adrenomedullin, and intermedin (also known as "AFP-6"). Amylin is a 37-amino acid protein hormone. It was isolated, purified and chemically characterized as the major component of amyloid deposits in the islets of pancreases of human Type 2 diabetics (Cooper et al., Proc. Natl. Acad. Sci., USA, 84:8628-8632 (1987)). The amylin molecule has two post-translational modifications: the C-terminus is amidated, and the cysteines in positions 2 and 7 are cross-linked to form an N-terminal loop. The sequence of the open reading frame of the human amylin gene shows the presence of the Lys-Arg dibasic amino acid proteolytic cleavage signal, prior to the N-terminal codon for Lys, and the Gly prior to the Lys-Arg proteolytic signal at the CLAIMS-terminal position, a typical sequence for amidation by protein amidating enzyme, PAM (Cooper et al., Biochem. Biophys. Acta, 1014:247-258 (1989)).

Amylin is believed to regulate gastric emptying, and suppress glucagon secretion and food intake, thus regulating the rate of glucose appearance in the circulation. It appears to complement the actions of insulin, which regulates the rate of glucose disappearance from the circulation and its uptake by peripheral tissues. These actions are supported by experimental findings in rodents and humans, which indicate that amylin complements the effects of insulin in postprandial glucose control by at least three independent mechanisms, all of which affect the rate of glucose appearance. First, amylin suppresses postprandial glucagon secretion. Compared to healthy adults, patients with type 1 diabetes have no circulating amylin and patients with type 2 diabetes have diminished postprandial amylin concentrations. Furthermore, infusion of an amylin specific monoclonal antibody, which bound circulating amylin, again resulted in greatly elevated glucagon concentrations relative to controls. Both of these results point to a physiological role of endogenous amylin in the regulation of postprandial glucagon secretion. Second, amylin slows gastrointestinal motility and gastric emptying. Finally, intrahypothalamic injections of rat amylin were shown to reduce feeding in rats and alter neurotransmitter metabolism in the hypothalamus. In certain studies, food intake was significantly reduced for up to eight hours following the intrahypothalamic injection of rat amylin and rat CGRP. In human trials, an amylin analog, pramlintide, has been shown to reduce weight or weight gain. Amylin may be beneficial in treating metabolic conditions such as diabetes and obesity. Amylin may also be used to treat pain, bone disorders, gastritis, to modulate lipids, in particular triglycerides, or to affect body composition such as the preferential loss of fat and sparing of lean tissue.

The hormone calcitonin (CT) was named for its secretion in response to induced hypercalcemia and its rapid hypocalcemic effect. It is produced in and secreted from neuroendocrine cells in the thyroid that have since been termed C cells. The best-studied action of CT(1-32) is its effect on the osteoclast. *In vitro* effects of CT include the rapid loss of ruffled borders and decreased release of lysosomal enzymes. Ultimately, the inhibition of osteoclast functions by CT results in a decrease in bone resorption. However, neither a chronic reduction of serum CT in the case of thyroidectomy nor the increased serum CT found in medullary thyroid cancer appears to be associated with changes in serum calcium or bone mass. It is thus most likely that a major function of CT(1-32) is to combat acute hypercalcemia in emergency situations and/or protect the skeleton during periods of "calcium stress" such as growth, pregnancy, and lactation. (Reviewed in Becker, JCEM, 89(4): 1512-1525 (2004) and Sexton, Current Medicinal Chemistry 6: 1067-1093 (1999)). Consistent with this is recent data from the calcitonin gene knockout mouse, which removes both the calcitonin and the CORP-I peptides, that revealed that the mouse had normal levels of basal calcium-related values, but an increased calcemic response (Kurihara H, et al., Hypertens Res. 2003 Feb; 26 Suppl:S 105-8).

CT has an effect on plasma calcium levels and inhibits osteoclast function and is widely used for the treatment of osteoporosis. Therapeutically, salmon CT (sCT) appears to increase bone density and decrease fracture rates with minimal adverse effects. CT has also been successfully used over the past 25 years as a therapy for Paget's disease of bone, which is a chronic skeletal disorder that may result in enlarged or deformed bones in one or more regions of the skeleton. CT is also widely used for its analgesic effect on bone pain experienced during osteoporosis, although the mechanism for this effect is not clearly understood.

Calcitonin gene related peptide (CGRP) is a neuropeptide whose receptors are widely distributed in the body, including the nervous system and the cardiovascular system.

This peptide seems to modulate sensory neurotransmission and is one of the most potent endogenous vasodilatory peptide discovered to date. Reported biological effects for CGRP include: modulation of substance P in inflammation, nicotinic receptor activity at the neuromuscular junction, stimulation of pancreatic enzyme secretion, a reduction of gastric acid secretion, peripheral vasodilation, cardiac acceleration, neuro-modulation, regulation of calcium metabolism, osteogenic stimulation, insulin secretion, an increase in body temperature and a decrease in food intake. (Wimalawansa, Amylin, calcitonin gene-related peptide, calcitonin and ADM: a peptide superfamily. Crit Rev Neurobiol. 1997; 11 (2-3):167-239). An important role of CGRP is to control blood flow to various organs by its potent vasodilatory actions, as evidenced by a decrease of mean arterial pressure following intravenous administration of α-CGRP. The vasodilatory actions are also supported by recent analysis of homozygous knockout CGRP mice, which demonstrated elevated peripheral vascular resistance and high blood pressure caused by increased peripheral sympathetic activity. (Kurihara H, et al., Targeted disruption of ADM and αCGRP genes reveals their distinct biological roles. Hypertens Res. 2003 Feb; 26 Suppl:S105-8). Thus, CGRP appears to elicit vasodilatory effects, hypotensive effects and an increase in heart rate among other actions.

Prolonged infusion of CGRP into patients with congestive cardiac failure has shown a sustained beneficial effect on hemodynamic functions without adverse effects, suggesting a use in heart failure. Other indications of CGRP use include renal failure, acute and chronic coronary artery ischemia, treatment of cardiac arrhythmia, other peripheral vascular disease such as Raynaud's phenomenon, subarachnoid hemorrhage, hypertension, and pulmonary hypertension. Preeclamptic toxemia of pregnancy and preterm labor is also potentially treatable. (Wimalawansa, 1997). Recent therapeutic uses include the use of CGRP antagonists for the treatment of migraine headaches.

Adrenomedullin (ADM) is almost ubiquitously expressed with many more tissues containing the peptide than not. A published review of ADM, (Hinson, J.P. et al., Endocrine Reviews (2000) 21(2): 138-167) details its effects on the cardiovascular system, cellular growth, the central nervous system and the endocrine system, with a range of biological actions including vasodilation, cell growth, regulation of hormone secretion, and natriuresis. Studies in rat, cat, sheep, and man confirm that intravenous infusion of ADM results in potent and sustained hypotension, and is comparable to that of CGRP. However, the hypotensive effect of ADM on mean arterial pressure in the anesthetized rat is not inhibited by the CGRP antagonist CGRP₈₋₃₇ suggesting that this effect is not mediated via CGRP receptors. Acute or chronic administration of human ADM in rats, anesthetized, conscious or hypertensive, results in a significant decrease in total peripheral resistance accompanied by a fall in blood pressure, with a concomitant rise in heart rate, cardiac output and stroke volume.

ADM has also been proposed as an important factor in embryogenesis and differentiation and as an apoptosis survival factor for rat endothelial cells. This is supported by recent mouse ADM knockout studies, in which mice homozygous for loss of the ADM gene demonstrated defective vascular formation during embryogenesis and thus died mid-gestation. It was reported that ADM +/- heterozygous mice had high blood pressure along with susceptibility to tissue injury (Kurihara H, et al., Hypertens Res. 2003 Feb; 26 Suppl:S105-8).

ADM affects such endocrine organs as the pituitary, the adrenal gland, reproductive organs and the pancreas. The peptide appears to have a role in inhibiting ACTH release from the pituitary. In the adrenal gland, it appears to affect the secretory activity of the adrenal cortex in both rat and human and it increases adrenal blood flow, acting as a vasodilator in the adrenal vascular bed in intact rats. ADM has been shown to be present throughout the female reproductive tract and plasma levels are elevated in normal pregnancy. Studies in a rat model of preeclampsia show that ADM can reverse hypertension and decrease pup mortality when given to rats during late gestation. Because it did not have a similar effect in animals in early gestation or non-pregnant rats in the preeclampsia model, this suggests that ADM may play an important regulatory role in the utero-placental cardiovascular system. In the pancreas, ADM most likely plays an inhibitory role since it attenuated and delayed insulin response to an oral glucose challenge, resulting in initial elevated glucose levels. ADM can also affect renal function. A bolus administered peripherally can significantly lower mean arterial pressure and raise renal blood flow, glomerular .filtration rate and urine flow. In some cases, there is also an increase in Na+ excretion.

ADM also has other peripheral effects on bone and on the lung. For bone, studies have supported a role beyond the cardiovascular system and fluid homeostasis and have demonstrated that ADM acts on fetal and adult rodent osteoblasts to increase cell growth comparable to those of known osteoblast growth factors such as transforming growth factor-β. This is important clinically as one of the major challenges in osteoporosis research is to develop a therapy that increases bone mass via osteoblastic stimulation. In the lung, ADM not only causes pulmonary vasodilation, but also inhibits bronchoconstriction induced by histamine or acetylcholine. Recent studies using aerosolized ADM to treat pulmonary hypertension in a rat model indicate that inhalation treatment of this condition is effective, as evidenced by the fact that mean pulmonary arterial pressure and total pulmonary resistance were markedly lower in rats treated with ADM than in those given saline. This result was achieved without an alteration in systemic arterial pressure or heart rate (Nagaya N et al., Am J Physiol Heart Circ Physiol. 2003;285:H2125-31).

In healthy volunteers, i.v. infusion of ADM has been shown to reduce arterial pressure and to stimulate heart rate, cardiac output, plasma levels of cAMP, prolactin, norepinephrine and rennin. In these patients, there was little or no increase in urine volume or sodium excretion observed. In patients with heart failure or chronic renal failure, i.v. ADM had similar effects to those seen in normal subjects, and also induced diuresis and natriuresis, depending on the dose administered (Nicholls, MG et al. Peptides. 2001; 22:1745-1752) Experimental ADM treatment has also been shown to be beneficial in arterial and pulmonary hypertension, septic shock and ischemia/reperfusion injury (Beltowski J., Pol J Pharmacol. 2004;56:5-27). Other indications for ADM treatment include: peripheral vascular disease, subarachnoid hemorrhage, hypertension, preeclamptic toxemia of pregnancy and preterm labor, and osteoporosis.

Expression of AFP-6 (*i.e*., intermedin) is primarily in the pituitary and gastrointestinal tract. A specific receptor for AFP-6 has not been reported; however, binding studies indicate that AFP-6 binds to all the known receptors of the Amylin Family. AFP-6 has been shown to increase cAMP production in SK-N-MC and L6 cells expressing endogenous CGRP receptors and competes with labeled CGRP for binding to its receptors in these cells. In published *in vivo* studies, AFP-6 administration led to blood pressure reduction in both normal and spontaneously hypertensive rats, most likely via interactions with the CRLR/RAMP receptors. *In vivo* administration in mice led to a suppression of gastric emptying and food intake. (Roh et al. J Biol Chem. 2004 Feb 20;279(8):7264-74.)

It has been reported that the biological actions of amylin family peptide hormones are generally mediated via binding to two closely related type II G protein-coupled receptors (GPCRs), the calcitonin receptor (CTR) and the calcitonin receptor like receptor (CRLR). Cloning and functional studies have shown that CGRP, ADM, and amylin interact with different combinations of CTR or the CRLR and the receptor activity modifying protein (RAMP). Many cells express multiple RAMPs. It is believed that co-expression of RAMPs and either the CTR or CRLR is required to generate functional receptors for calcitonin, CGRP, ADM, and amylin. The RAMP family comprises three members (RAMP1, -2, and -3), which share less then 30% sequence identity, but have a common topological organization. Co-expression of CRLR and RAMP1 leads to the formation of a receptor for CGRP. Co-expression of CRLR and RAMP2 leads to the formation of a receptor for ADM. Co-expression of CRLR and RAMP3 leads to the formation of a receptor for ADM and CGRP. Co-expression of hCTR2 and RAMP1 leads to the formation of a receptor for amylin and CGRP. Co-expression of hCTR2 and RAMP3 leads to the formation of a receptor for amylin.

Yet another peptide hormone family implicated in metabolic diseases and disorders is the leptin family. The mature form of circulating leptin is a 146-amino acid protein that is normally excluded from the CNS by the blood-brain barrier (BBB) and the blood-CSF barrier. See, *e.g*., Weigle et al., 1995. J Clin Invest 96 : 2065-2070. Leptin is the afferent signal in a negative feedback loop regulating food intake and body weight. The leptin receptor is a member of the cytokine receptor family. Leptin's anorexigenic effect is dependent on binding to homodimer of the Ob-Rb isoform of this receptor which encodes a long intra-cytoplasmic domain that includes several motifs for protein-protein interaction. Ob-Rb is highly expressed in the hypothalamus suggesting that this brain region is an important site of leptin action. Mutation of the mouse *ob* gene has been demonstrated to result in a syndrome that exhibits-pathophysiology that includes: obesity, increased body fat deposition, hyperglycemia, hyperinsulinemia, hypothermia, and impaired thyroid and reproductive functions in both male and female homozygous ob/ob obese mice (see e.g., Ingalis, et al., 1950. J Hered 41 : 317-318. Therapeutic uses for leptin or leptin receptor include (i) diabetes (see, *e.g.,* PCT Patent Applications WO 98/55139, WO 98/12224, and WO 97/02004); (ii) hematopoiesis (see, e.g., PCT Patent Applications WO 97/27286 and WO 98/18486); (iii) infertility (see, e.g., PCT Patent Applications WO 97/15322 and WO 98/36763); and (iv) tumor suppression (see, e.g., PCT Patent Applications WO 98/48831).

The leptin receptor (OB-R) gene has been cloned (GenBank Accession No. AF098792) and mapping to the db locus (see, *e.g.,* Tartaglia, et al., 1995. Cell 83: 1263-1271). Several transcripts of the OB-R, resulting from alternative splicing, have also been identified. Defects in OB-R produce a syndrome in the mutant diabetic ob/ob mouse that is phenotypically identical to the ob/ob mouse (see, e.g., Ghilardi, et al., 1996. Proc. Natl. Acad. Sci. USA 93: 6231-6235). In contrast to ob/ob mice, however, administration of recombinant leptin to C57BLKS/J-m ob/ob mice does not result in reduced food intake and body weight (see, e.g., Roberts and Greengerg, 1996. Nutrition Rev. 54: 41-49).

Most leptin-related studies able to report weight loss activity from administration of recombinant leptin, leptin fragments and/or leptin receptor variant have administered said constructs directly into the ventricles of the brain. See e.g., Weigle, et al., 1995. J Clin Invest 96 : 2065-2070; Barash, et al., 1996. Endocrinology 137: 3144-3147.

Other studies have shown significant weight loss activity due to administered of leptin peptides through intraperitoneally (i.p.) administration to test subjects. See, Grasso et al., 1997. Endocrinology 138: 1413-1418. Further, leptin fragments, and most particularly an 18 amino acid fragment comprising residues taken from full length human leptin, have been reported to function in weight loss, but only upon direct administration through an implanted cannula to the lateral brain ventricle of rats. See, *e.g*., PCT Patent Applications WO 97/46585, which is incorporated herein by reference in its entirety.

Another peptide hormone implicated in metabolic diseases and disorders is cholecystokinin (CCK). CCK was reportedly identified in 1928 from preparations of intestinal extracts by its ability to stimulate gallbladder contraction. Other biological actions of CCK have since been reported, including stimulation of pancreatic secretion, delayed gastric emptying, stimulation of intestinal motility and stimulation of insulin secretion. See Lieverse et al., Ann. N.Y. Acad. Sci. 713: 268-272 (1994). The actions of CCK, also reportedly include effects on cardiovascular function, respiratory function, neurotoxicity and seizures, cancer cell proliferation, analgesia, sleep, sexual and reproductive behaviors, memory, anxiety and dopamine-mediated behaviors. Crawley and Corwin, Peptides 15: 731-755 (1994). Other reported effects of CCK include stimulation of pancreatic growth, stimulation of gallbladder contraction, inhibition of gastric acid secretion, pancreatic polypeptide release and a contractile component of peristalsis. Additional reported effects of CCK include vasodilation. Walsh, "Gastrointestinal Hormones," In Physiology of the Gastrointestinal Tract (3d ed. 1994; Raven Press, New York).

It has been reported that injections of combinations of glucagon, CCK and bombesin potentiated the inhibition of intake of condensed milk test meals in nondeprived rats over the inhibitions observed with individual compounds. Hinton et al., Brain Res. Bull. 17:615-619 (1986). It has also been reported that glucagon and CCK synergistically inhibit sham feeding in rats. LeSauter and Geary, Am. J. Physiol. 253:R217-225 (1987); Smith and Gibbs, Annals N.Y. Acad. Sci. 713:236-241 (1994). It has also been suggested that estradiol and CCK can have a synergistic effect on satiety. Dulawa et al., Peptides 15:913-918 (1994); Smith and Gibbs, *supra.* It has also been proposed that signals arising from the small intestine in response to nutrients therein may interact synergistically with CCK to reduce food intake. Cox, Behav. Brain Res. 38:35-44 (1990). Additionally, it has been reported that CCK induces satiety in several species. For example, it has been reported that feeding depression was caused by CCK injected intraperitoneally in rats, intraarterially in pigs, intravenously in cats and pigs, into the cerebral ventricles in monkeys, rats, dogs and sheep, and intravenously in obese and non-obese humans. See Lieverse *et al., supra.* Studies from several laboratories have reportedly confirmed the behavioral specificity of low doses of CCK on inhibition in feeding, by comparing responding for food to responding for nonfood reinforcers in both monkeys and rats and by showing that CCK elicits the sequence of behaviors normally observed after meal ingestion (*i.e*.,- the postprandial satiety sequence). Additionally, comparison of behavior after CCK to behavior after food ingestion, alone or in combination with CCK has reportedly revealed behavioral similarities between CCK and food ingestion. Crawley and Corwin, *supra.* It has also been reported that CCK in physiological plasma concentrations inhibits food intake and increases satiety in both lean and obese humans. See Lieverse *et al., supra.*

CCK was characterized in 1966 as a 33-amino acid peptide. Crawley and Corwin, supra. Species-specific molecular variants of the amino acid sequence of CCK have been identified. The 33-amino acid sequence and a truncated peptide, its 8-amino acid C-terminal sequence (CCK-8) have been reportedly identified in pig, rat, chicken, chinchilla, dog and humans. A 39-amino acid sequence was reportedly found in pig, dog and guinea pig. A 58-amino acid sequence was reported to have been found in cat, dog and humans. Frog and turtle reportedly show 47-amino acid sequences homologous to both CCK and gastrin. Very fresh human intestine has been reported to contain small amounts of an even larger molecule, termed CCK-83. In the rat, a principal intermediate form has been reportedly identified, and is termed CCK-22. Walsh, "Gastrointestinal Hormones," In Physiology of the Gastrointestinal Tract (3d ed. 1994; Raven Press, New York). A non-sulfated CCK-8 and a tetrapeptide (termed CCK-4 (CCK(30-33)) have been reported in rat brain. The C-terminal pentapeptide (termed CCK-4 (CCK(29-33)) conserves the structural homology of CCK, and also homology with the neuropeptide, gastrin. The C-terminal sulfated octapeptide sequence, CCK-8, is reportedly relatively conserved across species. Cloning and sequence analysis of a cDNA encoding preprocholecystokinin from rat thyroid carcinoma, porcine brain, and porcine intestine reportedly revealed 345 nucleotides coding for a precursor to CCK, which is 115 amino acids and contains all of the CCK sequences previously reported to have been isolated. Crawley and Corwin, *supra.*

CCK is said to be distributed throughout the central nervous system and in endocrine cells and enteric nerves of the upper small intestine. CCK agonists include CCK itself (also referred to as CCK-33), CCK-8 (CCK(26-33)), non-sulfated CCK-8, pentagastrin (CCK-5 or CCK(29-33)), and the tetrapeptide, CCK-4 (CCK(30-33)). At the pancreatic CCK receptor, CCK-8 reportedly displaced binding with a 1000-5000 greater potency than unsulfated CCK-8 or CCK-4, and CCK-8 has been reported to be approximately 1000-fold more potent than unsulfated CCK-8 or CCK-4 in stimulating pancreatic amylase secretion. Crawley and Corwin, supra. In homogenates from the cerebral cortex, CCK receptor binding was said to be displaced by unsulfated CCK-8 and by CCK-4 at concentrations that were equimolar, 10-fold or 100-fold greater than sulfated CCK-8. Id.

Receptors for CCK have been reportedly identified in a variety of tissues, and two primary subtypes have been described: type A receptors and type B receptors. Type A receptors have been reported in peripheral tissues including pancreas, gallbladder, pyloric sphincter and afferent vagal fibers, and in discrete areas of the brain. The type A receptor subtype (CCK_{A}) has been reported to be selective for the sulfated octapeptide. The Type B receptor subtype (CCK_{B}) has been identified throughout the brain and in the stomach, and reportedly does not require sulfation or all eight amino acids. See Reidelberger, J. Nutr. 124 (8 Suppl.) 13275-1333S (1994); Crawley and Corwin, *supra.*

Yet another family of peptide hormones implicated in metabolic diseases and disorders is the pancreatic polypeptide family ("PPF"). Pancreatic polypeptide ("PPF was discovered as a contaminant of insulin extracts and was named by its organ of origin rather than functional importance (Kimmel et al., Endocrinology 83: 1323-30 (1968)). PP is a 36-amino acid peptide containing distinctive structural motifs. A related peptide was subsequently discovered in extracts of intestine and named Peptide YY ("PYY") because of the N- and C-terminal tyrosines (Tatemoto, Proc. Natl. Acad. Sci. USA 79: 2514-8 (1982)). A third related peptide was later found in extracts of brain and named Neuropeptide Y ("NPY") (Tatemoto, Proc. Natl. Acad. Sci. USA 79: 5485-9 (1982); Tatemoto et al., Nature 296: 659-60 (1982)).

These three related peptides have been reported to exert various biological effects. Effects of PP include inhibition of pancreatic secretion and relaxation of the gallbladder. Centrally administered PP produces modest increases in feeding that may be mediated by receptors localized to the hypothalamus and brainstem (reviewed in Gehlert, Proc. Soc. Exp. Biol. Mend. 218: 7-22 (1998)).

Release of PYY occurs following a meal. An alternate molecular form of PYY is PYY(3-36) (Eberlein et al., Peptides 10: 797-803 (1989); Grandt et al., Regul. Pept. 51: 151-9 (1994)). This fragment constitutes approximately 40% of total PYY-like immunoreactivity in human and canine intestinal extracts and about 36% of total plasma PYY immunoreactivity in a fasting state to slightly over 50% following a meal. It is apparently a dipeptidyl peptidase-IV (DPP4) cleavage product of PYY. PYY(3-36) is reportedly a selective ligand at the Y2 and Y5 receptors, which appear pharmacologically unique in preferring N-terminally truncated (*i.e*., C-terminal fragments of) NPY analogs. Peripheral administration of PYY reportedly reduces gastric acid secretion, gastric motility, exocrine pancreatic secretion (Yoshinaga et al., Am. J. Physiol. 263: G695-701 (1992); Guan et al., Endocrinology 128: 911-6 (1991); Pappas et al., Gastroenterology 91: 1386-9 (1986)), gallbladder contraction and intestinal motility (Savage et al., Gut 28: 166-70 (1987)). The effects of central injection of PYY on gastric emptying, gastric motility and gastric acid secretion, as seen after direct injection in or around the hindbrain/brainstem (Chen and Rogers, Am. J. Physiol. 269: R787-92 (1995); Chen et al., Regul. Pept. 61: 95-98 (1996); Yang and Tache, Am. J. Physiol. 268: G943-8 (1995); Chen et al., Neurogastroenterol. Motil. 9: 109-16 (1997)), may differ from those effects observed after peripheral injection. For example, centrally administered PYY had some effects opposite to those described herein for peripherally injected PYY(3-36) in that gastric acid secretion was stimulated, not inhibited. Gastric motility was suppressed only in conjunction with TRH stimulation, but not when administered alone, and was indeed stimulatory at higher doses through presumed interaction with PP receptors. PYY has been shown to stimulate food and water intake after central administration (Morley et al., Brain Res. 341: 200-3 (1985); Corp et al., Am. J. Physiol. 259: R317-23 (1990)).

Metabolic diseases and disorders take on may forms, including obesity, diabetes, dyslipidemia, insulin resistance, cellular apoptosis, *etc.* Obesity and its associated disorders are common and very serious public health problems in the United States and throughout the world. Upper body obesity is the strongest risk factor known for type 2 diabetes mellitus, and is a strong risk factor for cardiovascular disease. Obesity is a recognized risk factor for hypertension, atherosclerosis, congestive heart failure, stroke, gallbladder disease, osteoarthritis, sleep apnea, reproductive disorders such as polycystic ovarian syndrome, cancers of the breast, prostate, and colon, and increased incidence of complications of general anesthesia *(see, e.g.,* Kopelman, Nature 404: 635-43 (2000)). It reduces life-span and carries a serious risk of co-morbidities above, as well disorders such as infections, varicose veins, acanthosis nigricans, eczema, exercise intolerance, insulin resistance, hypertension hypercholesterolemia, cholelithiasis, orthopedic injury, and thromboembolic disease (Rissanen et al., Br. Med. J. 301: 835-7 (1990)). Obesity is also a risk factor for the group of conditions called insulin resistance syndrome, or "Syndrome X." Recent estimate for the medical cost of obesity and associated disorders is $150 billion worldwide. The pathogenesis of obesity is believed to be multifactorial but the basic problem is that in obese subjects nutrient availability and energy expenditure do not come into balance until there is excess adipose tissue. Obesity is currently a poorly treatable, chronic, essentially intractable metabolic disorder. A therapeutic drug useful in weight reduction of obese persons could have a profound beneficial effect on their health.

Diabetes is a disorder of carbohydrate metabolism characterized by hyperglycemia and glucosuria resulting from insufficient production or utilization of insulin. Diabetes severely affects the quality of life of large parts of the populations in developed countries. Insufficient production of insulin is characterized as type 1 diabetes and insufficient utilization of insulin is type 2 diabetes. However, it is now widely recognized that there are many distinct diabetes related diseases which have their onset long before patients are diagnosed as having overt diabetes. Also, the effects from the suboptimal control of glucose metabolism in diabetes gives rise to a wide spectrum of related lipid and cardiovascular disorders.

Dyslipidemia, or abnormal levels of lipoproteins in blood plasma, is a frequent occurrence among diabetics. Dyslipidemia is typically characterized by elevated plasma triglycerides, low HDL (High Density Lipoprotein) cholesterol, normal to elevated levels of LDL (Low Density Lipoprotein) cholesterol and increased levels of small dense, LDL (Low Density Lipoprotein) particles in the blood. Dyslipidemia is one of the main contributors to the increased incidence of coronary events and deaths among diabetic subjects. Epidemiological studies have confirmed this by showing a several-fold increase in coronary deaths among diabetic subjects when compared with non-diabetic subjects. Several lipoprotein abnormalities have been described among diabetic subjects.

Insulin resistance is the diminished ability of insulin to exert its biologically action across a broad range of concentrations. In insulin resistance, the body secretes abnormally high amounts of insulin to compensate for this defect and a state of impaired glucose tolerance develops. Failing to compensate for the defective insulin action, the plasma glucose concentration inevitable rises, resulting in the clinical state of diabetes. It is being recognized that insulin resistance and relative hyperinsulinemia have a contributory role in obesity, hypertension, atherosclerosis and type 2 diabetes. The association of insulin resistance with obesity, hypertension and angina has been described as a syndrome, Syndrome X, having insulin resistance as the common pathogenic link.

Apoptosis is an active process of cellular self-destruction that is regulated by extrinsic and intrinsic signals occurring during normal development. It is well documented that apoptosis plays a key role in regulation of pancreatic endocrine beta cells. There is increasing evidence that in adult mammals the beta-cell mass is subject to dynamic changes to adapt insulin production for maintaining euglycemia in particular conditions, such as pregnancy and obesity. The control of beta cell mass depends on a subtle balance between cell proliferation, growth and programmed cell death (apoptosis). A disturbance of this balance may lead to impairment of glucose homeostasis. For example, it is noteworthy that glucose intolerance develops with aging when beta cell replication rates are reduced and human autopsy studies repeatedly showed a 40-60% reduction of beta cell mass in patients with non-insulin-dependent-diabetes mellitus compared with nondiabetic subjects. It is generally agreed that insulin resistance is an invariable accompaniment of obesity but that normoglycemia is maintained by compensatory hyperinsulinemia until the beta cells become unable to meet the increased demand for insulin, at which point type 2 diabetes begins.

Attempts to treatment of the multiple abnormalities associated with diabetes have prompted for the administration of several anti-diabetic medicaments in order to address these abnormalities in the different patients. Examples of anti-diabetic medicaments are proteins such as insulin and insulin analogues, and small molecules such as insulin sensitizers, insulin secretagogues and appetite regulating compounds.

There remains a need to develop polypeptides to useful in the above described metabolic diseases, conditions, and disorders. Accordingly, it is an object of the present invention to provide hybrid polypeptides and methods for producing and using them.

All documents referred to herein are incorporated by reference into the present application as though fully set forth herein.

### SUMMARY OF THE INVENTION

The present invention according to the claims relates generally, to novel, selectable hybrid polypeptides useful as agents for the treatment and prevention of metabolic diseases and disorders which can be alleviated by control plasma glucose levels, insulin levels, and/or insulin secretion, such as diabetes and diabetes-related conditions. Such conditions and disorders include, but are not limited to, hypertension, dyslipidemia, cardiovascular disease, eating disorders, insulin-resistance, obesity, and diabetes mellitus of any kind, including type 1, type 2, and gestational diabetes.

In one aspect of the invention, hybrid polypeptides according to the claims exhibiting at least one hormonal activity are provided. The hybrid polypeptides of the invention according to the claims comprises at least two bio-active peptide hormone modules covalently linked together, wherein at least one of the bio-active peptide hormone modules exhibits at least one hormonal activity of a component peptide hormone. The bio-active peptide hormone modules are independently selected from: component peptide hormones, fragments of component peptide hormones that exhibit at least one hormonal activity of the component peptide hormones, analogs and derivatives of component peptide hormones that exhibit at least one hormonal activity of the component peptide hormones, fragments of analogs and derivatives of component peptide hormones that exhibit at least one hormonal activity of the component peptide hormones, and peptidic enhancers.

Component peptide hormones of the disclosure include: amylin, adrenomedullin (ADM), calcitonin (CT), calcitonin gene related peptide (CGRP), intennedin, cholecystokinin ("CCK"), leptin, peptide YY (PYY), glucagon-like peptide-1 (GLP-1), glucagon-like peptide 2 (GLP-2), oxylitomodulin (OXM), and exendin-4; Peptidic enhancers of the disclosure include: structural motifs of component peptide hormones that impart a desired chemical stability, conformational stability, metabolic stability, receptor interaction, protease inhibition, or other pharmacokinetic characteristic to the hybrid polypeptide, and structural motifs of analogs or derivatives of component peptide hormones that impart a desired chemical stability, conformational stability, metabolic stability, receptor interaction, protease inhibition, or other pharmacokinetic characteristic to the hybrid polypeptide.

In another aspect of the disclosure, methods for treating or preventing obesity are provided, wherein the method comprises administering a therapeutically or prophylactically effective amount of a hybrid polypeptide of the invention to a subject in need thereof. In a preferred disclosure, the subject is an obese or overweight subject. While "obesity" is generally defined as a body mass index over 30, for purposes of this disclosure, any subject, including those with a body mass index of less than 30, who needs or wishes to reduce body weight is included in the scope of "obese." Subjects who are insulin resistant, glucose intolerant, or have any form of diabetes mellitus (e.g., type 1, 2 or gestational diabetes) can benefit from this method.

In yct another aspect of the disclosure, methods of reducing food intake, reducing nutrient availability, causing weight loss, treating diabetes mellitus or diabetes-associated conditions, and improving lipid profile (including reducing LDL cholesterol and triglyceride levels and/or changing HDL cholesterol levels) are provided, wherein the methods comprise administering to a subject an effective amount of a hybrid polypeptide of the disclosure. In a preferred disclosure, the methods of the invention are used to treat or prevent conditions or disorders which can be alleviated by reducing nutrient availability in a subject in need thereof, comprising administering to said subject a therapeutically or prophylactically effective amount of a hybrid polypeptide of the disclosure. In another disclosure, the methods of the invention are used to treat or prevent conditions or disorders which can be alleviated by control plasma glucose levels, insulin levels, and/or insulin secretion. In an embodiment of the invention, the hybrid of the invention are used to treat diabetes and/or diabetes-related conditions. Such conditions and disorders include, but are not limited to, hypertension, dyslipidemia, cardiovascular disease, eating disorders, insulin-resistance, obesity, and diabetes mellitus of any kind, including Type I, Type II, and gestational diabetes, diabetes complications (neuropathy (based on, *e.g*., neurotrophic actions of exendin-4), neuropathic pain (based on, *e.g.,* amylin action), retinopathy, nephropathy, conditions of insufficient pancreatic beta cell mass (based on, *e.g*., islet neogenesis actions of exendin-4 and GLP-1).

The present invention also relates to pharmaceutical compositions comprising a therapeutically or prophylactically effective amount of at least one hybrid polypeptide of the invention, or a pharmaceutically acceptable salt thereof, together with pharmaceutically acceptable diluents, preservatives, solubilizers, emulsifiers, adjuvants and/or carriers useful in the delivery of the hybrid polypeptides.

These and other aspects of the invention will be more clearly understood with reference to the following preferred embodiments and detailed description.

### BRIEF DESCRIPTION OF THE DRAWINGS

Figure 1 demonstrates the effect of exemplary compounds of the invention in DIO mouse assay.
Figure 2 demonstrates the effect of exemplary compounds of the invention in DIO mouse assay.
Figures 3A-3B demonstrates the effect of exemplary compounds of the invention in DIO mouse assay.
Figure 4A-4B demonstrates the effects of exemplary compounds of the invention in food intake assay, compared to parent peptide compounds.

### DETAILED DESCRIPTION OF THE INVENTION

Type present invention according to the claims relates generally to novel, selectable hybrid polypeptides useful as agents for the treatment and prevention of metabolic diseases and disorders which can be alleviated by control plasma glucose levels, insulin levels, and/or insulin secretion, such as diabetes and diabetes-related conditions. Such conditions and disorders include, but are not limited to, hypertension, dyslipidemia, cardiovascular disease, eating disorders, insulin-resistance, obesity, and diabetes mellitus of any kind, including type 1, type 2, and gestational diabetes.

In one aspect, the invention according to the claims involves the modular assembly of physiologically, metabolically, and/or pharmacokinetically active peptidic modules that may be selectable based on "bio-activities", *e.g*., therapeutic efficacy, scope of function, duration of action, physicochemical properties, and/or other pharmacokinetic properties. Without intending to be limited by theory, the present invention according to the claims relates at least in part to a "toolbox" approach, wherein bio-active peptide hormone modules are linked in binary, tertiary or higher order combinations to create novel, efficacious therapeutic agents with selectable properties. The "bio-active peptide hormone modules" may be peptide hormones, peptide fragments with hormonal activity, or structural motifs of peptide hormones that impart chemical, metabolic, and/or other pharmacokinetic stability. The peptide hormones can include native peptide hormones, as well as peptide hormone analogs and derivatives according to the claims, as known in the art and described herein.

In one aspect of the invention, it has been found that the combination of certain physicochemical characteristics of two or more peptide hormones into a single modality can facilitate intervention at several points in a dysfunctional metabolic circuit. As such, in one aspect of the invention, rationally-designed hybrid polypeptides are provided that integrate selectable bio-activities into a single polypeptide agent. In one embodiment, the selectable hybrid polypeptides of the invention may involve the use of chemically stable linkers to covalently attach the bio-active modules. In another embodiment, the selectable hybrid polypeptides of the invention may involve the use of cleavable linkers, which themselves may be or form part of a bio-active module.

Again, without intending to be limited by theory, design of the hybrid polypeptides of the present disclosure may generally involve: (1) the identification, selection and pairing of bio-active peptide hormone modules for desired efficacy and therapeutic use, and (2) the covalent linking of the bio-active modules (*e.g*. native peptide hormones, peptide hormone analogs or derivatives with hormonal activity, peptide hormone fragments with hormonal activity, stabilising motifs, *etc*.) either directly or *via* a linker without loss of bio-activity of the component modules. In certain disclosures, module selection criteria may include, but not be limited to: (a) desired *in vivo* efficacy for desired therapeutic or prophylactic indication; (b) optional synergism or dual action of the linked modules for multiple therapeutic or prophylactic indications; and/or (c) a desired chemical stability, conformational stability, metabolic stability, receptor interaction, protease inhibition, and/or other pharmacokinetic characteristic.

The section headings are used herein for organizational purposes only, and are not to be construed as in any way limiting the subject matter described.

### Hybrid Polypeptides of the Invention

As mentioned above, the present invention according to the claims relates in part to hybrid polypeptides comprising at least two bio-active peptide hormone modules selectable from component peptide hormones described herein. The hybrid polypeptides of the present invention will generally be useful in the treatment and prevention of metabolic conditions and disorders. The hybrid polypeptides of the invention according to the claims will exhibit at least one hormonal activity of a component peptide hormone, and include at least one additional bio-activity of a second component peptide hormone.

In one embodiment according to the claims, the hybrid polypeptides of the invention may comprise at least two bio-active peptide hormone modules, wherein each of said at least two bio-active peptide hormone modules exhibits at least one hormonal activity of a component peptide hormone. In another disclosure, the hybrid polypeptides of the invention may comprise at least two bio-active peptide hormone modules, wherein at least one of said bio-active peptide hormone modules exhibits at least one hormonal activity of a component peptide hormone and at least one of said bio-active peptide hormone modules imparts a desired chemical stability, conformational stability, metabolic stability, receptor interaction, protease inhibition, and/or other phannacokinetic characteristic to the hybrid polypeptide.

In a preferred embodiment, the hybrid polypeptides of the invention may have comparable or higher potency in the treatment and/or prevention of metabolic conditions and disorders, as compared to the component peptide hormones. In another embodiment, the hybrid polypeptides of the invention may have comparable or higher potency in the treatment and/or prevention of diabetes and/or diabetes-related disorders, as compared to the component peptide hormones. Alternatively, preferred hybrid polypeptides of the invention may exhibit improved ease of manufacture, stability, and/or ease of formulation, as compared to the component peptide hormones.

More particularly, the hybrid polypeptides of the present invention according to the claims will generally comprise a first bio-active peptide hormone module covalently linked to at least one additional bio-active peptide hormone module. The bio-active peptide hormone modules may be covalently linked together in any manner known in the art, including but not limited to direct amide bonds or chemical linker groups, as described in further detail below. In one embodiment, chemical linker groups may include peptide mimetics which induce or stabilize polypeptide conformation.

The first bio-activc peptide hormone module according to the claims is selected from a first component peptide hormone, and may be a peptide hormone (including native peptide hormones as well as analogs and derivatives thereof), a peptide fragment with hormonal activity (including fragments of native peptides hormones as well as analogs and derivatives thereof), or a structural motif of a peptide hormone (including native peptide hormones as well as analogs and derivatives thereof) that imparts a desired chemical stability, conformational stability, metabolic stability, receptor interaction, protease inhibition, and/or other pharmacokinetic characteristic to the hybrid polypeptide. Likewise, the additional bio-active peptide module(s) according to the claims is selected from component peptide hormones, and may be a peptide hormone (including native peptide hormones as well as analogs and derivatives thereof), a peptide fragments with hormonal activity (including fragments of native peptides hormones as well as analogs and derivatives thereof), or a structural motif of a hormone peptide (including native peptide hormones as well as analogs and derivatives thereof) that imparts a desired chemical stability, conformational stability, metabolic stability, receptor interaction, protease inhibition. and/or other pharmacokinetic characteristic to the hybrid polypeptide. In the disclosure, the first peptide hormone and the additional peptide hormone may be the same peptide hormone, may be from the same family of peptide hormones, or may be different peptide hormones, depending on the desired characteristics of the bio-active peptide hormone modules.

As used herein, the term "bio-active" refers to (1) biological activity in at least one *in vivo* hormonal pathway, or (2) modulation of the therapeutic efficacy, scope of function, duration of action, physicochemical properties, and/or other pharmacokinetic properties of such biological activity. Biological activity may be evaluated through target hormone receptor binding assays, or through metabolic studies that monitor a physiological indication, as known in the art and described herein. Modulation of the therapeutic efficacy, scope of function, duration of action, physicochemical properties, and/or other pharmacokinetic properties of such biological activity may be modified through changed in, *e.g*., chemical stability, conformational stability, metabolic stability, receptor interaction, protease inhibition, and/or other pharmacokinetic characteristics.

In the disclosure, the hybrid polypeptides retain at least about 25%, preferably about 30%, 40%, 50%, 60%, 70%, 80%, 90%, 95%, 98%, or 99% percent of the biological activity of a component peptide hormone. Preferred hybrid polypeptides are those having a potency in one of the metabolic-related assays known in the art or described herein (*e.g*., receptor binding, food intake, gastric emptying, pancreatic secretion, insulin secretion, blood glucose lowering, weight reduction, *etc*.) which is equal to or greater than the potency of component peptide hormone in that same assay. Alternatively, preferred hybrid polypeptides may exhibit improved ease of manufacture, stability, and/or case of formulation, as compared to component peptide hormones.

In another disclosure, the hybrid polypeptides retain at least about 25%, preferably about 30%, 40%, 50'%, 60%, 70%, 80%, 90%, 95%, 98%, or 99% percent of the biological activity of a native component peptide hormone with regard to the reduction of nutrient availability, the reduction of food intake, the effect of body weight gain, and/or the treatment and prevention of metabolic conditions and disorders. In yet another disclosure, the hybrid polypeptides exhibit at least about. 110%, 125%, 130%, 140%, 150%, 200%, or more of the biological activity of a native peptide hormone with regard to the reduction of nutrient availability the reduction of food intake, the effect of body weight gain, and/or the treatment and prevention of metabolic conditions and disorders. In another disclosure,the hybrid-polypeptides exhibit improved component peptide hormone receptor agonist activity.

### Component Peptides Hormones, Analogs and Derivatives

Component peptide hormones generally include peptide hormones useful in the treatment or prevention of metabolic diseases and disorders including: (a) the amylin family, including amylin, adrenomedullin ("ADM"), calcitonin ("CT"), calcitonin gene related peptide ("CGRP"), intermedin (also known as "AFP-6") and related peptides; (b) cholecystokinin ("CCK"); (c) the leptin family, including leptin and leptin-like peptides; (d) the pancreatic polypeptide family, including pancreatic polypeptide ("PP") and peptide YY (``PYY"); and (e) incretins and incretin mimetics, including: peptide hormones derived from the proglucagon gene such as: glucagon, glucagon-like peptide-1 ("GLP-1"), glucagon-like peptide 2 ("GLP-2"), and oxyntomodulin ("OXM"); and exendins such as: exendin-3, and exendin-4. As discussed above, component peptide hormones of the invention also include analogs and derivatives that retain hormonal activity of these native peptide hormones. In one embodiment, such analogs and derivatives are agonists of the target hormone receptor.

By "amylin" is meant the human peptide hormone referred to as amylin and secreted from the beta cells of the pancreas, and species variations thereof, as described in U.S. Pat. No. 5,234,906, issued Aug. 10, 1993, for "Hyperglycemic Compositions," More particularly, amylin is a 37-amino acid polypeptide hormone normally co-secreted with insulin by pancreatic beta cells in response to nutrient intake (*see, e.g.,* Koda et al., Lancet 339:1179-1180, 1992). In this sense, "amylin," "wild-type amylin," and "native amylin," i.e., unmodified amylin, are used interchangeably.

By "adrenomedullin" or "ADM" is meant the human peptide hormone and species variants thereof. More particularly, ADM is generated from a 185 amino acid preprohormone through consecutive enzymatic cleavage and amidation. This process culminates in the liberation of a 52 amino acid bioactive peptide.

By "calcitonin" or "CT" is meant the human peptide hormone and species variants thereof, including salmon calcitonin ("sCT"). More particularly, CT is a 32 amino acid peptide cleaved from a larger prohormone. It contains a single disulfide bond, which causes the amino terminus to assume the shape of a ring. Alternative splicing of the calcitonin pre-mRNA can yield a mRNA encoding calcitonin gene-related peptide; that peptide appears to function in the nervous and vascular systems. The calcitonin receptor has been cloned and shown to be a member of the seven-transmembrane, G protein-coupled receptor family.

By "calcitonin gene related peptide" or "CGRP" is meant the human peptide hormone and species variants thereof, in any physiological form.

By "intermedin" or "AFP-6" is meant the human peptide hormone and species variants thereof, in any physiological form.

By "cholecystokinin" or "CCK" is meant the human peptide hormone and species variants thereof. More particularly, CCK is a 33-amino acid sequence first identified in humans, and includes a 8-amino acid *in vivo* C-terminal fragment ("CCK-8") that has been reportedly demonstrated in pig, rat, chicken, chinchilla, dog and humans. Thus, the term CCK-33 will generally refer to human CCK(1-33), while CCK-8 (CCK(26-33)) will refer to the C-terminal octapeptide generically in both the sulfated and unsulfated unless otherwise specified. Further, pentagastrin or CCK-5 will refer to the C-terminal peptide CCK(29-33), and the CCK-4 will refer to the C-terminal tetrapeptide CCK(30-33). However, as used herein, CCK will generally refer to all naturally occurring variations of the hormone, including CCK-33, CCK-8, CCK-5, and CCK-4, in the sulfated and unsulfated form unless otherwise specified.

By "leptin" is meant the naturally occurring leptin from any species, as well as biologically active D-isoforms, or fragments of naturally occurring leptin and variants thereof, and combinations of the preceding. Leptin is the polypeptide product of the ob gene as described in the International Patent Publication No. WO 96/05309, which is incorporated herein by reference in its entirety. Putative analogs and fragments of leptin are reported in US Patent 5,521,283, U. S. Patent 5,532,336, PCT/US96/22308 and PCT/US96/01471, each of which is incorporated herein by reference in its entirety.

By "PP" is meant human pancreatic peptide polypeptide or species variants thereof, in any physiological form. Thus, the term "PP" includes both the human full length, 36 amino acid peptide as set forth in SEQ ID NO: 1, and species variations of PP, including, *e.g.,* murine, hamster, chicken, bovine, rat, and dog PP. In this sense, "PP," "wild-type PP," and "native PP," *i.e.,* unmodified PP, are used interchangeably.

By "PYY" is meant human peptide YY polypeptide or species variants thereof, in any physiological form. Thus, the term "PYY" includes both the human full length, 36 amino acid peptide, and species variations of PYY, including *e.g.,* murine, hamster, chicken, bovine, rat, and dog PYY. In this sense, "PYY" and "wild-type PYY" and "native PYY," *i.e.,* unmodified PYY, are used interchangeably. In the context of the present invention, all modifications discussed with reference to the PYY analog polypeptides of the present invention are based on the 36 amino acid sequence of native human PYY.

By "GLP-1" is meant human glucagon like peptide-1 or species variants thereof, in any physiological form. The term "GLP-1" includes human GLP-1(1-37), GLP-1(7-37), and GLP-1(7-36)amide, with reference to the full length human GLP-1(1-37), and species variations of GLP-1, including, *e.g.,* murine, hamster, chicken, bovine, rat, and dog PP. In this sense, "GLP-1," "wild-type GLP-1," and "native GLP-1," *i.e.,* unmodified GLP-1, are used interchangeably.

By "GLP-2" is meant human glucagon like peptide-2 or species variants thereof, in any physiological form. More particularly, GLP-2 is a 33 amino acid peptide, co-secreted along with GLP-1 from intestinal endocrine cells in the small and large intestine.

By "OXM" is meant human oxyntomodulin or species variants thereof in any physiological form. More particularly, OXM is a 37 amino acid peptide that contains the 29 amino acid sequence of glucagon followed by an 8 amino acid carboxyterminal extension.

By "exendin" is meant a peptide hormone found in the saliva of the Gila-monster, a lizard endogenous to Arizona, and the Mexican Beaded Lizard, as well as species variants thereof. More particularly, Exendin-3 is present in the saliva of Heloderma horridum, and exendin-4 is present in the saliva of Heloderma suspectum (Eng, J., et al., J. Biol. Chem., 265:20259-62, 1990; Eng., J., et al., J. Biol. Chem., 267:7402-05 (1992)). The exendins have some sequence similarity to several members of the glucagon-like peptide family, with the highest identity, 53%, being to GLP-1 (Goke, et al., J. Biol. Chem., 268:19650-55 (1993)). In this sense, "exendin," "wild-type exendin," and "native exendin," *i.e.,* unmodified exendin, are used interchangeably.

As used herein, an "analog" refers to a peptide whose sequence was derived from that of a base reference peptide (*e.g.,* PP, PYY, amylin, GLP-1, exendin, *etc*.), including insertions, substitutions, extensions, and/or deletions of the reference amino acid sequence, preferably having at least 50 or 55% amino acid sequence identity with the base peptide, more preferably having at least 70%, 80%, 90%, or 95% amino acid sequence identity with the base peptide. In one embodiment, such analogs may comprise conservative or non-conservative amino acid substitutions (including non-natural amino acids and L and D forms).

A "derivative" is defined as a molecule having the amino acid sequence of a native reference peptide or analog, but additionally having chemical modification of one or more of its amino acid side groups, α-carbon atoms, terminal amino group, or terminal carboxylic acid group. A chemical modification includes, but is not limited to, adding chemical moieties, creating new bonds, and removing chemical moieties. Modifications at amino acid side groups include, without limitation, acylation of lysine ε-amino groups, N-alkylation of arginine, histidine, or lysine, alkylation of glutamic or aspartic carboxylic acid groups, and deamidation of glutamine or asparagine. Modifications of the terminal amino include, without limitation, the desamino, N-lower alkyl, N-di-lower alkyl, constrained alkyls (e.g. branched, cyclic, fused, adamantyl) and N-acyl modifications. Modifications of the terminal carboxy group include, without limitation, the amide, lower alkyl amide, constrained alkyls (e.g. branched, cyclic, fused, adamantyl) alkyl, dialkyl amide, and lower alkyl ester modifications. Lower alkyl is C1-C4 alkyl. Furthermore, one or more side groups, or terminal groups, may be protected by protective groups known to the ordinarily-skilled peptide chemist. The a-carbon of an amino acid may be mono- or dimethylated.

By "agonist" is meant a compound which elicits a biological activity of native human reference peptide, preferably having a potency better than the reference peptide, or within five orders of magnitude (plus or minus) of potency compared to the reference peptide, more preferably 4, 3, 2, or 1 order of magnitude, when evaluated by art-known measures such as receptor binding/competition studies. In one disclosure, the terms refer to a compound which elicits a biological effect similar to that of native human reference peptide, for example a compound (1) having activity in the food intake, gastric emptying, pancreatic secretion, or weight loss assays similar to native human reference peptide, or (2) which binds specifically in a reference receptor assay or in a competitive binding assay with labeled reference peptide. Preferably, the agonists will bind in such assays with an affinity of greater than 1 µM, and more preferably with an affinity of greater than 1-5 nM. In another disclosure, the terms refer to a compound which elicits a biological effect in the treatment of diabetes or a diabetes related condition or disorder. Such agonists may comprise a polypeptide comprising an active fragment of a reference peptide or a small chemical molecule.

By "amino acid" and "amino acid residue" is meant natural amino acids, unnatural amino acids, and modified amino acid. Unless stated to the contrary, any reference to an amino acid, generally or specifically by name, includes reference to both the D and the L stereoisomers if their structure allow such stereoisomeric forms. Natural amino acids include alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), Lysine (Lys), methionine (Met), phenylalanine (Phe), proline (Pro), serine (Ser), threonine (Thr), tryptophan (Trp), tyrosine (Tyr) and valine (Val). Unnatural amino acids include, but are not limited to homo-lysine, homo-arginine, azetidinecarboxylic acid, 2-aminoadipic acid, 3-aminoadipic acid, beta-alanine, aminopropionic acid, 2-aminobutyric acid, 4-aminobutyric acid, 6-aminocaproic acid, 2-aminoheptanoic acid, 2aminoisobutyric acid, 3-aminoisbutyric acid, 2-aminopimelic acid, tertiary-butylglycine, 2,4-diaminoisobutyric acid, desmosine, 2,2'-diaminopimelic acid, 2,3-diaminopropionic acid, N-ethylglycine, N-ethylasparagine, homoproline, hydroxylysine, allo-hydroxylysine, 3-hydroxyproline, 4-hydroxyproline, isodesmosine, allo-isoleucine, N-methylalanine, N-methylglycine, N-methylisoleucine, N-methylpentylglycine, N-methylvaline, naphthalanine, norvaline, norleucine, ornithine, pentylglycine, pipecolic acid and thioproline. Additional unnatural amino acids include modified amino acid residues which are chemically blocked, reversibly or irreversibly, or chemically modified on their N-terminal amino group or their side chain groups, as for example, N-methylated D and L amino acids or residues wherein the side chain functional groups are chemically modified to another functional group. For example, modified amino acids include methionine sulfoxide; methionine sulfone; aspartic acid-(beta-methyl ester), a modified amino acid of aspartic acid; N-ethylglycine, a modified amino acid of glycine; or alanine carboxamide, a modified amino acid of alanine. Additional residues that can be incorporated are described in Sandberg et al., J. Med. Chem. 41: 2481-91, 1998.

As used herein: "5 Apa" means 5 amino-pentanoyl, "12 Ado" means 12-amino dodecanoyl, "PEG(8)" mean 3,6,-dioxyoctanoyl, and "PEG(13)" means 1-amino-4,7,10-tioxa-13-tridecanamine succinimoyl.

As discussed above, native component peptide hormones are known in the art, as are their analogs and derivatives. For reference, the sequences of several native component peptide hormones are provided below in Table 1.

**Table 1: Exemplary Component Peptide Hormones**

| *Seq ID* | *Description* | *Sequence* |
|---|---|---|
| 44 | Rat Amylin | KCNTATCATQRLANFLVRSSNNLGPVLPPTNVGSNTY |
| 45 | h-Amylin: | KCNTATCATQRLANFLVHSSNNFGAILSSTNVGSNTY |
| 46 | h-ADM: | YRQSMNNFQGLRSFGCRFGTCTVQKLAHQIYQFTDKDKDNVAPRSKISPQGY |
| 47 | s-CT: | CSNLSTCVLGKLSEELHKLQTYPRTNTGSGTR |
| 48 | h-CT: | CGNLSTCMLGTYTQDFNKFHTFPQTAIGVGAP |
| 49 | h-CGRP α: | ACDTATCVTHRLAGLLSRSGGVVKNNFVPTNVGSKAF |
| 50 | h-CGRP ß: | ACNTATCVTHRLAGLLSRSGGMVKSNFVPTNVGSKAF |
| 51 | h-AFP-6 (1-47) | TQAQLLRVGCVLGTCQVQNLSHRLWQLMGPAGRQDSAPVDPSSPHSY |
| 52 | h-AFP-6 (8- * 47): | VGCVLGTCQVQNLSHRLWQLMGPAGRQDSAPVDPSSPHSY |
| 53 | Mouse AFP-6 (1-47): | PHAQLLRVGCVLGTCQVQNLSHRLWQLVRPAGRRDSAPVDPSSPHSY |
| 54 | Mouse AFP-6 (8-47): | VGCVLGTCQVQNLSHRLWQLVRPAGRRDSAPVDPSSPHSY |
| 55 | CCK-8 -sulfated: | DY(SO₃)MGWMDF |
| 56 | h-Leptin: | |
| 57 | hPYY: | YPIKPEAPGEDASPEELNRYYASLRHYLNLVTRQRY |
| 58 | hPYY(3-36) | IKPEAPGEDASPEELNRYYASLRHYLNLVTRQRY |
| 59 | hGLP-1 (1-37): | HDEFERHAEGTFTSDVSSTLEGQAALEFIAWLVKGRG |
| 60 | Frog GLP-1: | HAEGTYTNDVTEYLEEKAAKEFIEWLIKGKPKKIRYS-OH; HAEGTFTSDVTQQLDEKAAKEFIDWLINGGPSKEIIS-OH |
| 61 | h-GLP-1 (7-36): | HAEGTFTSDVSSYLEGQAALEFIAWLVKGR |
| 62 | h-GLP-2 | HADGSFSDEMNTILDNLAARDFINWLIETKITD |
| 63 | Frog GLP-2: | HAEGTFTNDMTNYLEEKAAKEFVGWLIKGRP-OH |
| 64 | OXM: | HSQGTFTSDYSKYLDSRRAQDFVQWLMNTKRNRNNIA |
| 65 | Exendin-3: | HSDGTFTSDL SKQMEEEAVR LFIEWLKNGG PSSGAPPPS |
| 66 | Exendin-4 | HGEGTFTSDLSKQMEEEAVRLFIEWLICNGGPSSGAPPPS |

These peptides are generally C-terminally amidated when expressed physiologically, but need not be for the purposes of the instant invention. In other words, the C-terminus of these peptides, as well as the hybrid polypeptides of the present invention, may have a free -OH or -NH₂ group. These peptides may also have other post-translational modifications. One skilled in the art will appreciate that the hybrid polypeptides of the present invention may also be constructed with an N-terminal methionine residue.

The analogs of the above component peptide hormones are known in the art, but generally include modifications such as substitutions, deletions, and insertions to the amino acid sequence of such component peptide hormones, and any combination thereof. The substitutions, insertions and deletions may be at the N-terminal or C-terminal end, or may be at internal portions of the component peptide hormone. In a preferred aspect, analogs of the component peptide hormones of the invention include one or more modifications of a "non-essential" amino acid residue. In the context of the invention, a "non-essential" amino acid residue is a residue that can be altered, *i.e*., deleted or substituted, in the native human amino acid sequence of the fragment, *e.g*., the component peptide hormone fragment, without abolishing or substantially reducing the component peptide hormone receptor agonist activity of the resulting analog.

Preferred substitutions include conserved amino acid substitutions. A "conservative amino acid substitution" is one in which the amino acid residue is replaced with an amino acid residue having a similar side chain, or physicochemical characteristics (*e.g*., electrostatic, hydrogen bonding, isosteric, hydrophobic features). Families of amino acid residues having similar side chains are known in the art. These families include amino acids with basic side chains (*e.g*., lysine, arginine, histidine), acidic side chains (*e.g*., aspartic acid, glutamic acid), uncharged polar side chains (*e.g*., glycine; asparagine, glutamine, serine, threonine, tyrosine, methionine, cysteine), nonpolar side chains (*e.g*., alanine, valine, leucine, isoleucine, proline, phenylalanine, tryptophan), ß-branched side chains (*e.g*., threonine, valine, isoleucine) and aromatic side chains (*e.g*., tyrosine, phenylalanine, tryptophan, histidine).

The present invention also relates to derivatives of the component peptide hormones, including component peptide hormones and analogs thereof conjugated to one or more water soluble polymer molecules, such as polyethylene glycol ("PEG") or fatty acid chains of various lengths (*e.g*., stearyl, palmitoyl, octanoyl, etc.), or by the addition of polyamino acids, such as poly-his, poly-arg, poly-lys, and poly-ala. Modifications to the component peptide hormones or analogs thereof can also include small molecule substituents, such as short alkyls and constrained alkyls (*e.g.*, branched, cyclic, fused, adamantyl), and aromatic groups. The water soluble polymer molecules will preferably have a molecular weight ranging from about 500 to about 20,000 Daltons.

Such polymer-conjugations and small molecule substituent modifications may occur singularly at the N- or C-terminus or at the side chains of amino acid residues within the sequence of the hybrid polypeptides. Alternatively, there may be multiple sites of derivatization along the hybrid polypeptide. Substitution of one or more amino acids with lysine, aspartic acid, glutamic acid, or cysteine may provide additional sites for derivatization. *See, e.g.,* U.S. Patent Nos. 5,824,784 and 5,824,778. Preferably, the hybrid polypeptides may be conjugated to one, two, or three polymer molecules.

The water soluble polymer molecules are preferably lined to an amino, carboxyl, or thiol group, and may be linked by N or C terminus, or at the side chains of lysine, aspartic acid, glutamic acid, or cysteine. Alternatively, the water soluble polymer molecules may be linked with diamine and dicarboxylic groups. In a preferred embodiment, the hybrid polypeptides of the invention are conjugated to one, two, or three PEG molecules through an epsilon amino group on a lysine amino acid.

Derivatives of the disclosure also include component peptide hormones or analogs with chemical alterations to one or more amino acid residues. Such chemical alterations include amidation, glycosylation, acylation, sulfation, phosphorylation, acetylation, and cyclization. The chemical alterations may occur singularly at the N- or C-terminus or at the side chains of amino acid residues within the sequence of the PPF hybrid polypeptides. In one disclosure, the C-terminus of these peptides may have a free -OH or -NH₂ group. In another disclosure, the N-terminal end may be capped with an isobutyloxycarbonyl group, an isopropyloxycarbonyl group, an n-butyloxycarbonyl group, an ethoxycarbonyl group, an isocaproyl group (isocap), an octanyl group, an octyl glycine group (G(Oct)), or an 8-aminooctanic acid group. In a preferred disclosure, cyclization can be through the formation of disulfide bridges. Alternatively, there may be multiple sites of chemical alteration along the hybrid polypeptide.

### The Amylin Family

As discussed above, component peptide hormones useful in the present invention include amylin family peptide hormones including amylin, adrenomedullin ("ADM"), calcitonin ("CT"), calcitonin gene related peptide ("CGRP"), intermedin (also known as "AFP-6") and related peptides. Native amylin family peptide hormones are known in art, as are functional peptide analogs and derivatives. Certain preferred native peptides, peptide analogs and derivatives are described herein, however it should be recognized that any known amylin family peptides that exhibit hormonal activity known in the art may be used in conjunction with the present invention.

Any amylin analog or derivative known in the art may be used in conjunction with the present invention according to the claims. In one embodiment, the amylin analogs and derivatives have at least one hormonal activity of native amylin. In certain embodiments, the amylin analogs are agonists of a receptor which native amylin is capable of specifically binding. Preferred amylin analogs and derivatives include those described in US 2003/0026812 A1.

Exemplary amylin analogs include:

| SEQ ID: | |
|---|---|
| 67 | ^{25,28,29}Pro-h-amylin (pramlintide) |
| 68 | des-¹Lys-h-amylin |
| 9 | ²⁵Pro,²⁶Val,^{28,29}Pro-h-amylin |
| 70 | ¹⁸Arg,^{25,28}Pro-h-amylin |
| 71 | des-¹Lys,¹⁸Arg,25,²⁸Pro-h-amylin |
| 72 | ¹⁸Arg,^{25,28,29}Pro-h-amylin |
| 73 | des-¹Lys,¹⁸Arg,^{25,28,29}Pro-h-amylin |
| 74 | des-¹,Lys^{25,28,29}Pro-h-amylin |
| 73 | ²⁵Pro,²⁶Val,^{28,29}Pro-h-amylin |
| 76 | ²⁸Pro-h-amylin, 2,7-Cyclo-[²Asp,⁷Lys]-h-amylin |
| 77 | ²⁻³⁷h-amylin |
| 78 | ¹Ala-h-amylin |
| 79 | ²Ala-h-amylin |
| 80 | ^{2,7}Ala-h-amylin |
| 81 | ¹Ser-h-amylin |
| 82 | ²⁹Pro-h-amylin |
| 83 | ^{23,28}Pro-h-amylin |
| 84 | des-¹Lys,^{25,28}Pro-h-amylin |
| 85 | ²³Leu,²⁵Pro,²⁶Val,^{28,29}Pro-h-amylin |
| 86 | ²³Leu²⁵Pro²⁶Val²⁸Pro-h-amylin |
| 87 | des-¹Lys,²³-Leu,²⁵Pro,²⁶Val,¹⁸Pro-h-amylin |
| 88 | ¹⁸Arg,²³Leu,²⁵Pro,²⁶Val,²⁸Pro-h-amylin |
| 89 | ¹⁸Arg,²³Leu,^{25,28,29}Pro-h-amylin |
| 90 | ¹⁸Arg²³Leu,^{25,28}Pro-h-amylin |
| 91 | ¹⁷Ile,²³Leu,^{25,26,29}Pro-h-amylin |
| 92 | ¹⁷Ile,^{25,28,29}Pro-h-amylin |
| 93 | des-¹Lys,¹⁷Ile,²³Leu,^{25,28,29}Pro-h-amylin |
| 94 | ¹⁷Ile,¹⁸Arg,²³Leu-h-amylin |
| 95 | ¹⁷Ile,¹⁸Arg,²³Leu,²⁶Val,²⁹Pro-h-amylin |
| 96 | ¹⁷Ile,¹⁸Arg,²³Leu,²⁵Pro,²⁶Val,^{28,29}Pro-h-amylin, |
| 97 | ¹³Thr,²¹His,²³Leu,²⁶Ala,²⁸Leu,²⁹Pro,³¹Asp-h-amylin |
| 98 | ¹³Thr,²¹His,²³Leu,²⁶Ala,²⁹Pro,³¹Asp-h-amylin |
| 99 | des-¹Lys,¹³Thr,²¹His,²³Leu²⁶,Ala,²⁸Pro,³¹Asp-h-amylin |
| 100 | ¹³Thr,¹⁸Arg,²¹His,²³Leu,²⁶Ala, ²⁹Pro,³¹Asp-h-amylin |
| 101 | ¹³Thr,¹⁸Arg,²¹His,²³Leu,²⁶Ala, ^{28,29}Pro,³¹Asp-h-amylin |
| 102 | ¹³Thr,¹⁸Arg,²¹His,²³Leu,²⁶Ala, ^{28,29}Pro,³¹Asp-h-amylin |

As known in the art, such amylin analogs are preferably amidated, but within the context of the present invention, may optionally be in the acid form unless otherwise specified.

Any ADM analog or derivative known in the art may be used in conjunction with the present invention. In one disclosure, the ADM analogs and derivatives have at least one hormonal activity of native ADM. In certain disclosures, the ADM analogs are agonists of a receptor which native ADM is capable of specifically binding.

Any CT analog or derivative known in the art may be used in conjunction with the present invention. In one disclosure, the CT analogs and derivatives have at least one hormonal activity of native CT. In certain disclosures, the CT analogs are agonists of a receptor which native CT is capable of specifically binding. Preferred CT analogs and derivatives include those described in U.S. Patent Nos. 4,652,627; 4,606,856; 4,604,238; 4,597,900; 4,537,716; 4,497,731; 4,495,097; 4,444,981; 4,414,149; 4,401,593; and 4,397,780,

Exemplary CT analogs include:

| SEQ ID: | |
|---|---|
| 103 | ⁸Gly-CT |
| 104 | ²²Leu-CT |
| 105 | ²Gly,³Ser,⁸Gly,²²des-Tyr-CT |
| 106 | ¹⁴Gln-sCT, |
| 107 | ¹⁸Arg-sCT, |
| 108 | ^{11,18}Arg-sCT, |
| 109 | ¹⁴Gln,¹⁸Arg-sCT, |
| 110 | ¹⁴Gln,^{11,18}Arg-sCT |

As known in the art, such CT analogs are preferably amidated, but within the context of the present invention, may optionally be in the acid form unless otherwise specified.

Any CGRP analog or derivative known in the art may be used in conjunction with the present invention. In one disclosure, the CGRP analogs and derivatives have at least one hormonal activity of native CGRP. In certain disclosures, the CGRP analogs are agonists of a receptor which native CGRP is capable of specifically binding. Preferred CGRP analogs and derivatives include those described in U.S. Patent Nos. 4,697,002; and 4,687,839,

Exemplary CGRP analogs include:

| SEQ ID: | |
|---|---|
| 111 | ³⁶D-Ser-CGRP |
| 112 | ³⁶D-Thr-CGRP |
| 113 | ³⁶D-Asp-CGRP |
| 114 | ³⁶D-Asn-CGRP |
| 115 | ³⁶Scr-CGRP |
| 116 | ³⁶Ser-CGRP |
| 117 | ³⁶Asp-CGRP |
| 118 | ^{36j}Thr-CGRP |
| 119 | ³⁶Asn-CGRP |

Any AFP-6 analog or derivative known in the art may be used in conjunction with the present invention. In one disclosure, the AFP-6 analogs and derivatives have at least one hormonal activity of native AFP-6. In certain disclosures, the AFP-6 analogs are agonists of a receptor which native AFP-6 is capable of specifically binding. Preferred AFP-6 analogs and derivatives include those described in WO 2003/022304.

Exemplary AFP-6 analogs include:

| SEQ ID: | |
|---|---|
| 120 | TQAQLLRVGCGNLSTCQVQNLSHRLWQLMGPAGRQDSAPVDPSSPHSY |
| 121 | TQAQLLRVGCDTATCQVQNLSHRLWQLMGPAGRQDSAPVDPSSPHSY |
| 122 | TQAQLLRVGMVLGTMQVQNLSHRLWQLMGPAGRQDSAPVDPSSPHSY |
| 123 | TQAQLLRVGCVLGTCQVQNLSHRLWQLMGPAGRQDSAPVEPSSPHSY |
| 124 | TQAQLLRVGCVLGTCQVQNLSHRLWQLMGPAGRQESAPVEPSSPHSY |
| 125 | TQAQLLRVGCVLGTCQVQNLSHRLWQL----RQDSAPVDPSSPHSY |
| 126 | TQAQLLRVGCVLGTCQVQNLSHRLWQL----DSAPVDPSSPHSY |
| 127 | RVGCVLGTCQVQNLSHRLWQLMGPAGRQDSAPVDPSSPHSY |
| 128 | VGCVLGTCQVQNLSHRLWQLMGPAGRQDSAPVEPSSPHSY |
| 129 | VGCVLGTCQVQNLSHRLWQL----RQDSAPVEPSSPHSY |
| 130 | GCVLGTCQVQNLSHRLWQLMGPAGRQDSAPVDPSSPHSY |
| 131 | GCNTATCQVQNLSHRLWQL----RQDSAPVDPSSPHSY |
| 132 | GCNTATCQVQNLSHRLWQL----RQDSAPVEPSSPHSY |
| 133 | GCSNLSTCQVQNLSHRLWQL---RQDSAPVEPSSPHSY |
| 134 | GCGNLSTCQVQNLSHRLWQL----RQDSAPVEPSSPHSY |
| 135 | GCVLGTCQVQNLSHRLWQL----RQESAPVEPSSPHSY |
| 136 | CVLGTCQVQNLSHRLWQLMGPAGRQDSAPVDPSSPHSY |
| 137 | QVQNLSHRLWQLMGPAGRQDSAPVDPSSPHSY |
| 138 | VQNLSHRLWQLMGPAGRQDSAPVDPSSPHSY |
| 139 | VQNLSHRL----QLMGPAGRQDSAPVDPSSPHSY |
| 140 | GTMQVQNLSHRLWQL----RQDSAPVEPSSPHSY |

As known in the art, such AFP-6 analogs are preferably amidated, but within the context of the present invention, may optionally be in the acid form unless otherwise specified.

### The CCK Family

CCKs, including hCCK and species variants, and various analogs thereof are known in the art. Generally, CCK has a 33-amino acid sequence first identified in humans, and includes a 8-amino acid *in vivo* C-terminal fragment ("CCK-8") that has been reportedly demonstrated in pig, rat, chicken, chinchilla, dog and humans. Other species variants include a 39-amino acid sequence found in pig, dog and guinea pig, and a 58-amino acid found in cat, dog and humans, and a 47-amino acid sequences homologous to both CCK and gastrin. The C-terminal sulfated octapeptide sequence (CCK-8) is relatively conserved across species, and may be the minimum sequence for biological activity in the periphery of rodents. Thus, the term CCK-33 will generally refer to human CCK(1-33), while CCK-8 (CCK(26-33)) will refer to the C-terminal octapeptide generically in both the sulfated and unsulfated unless otherwise specified. Further, pentagastrin or CCK-5 will refer to the C-terminal peptide CCK(29-33), and the CCK-4 will refer to the C-terminal tetrapeptide CCK(30-33).

The type A receptor subtype (CCK_{A}) has been reported to be selective for the sulfated octapeptide. The Type B receptor subtype (CCK_{B}) has been identified throughout the brain and in the stomach, and reportedly does not require sulfation or all eight amino acids.

Various *in vivo* and *in vitro* screening methods for CCK analogs are known in the art. Examples include *in vivo* assays involving the contraction of the dog or guinea pig gallbladder after rapid intravenous injection of the compound to be tested for CCK-like activity, and *in vitro* assays measuring using strips of rabbit gallbladder. *See* Walsh, "Gastrointestinal Hormones", In Physiology of the Gastrointestinal Tract (3d ed. 1994; Raven Press, New York).

Certain preferred CCKs and CCK analogs with CCK activity include:

| SEQ ID: | |
|---|---|
| 141 | DY(SO₃H)MGWMDF |
| 142 | DYMGWMDF |
| 143 | MGWMDF |
| 144 | GWMDF |
| 145 | WMDF |
| 146 | KDY(SO₃H)MGWMDF |
| 147 | DYMGWMDF |
| 148 | KMGWMDF |
| 149 | KOWMDF |
| 150 | KWMDF |

As known in the art, such CCK peptides are preferably amidated, but within the context of the present disclosure, may optionally be in the acid form unless otherwise specified.

### The Leptin Family

Component peptide hormones useful in the present disclosure also include leptin family peptide hormones. Native leptin family peptide hormones are known in art, as are functional peptide analogs and derivatives. Certain preferred native peptides, peptide analogs and derivatives are described herein, however it should be recognized that any known amylin family peptides that exhibit hormonal activity known in the art may be used in conjunction with the present disclosure.

Any leptin analog or derivative known in the art may be used in conjunction with the present disclosure. In one disclosure, the leptin analogs and derivatives have at least one hormonal activity of native leptin. In certain disclosures, the leptin analogs are agonists of a receptor which native leptin is capable of specifically binding. Preferred leptin analogs and derivatives include those described in, e.g., WO 2004/039832, WO 98/55139, WO 98/12224, and WO 97/02004.

Exemplary leptin analogs include those where the amino acid at position 43 is substituted with Asp or Glu; position 48 is substituted Ala; position 49 is substituted with Glu, or absent; position 75 is substituted with Ala; position 89 is substituted with Leu; position 93 is substituted with Asp or Glu; position 98 is substituted with Ala; position 117 is substituted with Ser, position 139 is substituted with Leu, position 167 is substituted with Ser, and any combination thereof.

Certain preferred CCKs and CCK analogs with CCK activity include:

| SEQ ID: | |
|---|---|
| 151 | ⁴³Asp-leptin |
| 152 | ⁴³Glu-leptin |
| 153 | ⁴⁸Ala-leptin |
| 154 | ⁴⁹Glu-leptin |
| 155 | ⁴⁹Des-AA-leptin |
| 156 | ⁷⁵Ala-leptin |
| 157 | ⁸⁹Leu-leptin |
| 158 | ⁹³Asp-leptin |
| 159 | ⁹³Glu-leptin |
| 160 | ⁹⁸Ala-leptin |
| 161 | ¹¹⁷Ser-leptin |
| 162 | ¹³⁹Leu-leptin |
| 163 | ¹⁶⁷Ser-leptin |
| 164 | ⁴³Asp, ⁴⁹Glu-leptin |
| 165 | ⁴³Asp,⁷⁵Ala-leptin |
| 166 | ⁸⁹Leu,¹¹⁷Ser-leptin |
| 167 | ⁹³Glu,¹⁶7Ser-leptin |

### The PPF Family

Component peptide hormones useful in the present disclosure also include PPF peptide hormones, including PP and PYY. Native PPF peptide hormones are known in art, as are functional peptide analogs and derivatives. Certain preferred native peptides, peptide analogs and derivatives are described herein, however it should be recognized that any known amylin family peptides that exhibit hormonal activity known in the art may be used in conjunction with the present disclosure.

Any PPF analog or derivative known in the art may be used in conjunction with the present disclosure. In one disclosure, the PPF analogs and derivatives have at least one hormonal activity of a native PPF polypeptide. In certain disclosures, the PPF analogs are agonists of a receptor which native PPF polypeptide is capable of specifically binding. Preferred PPF analogs and derivatives include those described in WO 03/026591 and WO 03/057235,

hi one disclosure, preferred PPF analogs and derivatives that exhibit at least one PPF hormonal activity generally comprise at least two PYY motifs including a polyproline motif and C-terminal tail motif. Such analogs are generally described in U.S. Provisional Application No. 60/543,406 filed February 11, 2004,

Other preferred PPF analogs are disclosed in PCT/US05/[XXXXX], entitled "Pancreatic Polypeptide Family Motifs and Polypeptides Comprising the Same", Attorney Docket 18528.832, filed concurrently herewith. By way of background, research has suggested that the differences in Y receptor binding affinities are correlated with secondary and tertiary structural differences. *See, e.g.,* Keire et al., Biochemistry 2000, 39, 9935-9942. Native porcine PYY has been characterized as including two C-terminal helical segments from residues 17 to 22 and 25 to 33 separated by a kink at residues 23, 24, and 25, a turn centered around residues 12-14, and the N-terminus folded near residues 30 and 31. Further, full-length porcine PYY has been characterized as including the PP fold, stabilized by hydrophobic interactions among residues in the N- and C- termini. *See id.*

A "PYY motif" is generally a structural component, primary, secondary, or tertiary, of a native PP family polypeptide that is critical to biological activity, i.e., biological activity is substantially decreased in the absence or disturbance of the motif. Preferred PYY motifs include the N-terminal polyproline type II motif of a native PP family polypeptide, the type II β-turn motif of native PP family polypeptide, the α-helical motif at the C-terminal end of native PP family polypeptide, and the C-terminal tail motif of native PP family polypeptide. More particularly, in the N-terminal polyproline region, amino acids corresponding to residues 5 and 8 of a native PP family polypeptide are generally conserved as a proline. The type II β-turn motif will generally include amino acids corresponding to residues 12-14 of a native PP family polypeptide. The α-helical motif can generally extend from amino acids corresponding to approximately residue 14 of a native PP family polypeptide to any point up to and including the C-terminal end, so long as the α-helical motif includes a sufficient number of amino acid residues such that an α-helical turn is formed in solution. The α-helical motif can also include amino acid substitutions, insertions and deletions to the native PP family sequence, so long as the α-helical turn is still formed in solution. The C-terminal tail motif generally includes amino acids corresponding to approximately the last 10 residues of a native PP family polypeptide, more preferably the last 7, 6, or 5 residues of a native PP family polypeptide, and more preferably amino acid residues 32-35.

Preferred PYY analogs include those with internal deletions, insertions, and substitutions in areas of the PYY molecule not corresponding to the polyproline motif and/or the C-terminal tail motif. For instance, internal deletions at positions 4, 6, 7, 9, or 10 are envisioned.

### Incretins and Incretin Mimetics

Component peptide hormones useful in the present disclosure also include GLP-1 peptide hormones. Native GLP-1 peptide hormones, including GLP-1(1-37), GLP-1(7-37), and GLP-1 (7-36)amide, are known in art, as are functional peptide analogs and derivatives. As used herein, GLP-1 refers to all native forms of GLP-1 peptide hormones. Certain preferred native peptides, peptide analogs and derivatives are described herein, however it should be recognized that any known GLP-1 peptides that exhibit hormonal activity known in the art may be used in conjunction with the present disclosure.

Any GLP-1 peptide analog or derivative known in the art may be used in conjunction with the present disclosure. In one disclosure, the GLP-1 peptide analogs and derivatives have at least one hormonal activity of a native GLP-1 peptide. In certain disclosures, the GLP-1 peptide analogs are agonists of a receptor which a native GLP-1 peptide is capable of specifically binding. Preferred GLP-1 peptide analogs and derivatives include those described in, *e.g.,* WO 91/11457,

GLP-1 analogs known in the art include:

| SEQ ID: | |
|---|---|
| 168 | ⁹Gln-GLP-1(7-37) |
| 169 | D-⁹Gln -GLP-1(7-37) |
| 170 | ¹⁶Thr-¹⁸Lys-GLP-1(7-37) |
| 171 | ¹⁸Lys-GLP-1(7-37) |
| 172 | ⁸Gly-GLP-1(7-36) |
| 173 | ⁹Gln-GLP-1(7-37) |
| 174 | D-⁹Gln-GLP-1(7-37) |
| 175 | acetyl-⁹Lys-GLP-1(7-37) |
| 176 | ⁹Thr-GLP-1(7-37) |
| 177 | D-⁹Thr-GLP-1(7-37) |
| 178 | ⁹Asn-GLP-1(7-37) |
| 179 | D-⁹Asn-GLP-1(7-37) |
| 180 | ²²Ser²³Arg²⁴Arg²⁶Gln-GLP-1(7-37) |
| 181 | ¹⁶Thr¹⁸Lys-GLP-1(7-37) |
| 182 | ¹⁸Lys-GLP-1(7-37) |
| 183 | ²³Arg-GLP-1(7-37) |
| 184 | ²⁴Arg-GLP-1(7-37) |

As known in the art, such GLP-1 analogs may preferably be amidated, but within the context of the present disclosure may optionally be in the acid form unless otherwise specified.

Other GLP-1 analogs and derivatives are disclosed in U.S. Pat. No. 5,545,618.

A preferred group of GLP-1 analogs and derivatives include those disclosed in U.S. Patent No. 6,747,006,

The use in the present disclosure of a molecule described in U.S. Pat. No. 5,188,666, is also contemplated. Another group of molecules for use in the present disclosure includes compounds described in U.S. Pat. No. 5,512,549.

Another preferred group of GLP-1 compounds for use in the present disclosure is disclosed in WO 91/11457.

Component peptide hormones useful in the present disclosure also include GLP-2 peptide hormones. Native GLP-2 peptide hormones, e.g., rat GLP-2 and its homologous including ox GLP-2, porcine GLP-2, degu GLP-2, bovine GLP-2, guinea pig GLP-2, hamster GLP-2, human GLP-2, rainbow trout GLP-2, and chicken GLP-2, are known in art, as are functional peptide analogs and derivatives. Certain preferred native peptides, peptide analogs and derivatives are described herein, however it should be recognized that any known GLP-2 peptides that exhibit hormonal activity known in the art may be used in conjunction with the present disclosure.

Any GLP-2 peptide analog or derivative known in the art may be used in conjunction with the present disclosure. In one disclosure, the GLP-2 peptide analogs and derivatives have at least one hormonal activity of a native GLP-2 peptide. In certain disclosures, the GLP-2 peptide analogs are agonists of a receptor which a native GLP-2 peptide is capable of specifically binding. Preferred GLP-2 peptide analogs and derivatives include those described in, *e.g*., U.S. Ser. No. 08/669,791 and PCT Application PCT/CA97/00252,

Specific GLP-2 analogs known in the art include: rat or human GLP-2 altered at position 2 to confer DPP-IV resistance by substituting a Gly for an Ala.

Component peptide hormones useful in the present disclosure also include oxyntomodulin (OXM) peptide hormones. Native OXM peptide hormones are known in art, as are functional peptide analogs and derivatives. Certain preferred native peptides, peptide analogs and derivatives are described herein, however it should be recognized that any known OXM peptides that exhibit hormonal activity known in the art may be used in conjunction with the present disclosure.

Any OXM peptide analog or derivative known in the art may be used in conjunction with the present disclosure In one disclosure, the OXM peptide analogs and derivatives have at least one hormonal activity of a native OXM peptide. In certain disclosures, the OXM peptide analogs are agonists of a receptor which a native OXM peptide is capable of specifically binding.

Component peptide hormones useful in the present invention also include exendin peptide hormones. Native exendin peptide hormones are known in art, as are functional peptide analogs and derivatives. Certain preferred native peptides, peptide analogs and derivatives are described herein, however it should be recognized that any known exendin peptides that exhibit hormonal activity known in the art may be used in conjunction with the present invention.

Any exendin peptide analog or derivative known in the art may be used in conjunction with the present invention according to the claims, in the embodiment, the exendin peptide analogs and derivatives have at least one hormonal activity of a native exendin peptide. In certain embodiments, the exendin peptide analogs are agonists of a receptor which a native exendin peptide is capable of specifically binding.

Preferred exendin analogs include:

| SEQ ID: | |
|---|---|
| 185 | ¹⁴Leu,²⁵Phe-exendin-4 |
| 186 | ⁵Ala,¹⁴Leu,²⁵Phe-exendin-4 |
| 187 | ¹⁴Leu,²²Ala,²⁵Phe-exendin-4 |

As known in the art, such exendin analogs are preferably amidated, but within the context of the present invention, may optionally be in the acid form unless otherwise specified.

Additional exemplary exendin analogs and derivatives are described in PCT Application Serial No. PCT/US98/16387 filed Aug. 6, 1998, entitled "Novel Exendin Agonist Compounds," which claims the benefit of U.S. patent application Ser. No. 60/055,404, filed Aug. 8, 1997. Other exendin analogs and derivatives are described in PCT Application Serial No. PCT/US98/24210, filed Nov. 13, 1998, entitled "Novel Exendin Agonist Compounds," which claims the benefit of U.S. Provisional Application No. 60/065,442 filed Nov. 14, 1997,

Still other exendin analogs and derivatives are described in PCT Application Serial No. PCT/US98/24273, filed Nov. 13, 1998, entitled "Novel Exendin Agonist Compounds," which claims the benefit of U.S. Provisional Application No. 60/066,029 filed Nov. 14, 1997.

- Still other exendin analogs and derivatives are described in PCT Application Serial No. PCT/US97/14199, filed Aug. 8, 1997, entitled "Methods for Regulating Gastrointestinal Activity," which is a continuation-in-part of U.S. patent application Ser. No. 08/694,954 filed Aug. 8, 1996,

Still other exendin analogs and derivatives are described in PCT Application Serial No. PCT/US98/00449, filed Jan. 7, 1998, entitled "Use of Exendins and Agonists Thereof for the Reduction of Food Intake," which claims priority to U.S. Provisional Application No. 60/034,905 filed Jan. 7, 1997.

Yet other exendin analogs and derivatives are described in US 2004/0209803 A1, filed December 19, 2003, entitled "Compositions for the Treatment and Prevention of Neuropathy."

### Bio-Active Peptide Hormone Modules

As discussed above, the hybrid polypeptides of the present invention according to the claims comprise at least two bio-active peptide hormone modules covalently linked together. The bio-active peptide hormone modules may be: (a) native component peptide hormones, (b) analogs or derivatives of native component peptide hormones that retain hormonal activity, (c) fragments of native component peptide hormones that retain hormonal activity, (d) fragments of analogs or derivatives of native component peptide hormones that retain hormonal activity, (e) structural motifs of native component peptide hormones that impart a desired chemical stability, conformational stability, metabolic stability, receptor interaction, protease inhibition, and/or other pharmacokinetic characteristic to the hybrid polypeptide; or (f) structural motifs of analogs or derivatives of native component peptide hormones that impart a desired chemical stability, conformational stability, metabolic stability, receptor interaction, protease inhibition, and/or other pharmacokinetic characteristic to the hybrid polypeptide. The structural motifs of (e) and (f) will collectively be referred to herein as "peptidic enhancers".

Preferred bio-active peptide hormone modules of the disclosure include native peptide hormones selected from: amylin, ADM, CT, CGRP, intermedin, CCK(1-33), CCK-8, leptin, PYY(1-36), PYY(3-36), GLP-1(1-37), GLP-1(7-37), GLP-1(7-36), GLP-2, OXM, exendin-3, and exendin-4.

Other preferred bio-active peptide hormone module of the disclosure include analogs and derivatives of a component peptide hormone selected from: amylin, ADM, CT, CGRP, intermedin, CCK, leptin, PYY(1-36), PYY(3-36), GLP-1(1-37), GLP-1(7-37), GLP-1(7-36), GLP-2, OXM, exendin-3, and exendin-4, wherein the analog or derivative exhibits at least one hormonal activity of the component peptide hormone. The analog may comprise one or more insertions, deletions, or substitutions of the amino acid sequence of the component peptide hormone, and the derivative may comprise one or more chemical modifications of an amino acid residue of an analog or component peptide hormone, as described more fully herein and known in the art.

More specifically, analogs and derivatives may be selected from any described above and/or known in the art. Particularly preferred analogs and derivatives that exhibit at least one hormonal activity useful as bio-active peptide hormone modules of the disclosure include the following:

| | |
|---|---|
| Amylin: | ²Ala-h-amylin, ^{2,7}Ala-h-amylin, ²⁸Pro-h-amylin, ^{25,28}Pro-h-amylin, ^{25,28,29}Pro-h-amylin, ²⁵Pro^{,26}Val, ^{28,29}Pro-h-amylin^{,18}Arg, ^{25,28,}Pro-h-amylin, ¹⁸Arg, ^{25,28,29}Pro-h-amylin, ²⁵Pro, ²⁶Val, ^{28,92}Pro-h-amylin, ¹⁸Arg, ²³Leu, ²³Leu, ¹⁸Arg, ²³Leu, ^{25,28,}Pro-h-amylin, and 2,7-Cyclo-[²Asp, 7Lys]-h-amylin |
| CT: | ¹⁴Gln-sCT, ¹⁸Arg-sCT, ^{11,18}Arg-sCT, ¹⁴Gln,¹⁸Arg-sCT, ¹⁴Gln,^{11,18}Arg-sCT |
| CGRP: | ³⁶D-Ser-CGRP, ³⁶D-Trh-CGRP ³⁶D-Asp-CGRP, ³⁶D-Asn-CGRP, ³⁶Ser-CGRP, ³⁶Hse-CGRP, ³⁶Asp-CGRP, ³⁶Thr-CGRP, ³⁶Asn-CGRP |
| AFP-6: | TQAQLLRVGCGNLSTCQVQNLSHRLWQLMGPAGRQDSAPVDPSSPHSY, TQAQLLRVGCDTATCQVQNLSHRLWQLMGPAGRQDSAPVDPSSPHSY, TQAQLLRVGMVLGTMQVQNLSHRLWQLMGPAGRQDSAPVDPSSPHSY, TQAQLLRVGCVLGTCQVQNLSHRLWQLMGPAGRQDSAPVEPSSPHSY, TQAQLLRVGCVLGTCQVQNLSHRLWQLMGPAGRQESAPVEPSSPHSY, |
| CCK: | DY(OSO₃H)MGWMDF, DYMGWMDF, MGWMDF, GWMDF, WMDF, KDY(OSO₃H)MGWMDF, KDYMGWMDF, kMDWMDF, KGWMDF, KWMDF |
| Leptin: | ⁴³Asp-leptin, ⁴³Glu-leptin, ⁴⁸Ala-leptin, ⁴⁹Des-AA-leptin, ⁷⁵Ala-leptin, ⁸⁹Leu-leptin, ⁹³Glu-leptin, ⁹³Glu-leptin, ⁹⁸Ala-leptin, ¹³⁹Leu-leptin, |
| PYY: | ³Leu-PYY, ³Val-PYY, ⁴Arg-PYY, ⁴Gin-PYY, ⁴Asn-PYY, ²⁵Lys-PYY, ³⁴Pro-PYY, ³⁴His-PYY, ^{1,36}Tyr-PYY, ¹³Pro¹⁴Ala-PYY, ³¹Leu³⁴Pro-PYY, des-AA-⁴-PYY |
| GLP-1 | ⁹Gln-GLP-1(7-37), D-⁹Gln-GLP-1(7-37), ¹⁶Thr-¹⁸Lys GLP-1(7-37), ¹⁸Lys-GLP-1(7-37), ⁸Gly-GLP-1(7-36), ⁹Gln-GPL-1(7-37), D-⁹Gln-GPL-1(7-37), acetyl-⁹Lys-GPL-1(7-37), ⁹Trh-GPL-1(7-37), D-⁹Thr-GPL-1(7-37),⁹Asn-GPL-1(7-37), ⁹AsnGPL-1(7-37), ²²Ser²³Arg²⁴Arg²⁶Gln-GPL-1(7-37), ¹⁶Thr¹⁸Lys-GPL-1(7-37), ¹⁸Lys-GPL-1(7-37), ²³Arg-GPL-1(7-37), ²⁴ArgGPL-1(7-37) |
| Exendin | ¹⁴Leu,²⁵Phe-exendin-4, ¹⁴Leu, ²⁵Phe-exendin-4, ⁵Ala, ¹⁴Leu, ²⁵Phe-exendin-4, and ¹⁴Leu, ²²Ala, ²⁵phe-exendin-4 |

As known in the art, such peptide compounds may preferably be amidated, but within the context of the present disclosure, may optionally be in the acid form unless otherwise specified.

Still other preferred bioactive peptide hormone modules include fragments of a component peptide hormone selected from: amylin, ADM, CT, CGRP, intermedin, CCK, leptin, PYY(1-36), PYY(3-36), GLP-1(1-37), GLP-1(7-37), GLP-1(7-36), GLP-2, OXM, exendin-3, and exendin-4, wherein the fragment exhibits at least one hormonal activity of the component peptide hormone.

Yet other preferred bioactive peptide hormone modules include fragments of analogs or derivatives of a component peptide hormone selected from: amylin, ADM, CT, CGRP, intermedin, CCK, leptin, PYY(1-36), PYY(3-36),GLP-1(1-37), GLP-1(7-37), GLP-1(7-36), GLP-2, OXM, exendin-3, and exendin-4, wherein the fragment exhibits at least one hormonal activity of the component peptide hormone. Again, the analog may comprise one or more insertions, deletions, or substitutions of the amino acid sequence of the component peptide hormone, and the derivative may comprise one or more chemical modifications of an amino acid residue of an analog or component peptide hormone, as described more fully herein and known in the art.

Certain preferred fragments that exhibit at least one hormonal activity include the following. However, it should be understood that combinations of the above-described analogs and derivatives taken with fragments known in the art, including the preferred fragments described below, are contemplated.

| Amylin: | amylin(1-36), amylin(1-35), amylin(1-20), amylin(1-18), amylin(1-17), amylin (1-16), amylin(1-15), amylin(1-7) |
|---|---|
| CT: | CT(8-32), CT(8-27), CT(8-26), CT(8-10), CT(18-26), CT(18-27) |
| AFP-6: | AFP-6(18-27) |
| CCK: | CCK-8, CCK-5, CCK-4 |
| Leptin: | leptin (22-167), leptin(56-73) |
| PYY: | PYY(1-35), PYY(1-30), PYY(1-25), PYY(1-15), PYY(1-10), PYY(2-36), PYY(3-36), PYY(4-36), PYY(5-36) |
| GLP-1 | GLP-1 (7-37), GLP-1(7-36), GLP-1(7-35) |
| Exendin | exendin-4(1-27), exendin-4(1-28), exendin-4(1-29), exendin-4(1-30) or longer |

Again, as known in the art, such peptide compounds may preferably be amidated, but within the context of the present disclosure, may optionally be in the acid form unless otherwise specified. Further, the above preferred fragments may be combined with any of the analogs or derivatives discussed herein or known in the art. For example, preferred analog fragments may include ⁵Ala,¹⁴Leu,²⁵Phe-exendin-4(1-28), ¹⁴Leu,²⁵Phe-exendin-4(1-27), ⁵Ala,¹⁴Leu,²⁵Phe-exendin-4(1-28) ¹⁴Leu,²⁵Phe-exendin-4(1-27), or any other combinations of the disclosed fragments, analogs, and derivatives.

Yet other preferred bio-active peptide modules include "peptidic enhancer", i.e., structural motifs of component peptide hormones (including analogs and derivatives thereof) that impart a desired chemical stability, conformational stability, metabolic stability, receptor interaction, protease inhibition, and/or other pharmacokinetic characteristic to the hybrid polypeptide. Exemplary peptidic enhancers include the following. Again, it should be understood that combinations of the above-described analogs and derivatives taken together with the following bio-active peptide modules are contemplated. For example, the last six amino acid residues of amylin family peptide hormone analogs and derivatives known in the art and/or described above are also contemplated as preferred bio-active peptide modules.

| | |
|---|---|
| Amylin Family | amylin(32-37), amylin(33-37), amylin(34-37), Amylin(35-37), amylin(36-37), amylin(37), ADM(47-52), ADM(48-52), ADM(49-52), ADM(50-52), ADM(51-52), ADM(52), CT(27-32), CT(27-32), CT(28-32), CT(29-32), CT(30-32), CT(31-32), CT(32), CGRP(32-37), CGRP(33-37), CGRP(34-37), CGRP(35-37), CGRP(36-37), CGRP(37), intermedin (42-47), intermedin (43-47), intermedin (44-47), intermedin (45-47), intermedin (46-47), intermedin(47) |
| PYY | PYY(25-36), PYY(26-36), PYY(27-36), PYY(28-36), PYY(29-36), PYY(30-36), PYY(31-36), PYY(32-36), PYY(25-35), PYY(26-35), PYY(27-35), PYY(28-35), PYY(29-35), PYY(30-35), PYY(31-35), PYY(32-35) |
| GLP-1 and 2 | frog GLP-1(29-37); frog GLP-1(30-37); frog GLP-2(24-31), frog GLP-2(25-31) |
| Exendin-4 | exendin-4(31-39), exendin-4(32-39), exendin-4(33-39), exendin-4(34-39), exendin-4(35-39), exendin-4(36-39), exendin-4(37-39), exendin-4(38-39), exendin-4(39) |

### Peptide Module Selection Considerations, Spacers, and Linking Groups

The hybrid polypeptides of the present invention according to the claims comprise at least two bio-active peptide hormone modules of the invention, wherein at least one of the bio-active peptide hormone modules exhibits at least one hormonal activity. The bio-active peptide hormone module that exhibits the at least one hormonal activity may be located at the N-terminal end of the hybrid polypeptide, the C-terminal end of the hybrid polypeptide, or in the event that the hybrid polypeptide comprises more than two bio-active peptide hormone modules, may be located in the internal portion of the hybrid polypeptide.

In certain embodiments, it may be preferable to locate the bio-active peptide hormone module exhibiting the at least one hormonal activity such that the C-terminal end of the bio-active peptide hormone module is amidated. Amidation of the C-terminal end of the bio-active peptide hormone module may be accomplished by locating the module at the C-terminal end of the hybrid peptide, or by configuring the module in the C-terminal-toN-terminal direction at the N-terminal end of the hybrid polypeptide. In both configurations, the C-terminal end of the bio-active peptide hormone module is available for amidation. Specific component peptide hormones where C-terminal amidation may be preferably include amylin family peptide hormones, CCK, PYY, hGLP-1(7-36) and hGLP-2. Specific component peptide hormones where. C-terminal amidation is not necessarily preferred (stated otherwise, where elongation at the C-terminal end of the module is easily tolerated) include exendin-4, exendin-4(1-28), GLP-1(7-37), frog GLP-1(7-36), and frog GLP-2. However, if these component peptide hormones are located at the C-terminal end of the hybrid polypeptide, they may still be optionally amidated, and in fact may preferably be optionally amidated.

The bio-active peptide hormone modules may be covalently linked in any manner known in the art. Stable linkages may be used, or cleavable linkage may be used. In one embodiment, the carboxy of a first module may be directly linked to the amino of a second module. In another embodiment, linking groups may be used to attached modules. Further, if desired, spacers or turn inducers known in the art may be employed to stabilize die linkage. By way of example, where amidation of the C-terminal end of the N-terminally located bio-active peptide hormone module is not desired, the module may be attached to a second module directly, or using any appropriate linking group known in the art, such as, an alkyl; PEG; amino acid, *e.g.*, Lys, Glu, β-Ala; polyaminoacids, *e.g*., poly-his, poly-arg, poly-lys, poly-ala, Gly-Lys-Arg (GKR) *etc.;* bifunctional linker (see, *e.g*., Pierce catalog, Rockford, II); aminocaproyl ("Aca"), β-alanyl, 8-amino-3,6-dioxaoctanoyl, or other cleavable and non-cleavable linker known in the art.

Where amidation, of the C-terminal end of N-terminally located bio-active peptide hormone module is desired, the module may again be attached to a second module using any appropriate linking group known in the art. More specifically, in the event that a bio-active peptide hormone module exhibiting at least one hormonal activity has been configured in the C-terminal-to-N-terminal orientation, resulting in an amino to amino linkage, preferred linking groups include dicarboxylic acids, alkyls, PEGs, and amino acids such as Lys, Cys, and Glu.

As mentioned above, the hybrid polypeptides may also preferably include spacer to further stablize the linkage of the bio-active peptide hormone modules. Any spacer or turn inducer known in the art may be used. By way of example, referred β-turn mimetics include mimic A and mimic B illustrated below, also Ala-Aib and Ala-Pro dipeptides.

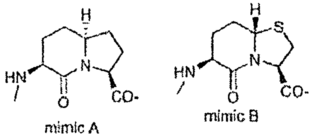

### Exemplary Combinations and Specific Embodiments

Exemplary combinations of bio-active peptide hormone modules to form the hybrid polypeptides of the invention according to the claims include combination of two or more bio-active peptide hormone modules selected from: native peptide hormones, analogs and derivatives of peptide hormones that exhibit at least one hormonal activity, fragments of native peptide hormones that exhibit at least one hormonal activity, fragments of analogs and derivatives of peptides hormones that exhibit at least one hormonal activity, and peptidic enhancers, with the proviso that at least one module exhibit at least one hormonal activity.

The hybrid polypeptides of the invention will include at least two bio-active peptide hormone modules, wherein each module is comprised from component peptide hormones. In the context of the present invention, the component peptide hormones of the hybrid polypeptide may be the same or different, with the proviso that at least two of the component peptide hormones are different. In a preferred embodiment, at least two of the component peptide hormones are from different peptide hormone families, e.g., the amylin family, CCK, the leptin family, PPF, the proglucagon family, and the exendin family.

In certain embodiments, the hybrid polypeptides of the invention may comprise two or more modules that exhibit at least one hormonal activity. For instance, the hybrid polypeptide may comprise a fragment of a first peptide hormone or analog that exhibits at least one hormonal activity covalently, linked to a fragment of at least one additional peptide hormone analog. The additional fragment(s) exhibit at least one hormonal activity. The first peptide hormone may be the same or different from the additional peptide hormone(s), with the proviso that at least one of the additional peptide hormones are different from the first peptide hormone, and the first hormonal activity may be the same or different from the optional additional hormonal activity.

In other disclosures, the hybrid polypeptides of the invention may comprise one or more modules that exhibit at least one hormonal activity in combination with one or more peptidic enhancer modules. For instance, a fragment of a first peptide hormone that exhibits a at least one hormonal activity may be covalently linked to a peptidic enhancer, or a fragment of a first peptide hormone that exhibits at least one hormonal activity may be covalently linked to a second peptide hormone that exhibits at least one hormonal activity, which is in turn linked to a peptidic enhancer. Alternatively, a peptidic enhancer may be located between two peptide hormone modules as a stabilizing spacer. Again, the first peptide hormone may be the same or different from the second peptide hormone, and the first hormonal activity may be the same or different from the second hormonal activity.

In another embodiment, the hybrid polypeptides of the invention may comprise two, three, four, or more bio-active peptide hormone modules. Exemplary combinations of the disclosure include a module with a hormonal activity in combination with one, two, or three peptidic enhancers; two modules with a hormonal activity in combination with one or two peptidic enhancers; three modules with a hormonal activity in combination with one peptidic enhancer, etc.

The component peptide hormones of the disclosure are preferably selected from amylin, adrenomedullin, calcitonin, calcitonin gene related peptide, intermedin, cholecystokinin, leptin peptide YY, glucagon-like peptide-1, glucagon-like peptide 2, oxyntomodulin, or exendin-4.

More particularly, preferred module combinations include those involving combinations of exendin, amylin, and PYY as the component peptide hormones. Particular combinations include exendin-4/PYY and PYY/exendin-4 combinations, with and without spacers or linking groups. Other combinations include exendin/amylin and amylin/exendin combinations, with and without spacers or linking groups. Yet other combinations include amylin/PYY and PYY/amylin combinations, with and without spacers or linking groups.

In one aspect of the invention according to the claims, preferred module combinations includes those involving a first module comprising exendin-4, a fragment of exendin-4 that exhibits at least one hormonal activity, an exendin-4 analog or derivative that exhibits at least one hormonal activity, or a fragment of an exendin-4 analog that exhibits at least one hormonal activity in combination with at least one additional bio-active peptide hormone module. In one embodiment, the first module is linked to one, two, or three additional bio-active peptide hormone modules.

In embodiments of the invention according to the claims, a first module comprising an exendin-4 peptide is linked to a second bio-active peptide hormone module comprising an amylin peptide that exhibits at least one hormonal activity. In another embodiment, the second module is further linked to a third bio-active peptide hormone module comprising a calcitonin peptide that exhibits at least one hormonal activity. In yet another embodiment, the third module may be further linked to a fourth bio-active peptide hormone module comprising a peptidic enhancer selected from amylin peptides. In one embodiment, the first module may be located at the C-terminal end of the hybrid polypeptide. Alternatively, the first module may be located at the N-terminal end of the hybrid polypeptide. In certain embodiments, spacers or linkers such as βAla may be inserted if desired to link the modules.

Preferred exendin-4 peptides include: exendin-4, exendin-4(1-27), exendin-4(1-28), ¹⁴Leu,²⁵Phe-exendin-4(1-28), and ⁵Ala,¹⁴Leu,²⁵Phe-exendin-4(1-28). Preferred amylin peptides that exhibit at least one hormonal activity include amylin, amylin fragments such as amylin(1-17), amylin (1-16), amylin(1-15), and amylin(1-7), and amylin analogs such as pramlintide, ²Ala-h-amylin, ^{2,7}Ala-h-amylin, and fragments thereof. Preferred calcitonin peptides that exhibit at least one hormonal activity sCT, sCT fragments such as sCT(8-10), sCT(8-27), and, and calcitonin analogs such as ¹⁸Arg-sCT, ¹⁴Gln,¹⁸Arg-sCT, ¹⁴Gln,^{11,18}Arg-sCT, and fragments thereof. Preferred amylin peptidic enhancers include amylin(32-37), amylin(33-37), and amylin(34-37), and analogs thereof. Amylin/sCT combinations useful in connection with the present invention inlcude those disclosed in PCT/US05/[XXXXX], Amylin Family Agonist, Attorney Docket 18528.835, filed concurrently herewith,

In one aspect of the disclosure preferred module combinations includes those involving a first module comprising exendin-4, a fragment of exendin-4 that exhibits at least one hormonal activity, an exendin-4 analog or derivative that exhibits at least one hormonal activity, or a fragment of an exendin-4 analog that exhibits at least one hormonal activity in combination with a peptidic enhancer. Preferred exendin-4 compounds include: exendin-4, exendin-4(1-27), exendin-4(1-28), ¹⁴Leu,²⁵Phe-exendin-4(1-28), and ⁵Ala,¹⁴Leu,²⁵Phe-exendin-4(1-28). Preferred peptidic enhancers include: PYY(25-36), PYY(30-36) and PYY(31-36). In one disclosure, the first module is located at the C-terminal end of the hybrid polypeptide and the peptidic enhancer is located at the N-terminal end of the hybrid polypeptide. Alternatively, the first module may be located at the N-terminal end of the hybrid polypeptide and the peptidic enhance may be located at the C-terminal end of the hybrid polypeptide. In certain disclosure, spacers or linkers such as βAla may be inserted if desired to attach the modules.

In another disclosure, preferred module combinations include those involving a first module comprising exendin-4, a fragment of exendin-4 that exhibits at least one hormonal activity, an exendin-4 analog or derivative that exhibits at least one hormonal activity, or a fragment of an exendin-4 analog that exhibits at least one hormonal activity in combination with a second module comprising CCK, a fragment of CCK that exhibits at least one hormonal activity, a CCK analog or derivative that exhibits at least one hormonal activity, or a fragment of a CCK analog that exhibits at least one hormonal activity. Again, preferred exendin-4 compounds include: exendin-4, exendin-4(1-27), exendin-4(1-28), ¹⁴Leu,²⁵Phe-exendin-4(1-28), ⁵Ala,¹⁴Leu,²⁵Phe-exendin-4(1-28), and ¹⁴Leu-exendin-4(1-28). Preferred CCK compounds include: CCK-8, and CCK-8(Phe(CH₂SO₃)). In one disclosure, the first module is located at the C-terminal end of the hybrid polypeptide and the second module is located at the N-terminal end of the hybrid polypeptide. Alternatively, the first module may be located at the N-terminal end of the hybrid polypeptide and the peptidic enhance may be located at the C-terminal end of the hybrid polypeptide. In certain disclosures, spacers or linkers such as βAla may be inserted if desired to attach the modules.

In another disclosure preferred module combinations include those involving a first module comprising amylin, a fragment of amylin that exhibits at least one hormonal activity, an amylin analog or derivative that exhibits at least one hormonal activity, or a fragment of an amylin analog that exhibits at least one hormonal activity in combination with a second module comprising with a peptidic enhancer, such as PYY(25-36) or PYY(30-36). In one disclosure, the first module is located at the C-terminal end of the hybrid polypeptide and the peptidic enhancer is located at the N-terminal end of the hybrid polypeptide. Alternatively, the first module may be located at the N-terminal end of the hybrid polypeptide and the peptidic enhance may be located at the C-terminal end of the hybrid polypeptide. In certain disclosures, spacers or linkers such as βAla may be inserted if desired to attach the modules.

Other preferred module combinations include those involving combinations of exendin and CCK or amylin, calcitonin, and CCK as a tertiary combination. Particular combinations include exendin/CCK and CCK/exendin, with and without spacers or linkers and linking groups. Other combinations include CCK/amylin/calcitonin and CCK/amylin/calcitonin/amylin, with and without spacers or linking groups. Each module may independently be a peptidic enhancer or may exhibit a hormonal activity, depending on the desired properties of the hybrid polypeptide.

Yet other preferred module combinations include those involving combinations of exendin, amylin and calcitonin as tertiary and tetra-hybrid molecules. Exemplary combinations include exendin/amylin/calcitonin; exendin/amylin/calcitonin/amylin; amylin/calcitonin/exendin; and amylin/calcitonin/amylin/exendin combinations, with and without spacers or linking groups. Each module may independently be a peptidic enhancer or may exhibit a hormonal activity, depending on the desired properties of the hybrid polypeptide.

In one disclosure, when one of the bio-active peptide hormone module(s) that exhibits at least one hormonal activity is amylin or an analog or fragment thereof, and a second bio-active peptide hormone module comprises CCK, then the hybrid polypeptide should preferably comprise a third bio-active peptide hormone module selected from a different component peptide hormone. Exemplary third bio-active peptide hormone modules include calcitonins, more preferably salmon calcitonin, analogs or fragments thereof.

In another disclosure, when one of the bio-active peptide hormone module(s) that exhibits at least one hormonal activity is amylin or an analog or fragment thereof, and a second bio-active peptide hormone module comprises CT, then the hybrid polypeptide should preferably comprise a third bio-active peptide hormone module selected from a different component peptide hormone. Exemplary third bio-active peptide hormone modules include exendin-4, analogs or fragments thereof.

In yet another disclosure, when one of the bio-active peptide hormone module(s) that exhibits at least one hormonal activity is GLP-1 or an analog or fragment thereof; and a second bio-active peptide hormone module is a peptidic enhancer comprising an exendin fragment, then the hybrid polypeptide should preferably comprise a third bio-active peptide hormone module. Exemplary third bio-active peptide hormone modules include PYY (including analogs, derivatives and fragments thereof) and CCK (including analogs, derivatives and fragments thereof).

Within each of the above described preferred combinations, it is understood that reference to a component peptide hormone includes reference to analogs, derivatives, fragments, as well as peptidic enhancers related thereto.

In a preferred aspect, the hybrid polypeptides include:

| SEQ ID: | |
|---|---|
| 1 | Exendin-4-PYY(22-36) |
| 2 | Exendin-4-PYY(25-36) |
| 3 | Exendin-4-PYY(18-36) |
| 4 | Exendin-4-βAla-βAla-PYY(22-36) |
| 5 | Exendin-4-βAla-βAla-PYY(25-36) |
| 6 | Exendin-4-βAla-βAla-PYY(31-36) |
| 7 | Exendin-4(1-28)-PYY(22-36) |
| 8 | Exendin-4(1-28)-PYY(25-36) |
| 9 | Exendin-4(1-28)-PYY(18-36) |
| 10 | Exendin-4(1-28)-βAla-βAla-PYY(22-36) |
| 11 | Exendin-4(1- 28)-βAla-βAla-PYY(25-36) |
| 12 | Exendin-4(1-28)-βAla-βAla-PYY(31-36) |
| 13 | ⁵Ala,¹⁴Leu,²⁵Phe-Exendin-4(1-28)-PYY(18-36) |
| 14 | ⁵Ala,¹⁴Leu,²⁵Phe-Exendin-4(1-28)-PYY(22-36) |
| 15 | ⁵Ala,¹⁴Leu,25Phe-Exendin-4(1-28)-PYY(25-36) |
| 16 | 5Ala,¹⁴Leu,²⁵Phe-Exendin-4(1-17)-PYY(18-36) |
| 17 | ⁵Ala,¹⁴Leu,²⁵Phe-Exendin-4(1-28)-βAla-βAla-PYY(22-36) |
| 18 | ⁵Ala, ¹⁴Leu, ²⁵Phe-Exendin-4(1-28)-βAla-βAla -PYY(25-36) |
| 19 | ⁵Ala, ¹⁴Leu, ²⁵Phe-Exendin-4(1-28)-βAla-βAla -PYY(31-36) |
| 20 | Exendin-4-CCK-8 |
| 21 | Exendin-4(1-28)-CCK-8 |
| 22 | Exendin-4(1-28)-CCK-8(Phe(CH₂SO₃)) |
| 23 | Exendin-4(1-28)-(8-amino-3,6-dioxactoanoyl)-CCK-8 |
| 24 | Exendin-4(1-28)-(8-amino-3,6-dioxactoanoyl)-CCK-8(Phe(CH₂SO₃)) |
| 25 | Exendin4(1-27)-hAmylin(1-7)-¹⁴Gln, ^{11,18}Arg-sCT(8-27)-Amylin(33-37) |
| 26 | Exendin-4(1-27)-^{2,7}Ala-hAmylin(1-7)-sCT(8-10) |
| 27 | ²⁹12 Ado-Exendin(1-28)-hAmylin(1-7)-^{11,1s}Arg-sCt(8-32)-hAmylin(33-37) |
| 28 | ²⁹12 Ado-Exendin(1-28)-¹des-Lys-hAmylin(1-7)-^{11,18}Arg-sCt(8-32)-hAmylin(33-37) |
| 29 | ²⁹3,6-dioxaoctanoyl-Exendin(1-28)-hAmylin(1-7)-^{11,18}Arg-sCt(8-32)-hAmylin(33-37) |
| 30 | ²⁹3,6-dioxaoctanoyl-Exendin(1-28)-¹des-Lys-hAmylin(1-7),^{11,18}Arg-sCt(8-32)-hAmylin(33-37) |
| 31 | ²⁹5 Apa -Exendin(1-28)-hAmylin(1-7)-^{11,18}Arg-sCt(8-32)-hAmylin(33-37) |
| 32 | ²⁹5 Apa Exendin(1-28) -¹des-Lys-hAmylin(1-7)-^{11,18}Arg-sCt(8-32)-hAmylin(33-37) |
| 33 | ²⁹βAla-βAla-Exendin(1-28),hAmylin(1-7)-^{11,18}Arg-sCt(8-32)-hAmylin(33-37) |
| 34 | ²⁹βAla-βAla-Exendin(1-28)-¹des-Lys-hAmylin(1-7)-^{11,18}Arg-sCt(8-32)-hAmylin(33-37) |
| 35 | ²⁹4,7,10-trioxa-13-tridecanamine succinimidyl-Exendin(1-28)-hAmylin(1-7) ^{11,18}Arg-sCt(8-32)-hAmylin(33-37) |
| 36 | ²⁹4,7,10-trioxa-13-tridecanamine succinimidyl-Exendin(1-28)-¹des-Lys-hAmylin(1-7)-^{11,18}Arg-sct(8-32)-hAmylin(33-37) |
| 37 | CCK-8-GKR-¹⁵Glu-hAmylin(1-17)-¹⁸Arg-sCT(18-26)-Amylin(32-37) |
| 38 | Amylin(1-18}-PYY(19-36) |
| 39 | isocaproyl-STAVL-(Aib)-K(formyl)-LSQEL-(Aib)-K(formyl)-LQT-PYY(18-36) |
| 40 | isocaproyl-STAVL-(Aib)-K(formyl)-LSQEL-(Aib)-K(formyl)-L-PYY(16-36) |
| 41 | CCK-8-[Succinoyl-Cys]-PYY(3-36) |
| 42 | CCK-8-[Bis-Cys(N-Acetyl)]-PYY(3-36) |
| 43 | CCK-8-[Cly-Aminoxymethylcarbonyl]-PYY(3-36) |

The hybrid polypeptides of the present invention according to the claims may also comprise further modifications including, but are not limited to, substitution, deletion, and insertion to the amino acid sequence of such hybrid polypeptides and any combination thereof. In a preferred aspect, the hybrid polypeptides of the invention include one or more modifications of a "non-essential" amino acid residue. In the context of the invention, a "non-essential" amino acid residue is a residue that can be altered, *i.e*., deleted or substituted, in the native human amino acid sequence of the fragment, *e.g*., the component peptide hormone fragment, without abolishing or substantially reducing the component peptide hormone receptor agonist activity of the hybrid polypeptide.

Preferred substitutions include conserved amino acid substitutions. A "conservative amino acid substitution" is one in which the amino acid residue is replaced with an amino acid residue having a similar side chain, or physicochemical characteristics (*e.g*., electrostatic, hydrogen bonding, isosteric, hydrophobic features). Families of amino acid residues having similar side chains are known in the art. These families include amino acids with basic side chains (*e.g*., lysine, arginine, histidine), acidic side chains (*e.g*., aspartic acid, glutamic acid), uncharged polar side chains (*e.g*., glycine, asparagine, glutamine, serine, threonine, tyrosine, methionine, cysteine), nonpolar side chains (*e.g*., alanine, valine, leucine, isoleucine, proline, phenylalanine, tryptophan), β-branched side chains (*e.g*., threonine, valine, isoleucine) and aromatic side chains (*e.g*., tyrosine, phenylalanine, tryptophan, histidine).

The present invention also relates to derivatives of the hybrid polypeptides, Including hybrid polypeptides conjugated to one or more water soluble polymer molecules, such as polyethylene glycol ("PEG") or fatty acid chains of various lengths (*e.g*., stearyl, palmitoyl, octanoyl, etc.), or by the addition of polyamino acids, such as poly-his, poly-arg, poly-lys, and poly-ala. In the disclosure, modifications to the hybrid polypeptides can also include small molecule substituents, such as short alkyls and constrained alkyls (*e.g*., branched, cyclic, fused, adamantyl), and aromatic groups. The water soluble polymer molecules will preferably have a molecular weight ranging from about 500 to about 20,000 Daltons.

Such polymer-conjugations and small molecule substituent modifications may occur singularly at the N- or C-tcerminus or at the side chains of amino acid residues within the sequence of the hybrid polypeptides. Alternatively, there may be multiple sites of derivatization along the hybrid polypeptide. Substitution of one or more amino acids with lysine, aspartic acid, glutamic acid, or cysteine may provide additional sites for derivatization. *See, e.g.,* U.S. Patent Nos. 5,824,784 and 5,824,778. Preferably, the hybrid polypeptides may be conjugated to one, two, or three polymer molecules.

The water soluble polymer molecules are preferably lined to an amino, carboxyl, or thiol group, and may be linked by N or C terminus, or at the side chains of lysine, aspartic acid, glutamic acid, or cysteine. Alternatively, the water soluble polymer molecules may be linked with diamine and dicarboxylic groups. In a preferred embodiment, the hybrid polypeptides of the invention are conjugated to one, two, or three PEG molecules through an epsilon amino group on a lysine amino acid.

Hybrid polypeptide derivatives of the disclosure also include hybrid polypeptides with chemical alterations to one or more amino acid residues. Such chemical alterations include amidation, glycosylation, acylation, sulfation, phosphorylation, acetylation, and cyclization. The chemical alterations may occur singularly at the N- or C-terminus or at the side chains of amino acid residues within the sequence of the PPF hybrid polypeptides. In one disclosure, the C-terminus of these peptides may have a free -OH or -NH₂ group. In another disclosure, the N-terminal end may be capped with an isobutyloxycarbonyl group, an isopropyloxycarbonyl group, an n-butyloxycarbonyl group, an ethoxycarbonyl group, an isocaproyl group (isocap), an octanyl group, an octyl glycine group (G(Oct)), or an 8-aminooctanic acid group. In a preferred disclosure, cyclization can be through the formation of disulfide bridges. Alternatively, there may be multiple sites of chemical alteration along the hybrid polypeptide.

Examples of the hybrid polypeptides of the present disclosure are provided in the Sequence Listing and further discussed in the Examples section below.

### Hybrid-Polypeptides for use in the Treatment or Prevention of Metabolic Conditions or Disorders

In another aspect of the disclosure, methods for treating or preventing obesity are provided, wherein the method comprises administering a therapeutically or prophylactically effective amount of a hybrid polypeptide to a subject in need thereof. In a preferred disclosure, the subject is an obese or overweight subject. While "obesity" is generally defined as a body mass index over 30, for purposes of this disclosure, any subject, including those with a body mass index of less than 30, who needs or wishes to reduce body weight is included in the scope of "obese." Subjects who are insulin resistant, glucose intolerant, or have any form of diabetes mellitus (*e.g*., type 1, 2 or gestational diabetes) can benefit from this method.

In other aspects of the disclosure, methods of reducing food intake, reducing nutrient availability, causing weight loss, affecting body composition, and altering body energy content or increasing energy expenditure, treating diabetes mellitus, and improving lipid profile (including reducing LDL cholesterol and triglyceride levels and/or changing HDL cholesterol levels) are provided, wherein the methods comprise administering to a subject an effective amount of a hybrid polypeptide of the disclosure. In a preferred disclosure, the methods of the disclosure are used to treat or prevent conditions or disorders which can be alleviated by reducing nutrient availability in a subject in need thereof, comprising administering to said subject a therapeutically or prophylactically effective amount of a hybrid polypeptide of the invention. Such conditions and disorders include, but are not limited to, hypertension, dyslipidemia, cardiovascular disease, eating disorders, insulin-resistance, obesity, and diabetes mellitus of any kind.

Without intending to be limited by theory, it is believed that the effects of peripherally-administered hybrid polypeptides of the present disclosure in the reduction of food intake, in the delay of gastric emptying, in the reduction of nutrient availability, and in the causation of weight loss are determined by interactions with one or more unique receptor classes in, or similar to, those in the PP family. More particularly, it appears that a receptor or receptors similar to the PYY-preferring (or Y7) receptors are involved.

Additional assays useful to the disclosure include those that can determine the effect of PPF compounds on body composition. An exemplary assay can be one that involves utilization of a diet-induced obese (DIO) mouse model for metabolic disease. Prior to the treatment period, male C57BL/6J mice can be fed a high-fat diet (#D12331, 58% of calories from fat; Research Diets, Inc.,) for 6 weeks beginning at 4 weeks of age. During the study, the mice can continue to eat their high-fat diet. Water can be provided *ad libitum* throughout the study. One group of similarly-aged non-obese mice can be fed a low-fat diet (#D12329, 11% of calories from fat) for purposes of comparing metabolic parameters to DIO groups.

DIO mice can be implanted with subcutaneous (SC) intrascapular osmotic pumps to deliver either vehicle (50% dimethylsulfoxide (DMSO) in water) or a compound of the invention. The pumps of the latter group can be set to deliver any amount, e.g., 1000 µg/kg/d of a compound of the invention for 7-28 days.

Body weights and food intake can be measured over regular intervals throughout the study periods. Respiratory quotient (RQ, defined as CO₂ production ÷ O₂ consumption) and metabolic rate can be determined using whole-animal indirect calorimetry (Oxymax, Columbus Instruments, Columbus, OH). The mice can be euthanized by isoflurane overdose, and an index of adiposity (bilateral epididymal fat pad weight) measured. Moreover, prior to determination of epididymal weight, body composition (lean mass, fat mass) for each mouse can be analyzed using a Dual Energy X-ray Absorptiometry (DEXA) instrument per manufacturer's instructions (Lunar Piximus, GE Imaging System). In the methods of the invention, preferred PPF polypeptide of the invention are those having a potency in one of the assays described herein (preferably food intake, gastric emptying, pancreatic secretion, weight reduction or body composition assays) which is greater than the potency of a component peptide hormone in that same assay.

In addition to the amelioration of hypertension in subjects in need thereof as a result of reduced food intake, weight loss, or treating obesity, compounds of the invention may be used to treat hypotension.

Compounds of the disclosure may also be useful for potentiating, inducing, enhancing or restoring glucose responsivity in pancreatic islets or cells. These actions may be useful for treating or preventing conditions associated with metabolic disorders such as those described above and in U.S. patent application no. US20040228846. Assays for determining such activity are known in the art. For example, in published U.S. patent application no. US20040228846, assays are described for islet isolation and culture as well as determining fetal islet maturation. In the examples of patent application US20040228846, intestine-derived hormone peptides including pancreatic polypeptide (PP), neuropeptide Y (NPY), neuropeptide K (NPK), PYY, secretin, glucagon-like peptide-1 (GLP-1) and bombesin were purchased from Sigma. Collagenase type XI was obtained from Sigma. RPMI 1640 culture medium and fetal bovine serum were obtained from Gibco. A radioimmunoassay kit containing anti-insulin antibody ([^{12S}I]-RIA kit) was purchased from Linco, St Louis.

Post-partem rat islets were obtained from P-02 year old rats. Adult rat islets were obtained from 6-8 week old rats. Fetal rat islets were obtained as follows. Pregnant female rats were sacrificed on pregnancy day e21. Fetuses were removed from the uterus.

10-14 pancreata were dissected from each litter and washed twice in Hanks buffer. The pancreas were pooled, suspended in 6 ml 1 mg/ml collagenase (Type XI, Sigma) and incubated at 37° C for 8-10 minutes with constant shaking. The digestion was stopped by adding 10 volumes of ice-cold Hanks buffer followed by three washes with Hanks buffer. The islets were then purified by Ficoll gradient and cultured in 10% fetal bovine serum (FBS)/RPMI medium with or without addition of 1 µM IBMX. At the end of five days, 20 islets were hand picked into each tube and assayed for static insulin release. Generally, islets were first washed with KRP buffer and then incubated with 1 ml of KRP buffer containing 3 mM (low) glucose for 30 minutes at 37° C. with constant shaking. After collecting the supernatant, the islets were then incubated with 17 mM (high) glucose for one hour at 37° C. The insulin released from low or high glucose stimulation were assayed by radioimmunoassay (RIA) using the [²⁵I]-RIA kit. E21 fetal islets were cultured for 5 days in the presence of 200 ng/ml PYY, PP, CCK, NPK, NPY, Secretin, GLP-1 or Bombesin.

An exemplary *in vivo* assays is also provided using the Zucker Diabetic Fatty (ZDF) male rat, an inbred (>F30 Generations) rat model that spontaneously expresses diabetes in all fa/fa males fed a standard rodent diet Purina 5008. In ZDF fa-fa males, hyperglycemia begins to develop at about seven weeks of age and glucose levels (fed) typically reach 500 mg/DL by 10 to 11 weeks of age. Insulin levels (fed) are high during the development of diabetes. However, by 19 weeks of age insulin drops to about the level of lean control litter mates. Triglyceride and cholesterol levels of obese rats are normally higher than those of leans. In the assay, three groups of 7-week old ZDF rats, with 6 rats per group, received the infusion treatment by ALZA pump for 14 days: 1) vehicle control, 2) and 3), PYY with two different doses, 100 pmol/kg/hr and 500 pmol/kg/hr respectively. Four measurements were taken before the infusion and after the infusion at day 7 and day 14: 1) plasma glucose level, 2) plasma insulin level, and 3) plasma triglycerides (TG) level, as well as oral glucose tolerance (OGTT) test. Accordingly, these assays can be used with compounds of the invention to test for desired activity.

Other uses contemplated for the hybrid polypeptides include methods for reducing aluminum (Al) concentrations in the central nervous system (see U.S. Pat. 6,734,166) for treating, preventing, or delay the onset of Alzheimer's disease. Assays for determining effects on Al are known in the art and can be found in US Pat 6,734,166 using diploid and Ts mice. These mice were individually housed in Nalgene® brand metabolism or polypropylene cages and given three days to adjust to the cages before experimentation. Mice had free access to food (LabDiet® NIH Rat and Moust/Auto 6F5K52, St. Louis, Mo.) and water during the experiment except for the 16 hours prior to euthanasia when no food was provided. Mice were given daily subcutaneous injections of either active compound or saline. Mice were sacrificed at the end of day 13 for one experiment and day 3 for another, and samples were collected. Mice brain samples were weighted in clean teflon liners and prepared for analysis by microwave digestion in low trace element grade nitric acid. Sample were then analyzed for Al content using Inductively Coupled Plasma Mass Spectrometry (Nuttall et al., Annals of Clinical and Laboratory Science 25, 3, 264-271 (1995)). All tissue handling during analysis took place in a clean room environment utilizing HEPA air filtration systems to minimize background contamination.

The compounds of the disclosure exhibit a broad range of biological activities, some related to their antisecretory and antimotility properties. The compounds may suppress gastrointestinal secretions by direct interaction with epithelial cells or, perhaps, by inhibiting secretion of hormones or neurotransmitters which stimulate intestinal secretion. Anti-secretory properties include inhibition of gastric and/or pancreatic secretions and can be useful in the treatment or prevention of diseases and disorders including gastritis, pancreatitis, Barrett's esophagus, and Gastroesophageal Reflux Disease.

Compounds of the disclosure are useful in the treatment of any number of gastrointestinal disorders (see *e.g*., Harrison's Principles of Internal Medicine, McGraw-Hill Inco, New York, 12th Ed.) that are associated with excess intestinal electrolyte and water secretion as well as decreased absorption, *e.g*., infectious diarrhea, inflammatory diarrhea, short bowel syndrome, or the diarrhea which typically occurs following surgical procedures, *e.g*., ileostomy. Examples of infectious diarrhea include, without limitation, acute viral diarrhea, acute bacterial diarrhea (*e.g*., salmonella, campylobacter, and clostridium or due to protozoal infections), or traveller's diarrhea (*e.g*., Norwalk virus or rotavirus).

Examples of inflammatory diarrhea include, without limitation, malabsorption syndrome, tropical sprue, chronic pancreatitis, Crohn's disease, diarrhea, and irritable bowel syndrome. It has also been discovered that the peptides of the invention can be used to treat an emergency or life-threatening situation involving a gastrointestinal disorder, e.g., after surgery or due to cholera.

Compounds of the disclosure may also be useful for treating or preventing intestinal damage as opposed to merely treating the symptoms associated with the intestinal damage (for example, diarrhea). Such damage to the intestine may be, or a result of, ulcerative colitis, inflammatory bowel disease, bowel atrophy, loss bowel mucosa, and/or loss of bowel mucosal function (see WO 03/105763).

Assays for such activity, as described in WO 03/105763, include 11 week old male HSD rats, ranging 250- 300 grams housed in a 12:12 light:dark cycle, and allowed ad libitum access to a standard rodent diet (Teklad LM 485, Madison, WI) and water. The animals were fasted for 24 hours before the experiment. A simple and reproducible rat model of chronic colonic inflammation has been previously described by Morris GP, et al., "Hapten- induced model of chronic inflammation and ulceration in the rat colon." Gastroenterology. 1989; 96:795-803. It exhibits a relatively long duration of inflammation and ulceration, affording an opportunity to study the pathophysiology of colonic inflammatory disease in a specifically controlled fashion, and to evaluate new treatments potentially applicable to inflammatory bowel disease in humans.

Rats were anesthetized with 3% isofluorane and placed on a regulated heating pad set at 37°C. A gavage needle was inserted rectally into the colon 7 cm. The hapten trinitrobenzenesulfonic acid (TNBS) dissolved in 50% ethanol (v/v) was delivered into the lumen of the colon through the gavage needle at a dose of 30 mg/kg, in a total volume of 0 0.4-0.6 mL, as described in Mazelin, et al., Juton Nerv Syst. 1998;73:38 45. Control groups received saline solution (NaCl 0.9%) intracolonically.

Four days after induction of colitis, the colon was resected from anesthetized rats, which were then euthanized by decapitation. Weights of excised colon and spleen were measured, and the colons photographed for scoring of gross morphologic damage. Inflammation was defined as regions of hyperemia and bowel wall thickening.

Hybrid polypeptides of the disclosure may also be used to treat or prevent pancreatic tumors (*e.g*., inhibit the proliferation of pancreatic tumors). Methods of the invention include reducing the proliferation of tumor cells. The types of benign pancreatic tumor cells which may be treated in accordance with the present invention include serous cyst adenomas, microcystic tumors, and solid-cystic tumors. The method is also effective in reducing the proliferation of malignant pancreatic tumor cells such as carcinomas arising from the ducts, acini, or islets of the pancreas. U.S. Pat. 5,574,010 provides exemplary assays for testing anti-proliferative properties. For example, the '010 patent provides that PANC-1 and MiaPaCa-2 are two human pancreatic adenocarcinoma cancer cell lines which are available commercially from suppliers such as American Type Culture Collection, ATCC (Rockville, Md.). The two tumor cells were grown in RPMI-1640 culture media supplemented with 10% fetal bovine serum, 29.2 mg/L of glutamine, 25 µg gentamicin, 5 ml penicillin, streptomycin, and fungizone solution (JRH Biosciences, Lenexa, Kans.) at 37 degrees Celcius in a NAPCO water jacketed 5 % CO₂ incubator. All cell lines were detached with 0.25 % trypsin (Clonetics, San Diego, Calif.) once to twice a week when a confluent monolayer of tumor cells was achieved. Cells were pelleted for 7 minutes at 500 g in a refrigerated centrifuge at 4 degrees Celcius, and resuspended in trypsin free fortified RPMI 1640 culture media. Viable cells were counted on a hemocytometer slide with trypan blue.

Ten thousand, 20,000, 40,000 and 80,000 cells of each type were added to 96 well microculture plates (Costar, Cambridge, Mass.) in a total volume of 200 ul of culture media per well. Cells were allowed to adhere for 24 hours prior to addition of the PYY or test peptide. Fresh culture media was exchanged prior to addition of peptides. In vitro incubation of pancreatic tumor cells with either PYY or test compound was continued for 6 hours and 36 hours in length. PYY was added to cells at doses of 250 pmol, 25 pmol, and 2.5 pmol per well (N =14). Test compound was added to cells cultures at doses of 400 pmol, 40 pmol, and 4 pmol per well. Control wells received 2 ul of 0.9% saline to mimic the volume and physical disturbance upon adhered tumor cells. Each 96 well plate contained 18 control wells to allow for comparison within each plate during experimentation. Ninety-six (96) well plates were repeated 6 times with varying concentrations of PYY and test compound in both the PANC-1 and MiaPaCa-2 cells.

At the end of the incubation period, 3-(4,5-dimethylthiazolyl-2-yl)-2,5-diphenyltetrazolium Bromide, MTr tetrazolium bromide (Sigma, St. Louis, Mo.) was added to fresh culture media at 0.5 mg/ml. Culture media was exchanged and tumor cells were incubated for 4 hours with MTT tetrazolium bromide at 37°C. At the end of incubation, culture media was aspirated. Formazon crystal precipitates were dissolved in 200 µl of dimethyl sulfoxide (Sigma, St. Louis, Mo.). Quantitation of solubilized formazon was performed by obtaining absorption readings at 500 nm wavelength on an ELISA reader (Molecular Devices, Menlo Park, Calif.). The MTT assay measures mitochondrial NADH dependent dehydrogenase activity, and it has been among the most sensitive and reliable method to quantitative *in vitro* chemotherapy responses of tumor cells. (Alley, M. C., et al., Cancer Res., 48:589-601, 1988; Carmichael, J., et al., Cancer Res., 47:936-942, 1987; McHale, A. P., et al., Cancer Lett., 41:315-321, 1988; and Saxton, R. E., et al., J. Clin. Laser Med. and Surg., 10(5):331-336, 1992.) Analysis of absorption readings at 550 nm were analyzed by grouping wells of the same test conditions and verifying differences occurring between control and the various peptide concentration treatments by one-way ANOVA.

An exemplary *in vivo* assay is also provided. The human pancreatic ductal adenocarcinoma Mia Paca-2 was examined for in vivo growth inhibition by peptide YY and test compound. Seventy thousand to 100,000 human Mia PaCa-2 cells were orthotopically transplanted into 48 male athymic mice. After one week, the animals were treated with either PYY or test compound at 200 pmol/kg/hr via mini-osmotic pumps for four weeks. The paired cultures received saline. At sacrifice, both tumor size and mass were measured. Control mice had significant human cancer growth within the pancreas as evidenced by histologic sections. At 9 weeks, ninety percent (90%) of control mice had substantial metastatic disease. Tumor mass was decreased by 60.5 % in test treated mice and 27% in PYY treated mice.

For all indications, in preferred embodiments, the hybrid polypeptide of the invention is administered peripherally at a dose of about 0.5 µg to about 5 mg per day in single or divided doses or controlled continual release, or at about 0.01 µg/kg to about 500 µg/kg per dose, more preferably about 0.05 µg/kg to about 250 µg/kg, most preferably below about 50 µg/kg. Dosages in these ranges will vary with the potency of each analog or derivative, of course, and may be determined by one of skill in the art.

In the methods of the present disclosure, hybrid polypeptides of the invention may be administered separately or together with one or more other compounds and compositions that exhibit a long term or short-term action to reduce nutrient availability, including, but not limited to other compounds and compositions that comprise an amylin or amylin analog agonist, salmon calcitonin, a cholecystokinin (CCK) or CCK agonist, a leptin (OB protein) or leptin agonist, an exendin or exendin analog agonist, or a GLP-1 or GLP-1 analog agonist. Suitable amylin agonists include, for example, [^{25,28,29}Pro-] human amylin (also known as "pramlintide," and described in U.S. Pat. Nos. 5,686,511 and 5,998,367). The CCK used is preferably CCK octapeptide (CCK-8). Leptin is discussed in, for example, (Pelleymounter et al., Science 269: 540-3 (1995); Halaas et al., Science 269: 543-6 (1995); Campfield et al., Science 269: 546-9 (1995)). Suitable exendins include exendin-3 and exendin-4, and exendin agonist compounds include, for example, those described in PCT Publications WO 99/07404, WO 99/25727, and WO 99/25728.

### Polypeptide Production and Purification

The hybrid polypeptides described herein may be prepared using standard recombinant techniques or chemical peptide synthesis techniques known in the art, e.g., using an automated or semi-automated peptide synthesizer, or both.

The hybrid polypeptides of the invention can be synthesized in solution or on a solid support in accordance with conventional techniques. Various automatic synthesizers are commercially available and can be used in accordance with known protocols. *See, e,g.,* Stewart and Young, Solid Phase Peptide Synthesis, 2d. ed., Pierce Chemical Co. (1984); Tam et al., J. Am. Chem. Soc. 105: 6442 (1983); Merrifield, Science 232: 341-7 (1986); and Barany and Merrifield, The Peptides, Gross and Meienhofer, eds., Academic Press, New York, 1-284 (1979). Solid phase peptide synthesis may be carried out with an automatic peptide synthesizer (*e.g*., Model 430A, Applied Biosystems Inc., Foster City, California) using the NMP/HOBt (Option 1) system and tBoc or Fmoc chemistry (*see,* Applied Biosystems User's Manual for the ABI 430A Peptide Synthesizer, Version 1.3B July 1, 1988, section 6, pp. 49-70, Applied Biosystems, Inc., Foster City, California) with capping. Peptides may also be assembled using an Advanced Chem Tech Synthesizer (Model MPS 350, Louisville, Kentucky). Peptides may be purified by RP-HPLC (preparative and analytical) using, *e.g*., a Waters Delta Prep 3000 system and a C4, C8, or C18 preparative column (10 µ, 2.2x25 cm; Vydac, Hesperia, California). The active peptide can be readily synthesized and then screened in screening assays designed to identify reactive peptides.

The hybrid polypeptides of the present invention may alternatively be produced by recombinant techniques well known in the art. *See, e.g.,* Sambrook et al., Molecular Cloning: A Laboratory Manual, 2d ed., Cold Spring Harbor (1989). These hybrid polypeptides produced by recombinant technologies may be expressed from a polynucleotide. One skilled in the art will appreciate that the polynucleotides, including DNA and RNA, that encode such the various fragments of the hybrid polypeptides may be obtained from the wild-type cDNA, taking into consideration the degeneracy of codon usage, or may be engineered as desired. These polynucleotide sequences may incorporate codons facilitating transcription and translation of mRNA in microbial hosts. Such manufacturing sequences may readily be constructed according to the methods well known in the art. *See, e.g.,* WO 83/04053. The polynucleotides above may also optionally encode an N-terminal methionyl residue. Non-peptide compounds useful in the present invention may be prepared by art-known methods. For example, phosphate-containing amino acids and peptides containing such amino acids may be prepared using methods known in the art. *See, e.g.,* Bartlett and Landen, Bioorg. Chem. 14: 356-77 (1986).

A variety of expression vector/host systems may be utilized to contain and express a hybrid polypeptide coding sequence. These include but are not limited to microorganisms such as bacteria transformed with recombinant bacteriophage, plasmid or cosmid DNA expression vectors; yeast transformed with yeast expression vectors; insect cell systems infected with virus expression vectors (*e.g*., baculovirus); plant cell systems transfected with virus expression vectors (*e.g*., cauliflower mosaic virus, CaMV; tobacco mosaic virus, TMV) or transformed with bacterial expression vectors (*e.g*., Ti or pBR322 plasmid); or animal cell systems. Mammalian cells that are useful in recombinant protein productions include but are not limited to VERO cells, HeLa cells, Chinese hamster ovary (CHO) cell lines, COS cells (such as COS-7), Wl 38, BHK, HepG2, 3T3, RIN, MDCK, A549, PC12, K562 and 293 cells. Exemplary protocols for the recombinant expression of the protein are described herein below.

As such, polynucleotide sequences provided by the invention arc useful in generating new and useful viral and plasmid DNA vectors, new and useful transformed and transfected procaryotic and eucaryotic host cells (including bacterial, yeast, and mammalian cells grown in culture), and new and useful methods for cultured growth of such host cells capable of expression of the present hybrid polypeptides. The polynucleotide sequences encoding hybrid polypeptides herein may be useful for gene therapy in instances where underproduction of the component peptide hormone(s) of the chimera would be alleviated, or the need for increased levels of such would be met.

The present disclosure also provides for processes for recombinant DNA production of the present hybrid polypeptides. Provided is a process for producing the hybrid polypeptides from a host cell containing nucleic acids encoding such hybrid polypeptides comprising: (a) culturing said host cell containing polynucleotides encoding such hybrid polypeptides under conditions facilitating the expression of such DNA molecule; and (b) obtaining such hybrid polypeptides.

Host cells may be prokaryotic or eukaryotic and include bacteria, mammalian cells (such as Chinese Hamster Ovary (CHO) cells, monkey cells, baby hamster kidney cells, cancer cells or other cells), yeast cells, and insect cells.

Mammalian host systems for the expression of the recombinant protein also are well known to those of skill in the art. Host cell strains may be chosen for a particular ability to process the expressed protein or produce certain post-translation modifications that will be useful in providing protein activity. Such modifications of the polypeptide include, but are not limited to, acetylation, carboxylation, glycosylation, phosphorylation, lipidation and acylation. Post-translational processing, which cleaves a "prepro" form of the protein, may also be important for correct insertion, folding and/or function. Different host cells, such as CHO, HeLa, MDCK, 293, WI38, and the like, have specific cellular machinery and characteristic mechanisms for such post-translational activities, and may be chosen to ensure the correct modification and processing of the introduced foreign protein.

Alternatively, a yeast system may be employed to generate the hybrid polypeptides of the present invention. The coding region of the hybrid polypeptide cDNA is amplified by PCR. A DNA encoding the yeast pre-pro-alpha leader sequence is amplified from yeast genomic DNA in a PCR reaction using one primer containing nucleotides 1-20 of the alpha mating factor gene and another primer complementary to nucleotides 255-235 of this gene (Kurjan and Herskowitz, Cell, 30: 933-43 (1982)). The pre-pro-alpha leader coding sequence and hybrid polypeptide coding sequence fragments are ligated into a plasmid containing the yeast alcohol dehydrogenase (ADH2) promoter, such that the promoter directs expression of a fusion protein consisting of the pre-pro-alpha factor fused to the mature hybrid polypeptide. As taught by Rose and Broach, Meth. Enz. 185: 234-79, Goeddel ed., Academic Press, Inc., San Diego, California (1990), the vector further includes an ADH2 transcription terminator downstream of the cloning site, the yeast "2-micron" replication origin, the yeast leu-2d gene, the yeast REP1 and REP2 genes, the E. coli β-lactamase gene, and an E. coli origin of replication. The β-lactamase and leu-2d genes provide for selection in bacteria and yeast, respectively. The leu-2d gene also facilitates increased copy number of the plasmid in yeast to induce higher levels of expression. The REP1 and REP2 genes encode proteins involved in regulation of the plasmid copy number.

The DNA construct described in the preceding paragraph is transformed into yeast cells using a known method, *e.g*., lithium acetate treatment (Steams et al., Meth. Enz. 185: 280-97 (1990)). The ADH2 promoter is induced upon exhaustion of glucose in the growth media (Price et al., Gene 55: 287 (1987)). The pre-pro-alpha sequence effects secretion of the fusion protein from the cells. Concomitantly, the yeast KEX2 protein cleaves the pre-pro sequence from the mature PYY analog polypeptides (Bitter et al., Proc. Natl. Acad. Sci. USA 81: 5330-4 (1984)).

Hybrid polypeptides of the invention may also be recombinantly expressed in yeast using a commercially available expression system, *e.g*., the Pichia Expression System (Invitrogen, San Diego, California), following the manufacturer's instructions. This system also relies on the pre-pro-alpha sequence to direct secretion, but transcription of the insert is driven by the alcohol oxidase (AOX1) promoter upon induction by methanol. The secreted hybrid polypeptide is purified from the yeast growth medium by, *e.g*., the methods used to purify hybrid polypeptide from bacterial and mammalian cell supernatants.

Alternatively, the cDNA encoding hybrid polypeptides may be cloned into the baculovirus expression vector pVL1393 (PharMingen, San Diego, California). This hybrid polypeptide-containing vector is then used according to the manufacturer's directions (PharMingen) to infect Spodoptera frugiperda cells in sF9 protein-free media and to produce recombinant protein. The protein is purified and concentrated from the media using a heparin-Sepharose column (Pharmacia, Piscataway, New Jersey) and sequential molecular sizing columns (Amicon, Beverly, Massachusetts), and resuspended in PBS. SDS-PAGE analysis shows a single band and confirms the size of the protein, and Edman sequencing on a Proton 2090 Peptide Sequencer confirms its N-terminal sequence.

For example, the DNA sequence encoding the hybrid polypeptide may be cloned into a plasmid containing a desired promoter and, optionally, a leader sequence (*see, e.g.,* Better et al., Science 240: 1041-3 (1988)). The sequence of this construct may be confirmed by automated sequencing. The plasmid is then transformed into E. coli, strain MC1061, using standard procedures employing CaC12 incubation and heat shock treatment of the bacteria (Sambrook *et al*., *supra*). The transformed bacteria are grown in LB medium supplemented with carbenicillin, and production of the expressed protein is induced by growth in a suitable medium. If present, the leader sequence will affect secretion of the hybrid polypeptide and be cleaved during secretion. The secreted recombinant protein is purified from the bacterial culture media by the method described herein below.

Alternatively, the hybrid polypeptides of the invention may be expressed in an insect system. Insect systems for protein expression are well known to those of skill in the art. In one such system, Autographa californica nuclear polyhedrosis virus (AcNPV) is used as a vector to express foreign genes in Spodoptera frugiperda cells or in Trichoplusia larvae. The hybrid polypeptide coding sequence is cloned into a nonessential region of the virus, such as the polyhedrin gene, and placed under control of the polyhedrin promoter. Successful insertion of hybrid polypeptide will render the polyhedrin gene inactive and produce recombinant virus lacking coat protein coat. The recombinant viruses are then used to infect S. frugiperda cells or Trichoplusia larvae in which hybrid polypeptide is expressed (Smith et al., J. Virol 46: 584 (1983); Engelhard et al., Proc. Natl. Acad. Sci. USA 91: 3224-7 (1994)).

In another example, the DNA sequence encoding the hybrid polypeptide may be amplified by PCR and cloned into an appropriate vector, for example, pGEX-3X (Pharmacia, Piscataway, New Jersey). The pGEX vector is designed to produce a fusion protein comprising glutathione-S-transferase (GST), encoded by the vector, and a protein encoded by a DNA fragment inserted into the vector's cloning site. The primers for the PCR may be generated to include, for example, an appropriate cleavage site. The recombinant fusion protein may then be cleaved from the GST portion of the fusion protein. The pGEX-3X/PYY analog polypeptide construct is transformed into E. coli XL-1 Blue cells (Stratagene, La Jolla, California), and individual transformants are isolated and grown at 37°C in LB medium (supplemented with carbenicillin) to an optical density at wavelength 600 nm of 0.4, followed by further incubation for 4 hours in the presence of 0.5 mM Isopropyl β-D-Thiogalactopyranoside (Sigma Chemical Co., St. Louis, Missouri). Plasmid DNA from individual transformants is purified and partially sequenced using an automated sequencer to confirm the presence of the desired PPF hybrid polypeptide-encoding gene insert in the proper orientation.

The fusion protein, expected to be produced as an insoluble inclusion body in the bacteria, may be purified as follows. Cells are harvested by centrifugation; washed in 0.15 M NaCl, 10 mM Tris, pH 8, 1 mM EDTA; and treated with 0.1 mg/mL lysozyme (Sigma Chemical Co.) for 15 min. at room temperature. The lysate is cleared by sonication, and cell debris is pelleted by centrifugation for 10 min. at 12,000xg. The fusion protein-containing pellet is resuspended in 50 mM Tris, pH 8, and 10 mM EDTA, layered over 50% glycerol, and centrifuged for 30 min. at 6000xg. The pellet is resuspended in standard phosphate buffered saline solution (PBS) free of Mg⁺ and Ca⁺⁺. The fusion protein is further purified by fractionating the resuspended pellet in a denaturing SDS polyacrylamide gel (Sambrook et al., supra). The gel is soaked in 0.4 M KCl to visualize the protein, which is excised and electroeluted in gel-running buffer lacking SDS. If the GST/PYY analog polypeptide fusion protein is produced in bacteria as a soluble protein, it may be purified using the GST Purification Module (Pharmacia Biotech).

The fusion protein may be subjected to digestion to cleave the GST from the PPF hybrid polypeptide. The digestion reaction (20-40 µg fusion protein, 20-30 units human thrombin (4000 U/mg (Sigma) in 0.5 mL PBS) is incubated 16-48 hrs. at room temperature and loaded on a denaturing SDS-PAGE gel to fractionate the reaction products. The gel is soaked in 0.4 M KCl to visualize the protein bands. The identity of the protein band corresponding to the expected molecular weight of the hybrid polypeptide may be confirmed by partial amino acid sequence analysis using an automated sequencer (Applied Biosystems Model 473A, Foster City, California).

In a particularly preferred method of recombinant expression of the hybrid polypeptides of the present invention, 293 cells may be co-transfected with plasmids containing the hybrid polypeptide cDNA in the pCMV vector (5' CMV promoter, 3' HGH poly A sequence) and pSV2neo (containing the neo resistance gene) by the calcium phosphate method. Preferably, the vectors should be linearized with ScaI prior to transfection. Similarly, an alternative construct using a similar pCMV vector with the neo gene incorporated can be used. Stable cell lines are selected from single cell clones by limiting dilution in growth media containing 0.5 mg/mL G418 (neomycin-like antibiotic) for 10-14 days. Cell lines are screened for hybrid polypeptide expression by ELISA or Western blot, and high-expressing cell lines are expanded for large scale growth.

It is preferable that the transformed cells are used for long-term, high-yield protein production and as such stable expression is desirable. Once such cells are transformed with vectors that contain selectable markers along with the desired expression cassette, the cells may be allowed to grow for 1-2 days in an enriched media before they are switched to selective media. The selectable marker is designed to confer resistance to selection, and its presence allows growth and recovery of cells that successfully express the introduced sequences. Resistant clumps of stably transformed cells can be proliferated using tissue culture techniques appropriate to the cell.

A number of selection systems may be used to recover the cells that have been transformed for recombinant protein production. Such selection systems include, but are not limited to, HSV thymidine kinase, hypoxanthine-guanine phosphoribosyltransferase and adenine phosphoribosyltransferase genes, in tk-, hgprt- or aprt- cells, respectively. Also, anti-metabolite resistance can be used as the basis of selection for dhfr, that confers resistance to methotrexate; gpt, that confers resistance to mycophenolic acid; neo, that confers resistance to the aminoglycoside, G418; also, that confers resistance to chlorsulfuron; and hygro, that confers resistance to hygromycin. Additional selectable genes that may be useful include trpB, which allows cells to utilize indole in place of tryptophan, or hisD, which allows cells to utilize histinol in place of histidine. Markers that give a visual indication for identification of transformants include anthocyanins, β-glucuronidase and its substrate, GUS, and luciferase and its substrate, luciferin.

Many of the hybrid polypeptides of the present invention may be produced using a combination of both automated peptide synthesis and recombinant techniques. For example, a hybrid polypeptide of the present invention may contain a combination of modifications including deletion, substitution, and insertion by PEGylation. Such a hybrid polypeptide may be produced in stages. In the first stage, an intermediate polypeptide containing the modifications of deletion, substitution, insertion, and any combination thereof, may be produced by recombinant techniques as described. Then after an optional purification step as described below, the intermediate polypeptide is PEGylated through chemical modification with an appropriate PEGylating reagent (e.g., from Nectar Transforming Therapeutics, San Carlos, California) to yield the desired hybrid polypeptide. One skilled in the art will appreciate that the above-described procedure may be generalized to apply to a hybrid polypeptide containing a combination of modifications selected from deletion, substitution, insertion, derivation, and other means of modification well known in the art and contemplated by the present invention.

It may be desirable to purify the hybrid polypeptides generated by the present invention. Peptide purification techniques are well known to those of skill in the art. These techniques involve, at one level, the crude fractionation of the cellular milieu to polypeptide and non-polypeptide fractions. Having separated the polypeptide from other proteins, the polypeptide of interest may be further purified using chromatographic and electrophoretic techniques to achieve partial or complete purification (or purification to homogeneity). Analytical methods particularly suited to the preparation of a pure peptide are ion-exchange chromatography, exclusion chromatography, polyacrylamide gel electrophoresis, and isoelectric focusing. A particularly efficient method of purifying peptides is reverse phase HPLC, followed by characterization of purified product by liquid chromatography/mass spectrometry (LC/MS) and Matrix-Assisted Laser Desorption Ionization (MALDI) mass spectrometry. Additional confirmation of purity is obtained by determining amino acid analysis.

Certain aspects of the present invention concern the purification, and in particular embodiments, the substantial purification, of an encoded protein or peptide. The term "purified peptide" as used herein, is intended to refer to a composition, isolatable from other components, wherein the peptide is purified to any degree relative to its naturally obtainable state. A purified peptide therefore also refers to a peptide, free from the environment in which it may naturally occur.

Generally, "purified" will refer to a peptide composition that has been subjected to fractionation to remove various other components, and which composition substantially retains its expressed biological activity. Where the term "substantially purified" is used, this designation will refer to a composition in which the peptide forms the major component of the composition, such as constituting about 50%, about 60%, about 70%, about 80%, about 90%, about 95% or more of the peptides in the composition.

Various techniques suitable for use in peptide purification will be well known to those of skill in the art. These include, for example, precipitation with ammonium sulphate, PEG, antibodies, and the like; heat denaturation, followed by centrifugation; chromatography steps such as ion exchange, gel filtration, reverse phase, hydroxylapatite and affinity chromatography; isoelectric focusing; gel electrophoresis; and combinations of such and other techniques. As is generally known in the art, it is believed that the order of conducting the various purification steps may be changed, or that certain steps may be omitted, and still result in a suitable method for the preparation of a substantially purified protein or peptide.

There is no general requirement that the peptides always be provided in their most purified state. Indeed, it is contemplated that less substantially purified products will have utility in certain embodiments. Partial purification may be accomplished by using fewer purification steps in combination, or by utilizing different forms of the same general purification scheme. For example, it is appreciated that a cation-exchange column chromatography performed, utilizing an HPLC apparatus, will generally result in a greater "-fold" purification than the same technique utilizing a low pressure chromatography system. Methods exhibiting a lower degree of relative purification may have advantages in total recovery of protein product; or in maintaining the activity of an expressed protein.

One may optionally purify and isolate such hybrid polypeptides from other components obtained in the process. Methods for purifying a polypeptide can be found in U.S. Patent No. 5,849,883. These documents describe specific exemplary methods for the isolation and purification of G-CSF compositions that may be useful in isolating and purifying the hybrid polypeptides of the present invention. Given the disclosure of these patents, it is evident that one of skill in the art would be well aware of numerous purification techniques that may be used to purify hybrid polypeptides from a given source.

Also it is contemplated that a combination of anion exchange and immunoaffinity chromatography may be employed to produce purified hybrid polypeptide compositions of the present invention.

### Pharmaceutical compositions

The present invention also relates to pharmaceutical compositions according to the claims comprising a therapeutically or prophylactically effective amount of at least one hybrid polypeptide of the invention, or a pharmaceutically acceptable salt thereof, together with pharmaceutically acceptable diluents, preservatives, solubilizers, emulsifiers, adjuvants and/or carriers useful in the delivery of the hybrid polypeptides. Such compositions may include diluents of various buffer content (*e.g*., Tris-HCl, acetate, phosphate), pH and ionic strength; additives such as detergents and solubilizing agents (*e.g*., Tween 80, Polysorbate 80), anti-oxidants (*e.g*., ascorbic acid, sodium metabisulfite), preservatives (*e.g*., thimersol, benzyl alcohol), and bulking substances (*e.g*., lactose, mannitol); incorporation of the material into particulate preparations of polymeric compounds, such as polylactic acid, polyglycolic acid, *etc.,* or in association with liposomes. Such compositions will influence the physical state, stability, rate of *in vivo* release, and rate of in vivo clearance of the present hybrid polypeptides. *See, e.g.,* Remington's Pharmaceutical Sciences 1435-7I2, 18th ed., Mack Publishing Co., Easton, Pennsylvania (1990).

In general, the present hybrid polypeptides will be useful in the same way that the individual component polypeptides are useful in view of their pharmacological properties. One preferred use is to peripherally administer such hybrid polypeptides for the treatment or prevention of metabolic conditions and disorders. In particular, the compounds of the invention possess activity as agents to reduce nutrient availability, reduce of food intake, and effect weight loss. In another embodiment, a preferred use is to administer such hybrid polypeptides for the treatment of diabetes or diabetes related conditions and disorders.

The present hybrid polypeptides may be formulated for peripheral administration, including formulation for injection, oral administration, nasal administration, pulmonary administration, topical administration, or other types of administration as one skilled in the art will recognize. More particularly, administration of the pharmaceutical compositions according to the present invention may be via any common route so long as the target tissue is available *via* that route. In a preferred embodiment, the pharmaceutical compositions may be introduced into the subject by any conventional peripheral method, *e.g*., by intravenous, intradermal, intramusclar, intramammary, intraperitoneal, intrathecal, retrobulbar, intrapulmonary (*e.g*., term release); by oral, sublingual, nasal, anal, vaginal, or transdermal delivery, or by surgical implantation at a particular site. The treatment may consist of a single dose or a plurality of doses over a period of time. Controlled continual release of the compositions of the present invention is also contemplated.

The formulation may be liquid or may be solid, such as lyophilized, for reconstitution. Aqueous compositions of the present invention comprise an effective amount of the hybrid polypeptide, dissolved or dispersed in a pharmaceutically acceptable carrier or aqueous medium. The phrase "pharmaceutically or pharmacologically acceptable" refer to molecular entities and compositions that do not produce adverse, allergic, or other untoward reactions when administered to an animal or a human. As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in therapeutic compositions is contemplated. Supplementary active ingredients also can be incorporated into the compositions. In some cases, it will be convenient to provide a hybrid polypeptide of the invention and another food-intake-reducing, diabetes treating, plasma glucose-lowering, or plasma lipid-altering agent, such as an amylin, an amylin agonist analog, a CCK or CCK agonist, or a leptin or leptin agonist, or an exendin or exendin agonist analog, in a single composition or solution for administration together. In other cases, it may be more advantageous to administer the additional agent separately from said hybrid polypeptide.

The hybrid polypeptide of the invention may be prepared for administration as solutions of free base, or pharmacologically acceptable salts in water suitably mixed with a surfactant, such as hydroxypropylcellulose. Pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups also can be derived from inorganic bases such as, for example, sodium, potassium, ammonium, calcium, or ferric hydroxides, and such organic bases as isopropylamine, trimethylamine, histidine, procaine and the like. Such products are readily prepared by procedures well known to those skilled in the art. Dispersions also can be prepared in glycerol, liquid polyethylene glycols, and mixtures thereof and in oils. Under ordinary conditions of storage and use, these preparations contain a preservative to prevent the growth of microorganisms.

In one embodiment, the pharmaceutical compositions of the present invention are formulated so as to be suitable for parenteral administration, *e.g*., via injection or infusion. Preferably, the hybrid polypeptide is suspended in an aqueous carrier, for example, in an isotonic buffer solution at a pH of about 3.0 to about 8.0, preferably at a pH of about 3.5 to about 7.4, 3.5 to 6.0, or 3.5 to about 5.0. Useful buffers include sodium citrate-citric acid and sodium phosphate-phosphoric acid, and sodium acetate/acetic acid buffers. A form of repository or "depot" slow release preparation may be used so that therapeutically effective amounts of the preparation are delivered into the bloodstream over many hours or days following transdermal injection or delivery.

The pharmaceutical compositions suitable for injectable use include sterile aqueous solutions or dispersions and sterile powders for the extemporaneous preparation of sterile injectable solutions or dispersions. In all cases, the form should be sterile and should be fluid to the extent that easy syringability exists. It is also desirable for the hybrid polypeptide of the invention to be stable under the conditions of manufacture and storage and must be preserved against the contaminating action of microorganisms, such as bacteria and fungi: The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial an antifungal agents, for example, parabens, chlorobutanol, phenol, sorbic acid, thimerosal, and the like. In many cases, it will be preferable to include isotonic agents (for example, sugars or sodium chloride). Prolonged absorption of the injectable compositions can be brought about by the use in the compositions of agents delaying absorption (for example, aluminum monostearate and gelatin).

Sterile injectable solutions may be prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle that contains the basic dispersion medium and the required other ingredients from those enumerated above. In the case of sterile powders for the preparation of sterile injectable solutions, the preferred methods of preparation are vacuum-drying and freeze-drying techniques that yield a powder of the active ingredient plus any additional desired ingredient from a previously sterile-filtered solution thereof.

Generally, a therapeutically or prophylactically effective amount of the present hybrid polypeptides will be determined by the age, weight, and condition or severity of the diseases, conditions or disorders of the recipient. *See, e.g.,* Remington's Pharmaceutical Sciences 697-773. *See also* Wang and Hanson, Parenteral Formulations of Proteins and Peptides Stability and Stabilizers, Journal of Parenteral Science and Technology, Technical Report No. 10, Supp. 42:2S (1988). Typically, a dosage of between about 0.001 µg/kg body weight/day to about 1000 µg/kg body weight/day, may be used, but more or less, as a skilled practitioner will recognize, may be used. Dosing may be one or more times daily, or less frequently, and may be in conjunction with other compositions as described herein. It should be noted that the present invention is not limited to the dosages recited herein.

Appropriate dosages may be ascertained through the use of established assays for determining level of metabolic conditions or disorders in conjunction with relevant dose-response data. The final dosage regimen will be determined by the attending physician, considering factors that modify the action of drugs, *e.g*., the drug's specific activity, severity of the damage and the responsiveness of the patient, the age, condition, body weight, sex and diet of the patient, the severity of any infection, time of administration and other clinical factors. As studies are conducted, further information will emerge regarding appropriate dosage levels and duration of treatment for specific diseases and conditions.

An effective dose will typically be in the range of about 1 to 30 µg to about 5 mg/day, preferably about 10 to 30 µg to about 2 mg/day and more preferably about 5 to 100 µg to about 1 mg/day, most preferably about 5 µg to about 500 µg/day, for a 50 kg patient, administered in a single or divided doses. Preferably, dosages are between about 0.01 to about 100 µg/kg/dose. The exact dose to be administered may be determined by one of skill in the art and is dependent upon the potency of the particular compound, as well as upon the age, weight and condition of the individual. Administration should begin whenever, *e.g*., suppression of nutrient availability, food intake, weight, blood glucose or plasma lipid modulation is desired, for example, at the first sign of symptoms or shortly after diagnosis of obesity, diabetes mellitus, or insulin-resistance syndrome. Administration may be by any route, *e.g*., injection, preferably subcutaneous or intramuscular, oral, nasal, transdermal, *etc*. Dosages for certain routes, for example oral administration, may be increased to account for decreased bioavailablity, for example, by about 5-100 fold.

In one embodiments, where the pharmaceutical formulation is to be administered parenterally, the composition is formulation so as to deliver a dose of hybrid polypeptide ranging from 1 µg/kg to 100 mg/kg body weight/day, preferably at doses ranging from 0.1 mg/kg to about 50 mg/kg body weight/day. Parenteral administration may be carried out with an initial bolus followed by continuous infusion to maintain therapeutic circulating levels of drug product. Those of ordinary skill in the art will readily optimize effective dosages and administration regimens as determined by good medical practice and the clinical condition of the individual patient.

The frequency of dosing will depend on the pharmacokinetic parameters of the agents and the routes of administration. The optimal pharmaceutical formulation will be determined by one of skill in the art depending on the route of administration and the desired dosage. *See, e.g.,* Remington's Pharmaceutical Sciences, supra, pages 1435-1712. Such formulations may influence the physical state, stability, rate of in vivo release and rate of in vivo clearance of the administered agents. Depending on the route of administration, a suitable dose may be calculated according to body weight, body surface areas or organ size. Further refinement of the calculations necessary to determine the appropriate treatment dose is routinely made by those of ordinary skill in the art without undue experimentation, especially in light of the dosage information and assays disclosed herein, as well as the pharmacokinetic data observed in animals or human clinical trials.

It will be appreciated that the pharmaceutical compositions and the compositions for use in treatment methods of the invention may be useful in fields of human medicine and veterinary medicine. Thus the subject to be treated may be a mammal, preferably human or other animal. For veterinary purposes, subjects include for example, farm animals including cows, sheep, pigs, horses and goats, companion animals such as dogs and cats, exotic and/or zoo animals, laboratory animals including mice, rats, rabbits, guinea pigs and hamsters; and poultry such as chickens, turkeys, ducks and geese.

In addition, the present disclosure contemplates a kit comprising a hybrid polypeptide of the disclosure, components suitable for preparing said hybrid polypeptide of the invention for pharmaceutical application, and instructions for using said hybrid polypeptide and components for pharmaceutical application.

To assist in understanding the present invention, the following Examples are included. The experiments relating to this invention should not, of course, be construed as specifically limiting the invention and such variations of the invention, now known or later developed, which would be within the purview of one skilled in the art are considered to fall within the scope of the invention as described herein and hereinafter claimed.

### EXAMPLES

The present invention is described in more detail with reference to the following nonlimiting examples, which are offered to more fully illustrate the invention, but are not to be construed as limiting the scope thereof. The examples illustrate the preparation of the present hybrid polypeptides, and the testing of these hybrid polypeptides of the invention *in vitro* and/or *in vivo.* Those of skill in the art will understand that the techniques described in these examples represent techniques described by the inventors to function well in the practice of the invention, and as such constitute preferred modes for the practice thereof. However, it should be appreciated that those of skill in the art should in light of the present disclosure, appreciate that many changes can be made in the specific methods that are disclosed and still obtain a like or similar result without departing from the spirit and scope of the invention.

### Example 1. Preparation of Hybrid Polypeptides

Peptides of the invention may be assembled on a Symphony peptide synthesizer (Protein Technologies, Inc.) using Rink amide resin (Novabiochem) with a loading of 0.43-0.49 mmol/g at 0.050-0.100 mmol or a pre-loaded Wang Resin (Fmoc-Tyr(tBu)-Wang resin) 0.63 mmol/g (Novabiochem). Fmoc amino acid (5.0 eq, 0.250-.500 mmol) residues are dissolved at a concentration of 0.10 M in 1-methyl-2-pyrrolidinone. All other reagents (HBTU, 1-Hydroxybenzotriazole hydrate and N,N-Diisopropylethylamine ) are prepared as 0.55 M Dimethylformamide solutions. The Fmoc protected amino acids are then coupled to the resin-bound amino acid using, HBTU (2.0 eq, 0.100-0.200 mmol), 1-Hydroxybenzotriazole hydrate (1.8 eq, 0.090-0.18 mmol), NN'-Diisopropylethylamine (2.4 eq, 0.120-0.240 mmol) for 2 hours. Following the last amino acid coupling, the peptide is deprotected using 20% (v/v) piperidine in dimethylformamide for 1 hour. Once peptide sequence is complete, the Symphony peptide synthesizer is programmed to cleave the resin. Trifluoroacetic acid (TFA) cleavage of the peptide from resin is carried out using 93% TFA, 3% phenol, 3% water and 1% triisopropylsilane for 1 hour. The cleaved peptide is precipitated using tert-butyl methyl ether, pelleted by centrifugation and lyophilized. The pellet is re-dissolved in water (10-15 mL), filtered and purified via reverse phase HPLC using a C18 column and an acetonitrile/water gradient containing 0.1% TFA.

A general procedure for N-capping the peptides of the invention with fatty acids (*e.g*., octanoic and stearic acids) is as follows: Peptide on rink amide resin (0.1 mmol) is suspended in NMP (5 mL). In a separate vial, HBTU (0.3 mmol), HOBt (0.3 mmol) is dissolved in DMF (5 mL) followed by the addition of DIEA (0.6 mmol). This solution is added to the resin and this suspension is shaken for 2 hrs. The solvent is filtered and washed thoroughly with NMP (5 mLx4) and CH₂Cl₂ (20 mL), dried and is subjected to the TFA cleavage for 1 hr. The yield of the desired peptide is ca. 40 mg after cleavage and purification.

PEG modification may be carried out in solution on a free epsilon-amino group of lysine or a terminal amino group of a purified peptide using commercially available activated PEG esters. The resulting PEGylated derivatives are purified to homogeneity by reverse phase HPLC and the purity is confirmed by LC/MS and MALDI-MS.

Certain exemplary hybrid polypeptides of the invention are shown below in Table 1-1. Various modifications to the embodied compounds are envisioned, such as chemical modifications such as glycosylation, PEG modifications, etc.; amino acid modifications such as substitutions, insertions and deletions, etc. Further, even though represented as C-terminally amidated, it is understood that the hybrid polypeptides of the invention may alternatively be in the free acid form.

**Table 1-1: Certain Exemplary Hybrid Compounds of the Invention**

| SEQ ID: | |
|---|---|
| 1 | HGEGTFTSDLSKQMEEEAVRLFIEWLKNGGPSSGAPPPS-ASLRHYLNLVTRQRY-NH₂ |
| 2 | HGEGTFTSDLSKQMEEEAVRLFIEWLKNGGPSSGAPPPS-RHYLNLVTRQRY-NH₂ |
| 3 | HGEGTFTSDLSKQ1V.IEEEEAVRLFIEWLKNGGPSSGAPPPS-NRYYASLRHYLNLVTRQRY-NH₂ |
| 4 | HGEGTFTSDLSKQMEEEAVRLFIEWLKNGGPSSGAPPPS-(3Ala-(3Ala-ASLRHYLNLVTRQRY-NH₂ |
| 5 | HGEGTFTSDLSKQMEEEAVRLFIEWLKNGGPSSGAPPPS-βAla-βAla-RHYLNLVTRQRY-NH₂ |
| 6 | HGEGTFTSDLSKQMEEEAVRLFIEWLKNGGPSSGAPPPS-βAla-βAla-VTRQRY-NH₂ |
| 7 | HGEGTFTSDLSKQMEEEAVRLFIEWLKN-ASLRHYLNLVTRQRY-NH₂ |
| 8 | HGEGTFTSDLSKQMEEEAVRLFIEWLKN-RHYLNLVTRQRY-NH₂ |
| .9 | HGEGTFTSDLSKQMEEEAVRLFIEWLKN-NRYYASLRHYLNLVTRQRY-NH₂ |
| 10 | HGEGTFTSDLSKQMEEEAVRLFIEWLKN-βAla-βAla-ASLRHYLNLVTRQRY-NH₂ |
| 11 | HGEGTFTSDLSKQMEEEAVRLFIEWLKN-βAla-βAla-RHYLNLVTRQRY-NH₂ |
| 12 | HGEGTFTSDLSKQMEEEAVRLFIEWLKN-βAla-βAla-VTRQRY-NH₂ |
| 13 | HGEGAFTSDLSKQLEEEAVRLFIEFLKNNRYYASLRHYLNLVTRQRY-NH₂ |
| 14 | HGEGAFTSDLSKQLEEEAVRLFIEFLKNASLRHYLNLVTRQRY-NH₂ |
| 15 | HGEGAFTSDLSKQLEEEAVRLFIEFLKNRHYLNLVTRQRY-NH₂ |
| 16 | HGEGAFTSDLSKQLEEENRYYASLRHYLNLVTRQRY-NH₂ |
| 17 | HGEGAFTSDLSKQLEEEAVRLFIEFLKN-βAla-βAla-ASLRHYLNLVTRQRY-NH₂ |
| 18 | HGEGAFTSDLSKQLEEEAVRLFIEFLKN-βAla-βAla-RHYLNLVTRQRY-NH₂ |
| 19 | HGEGAFTSDLSKQLEEEAVRLFIEFLKN-βAla-βAla-VTRQRY-N NH₂ |
| 20 | HGEGTFTSDLSKQMEEEAVRLFIEWLKNGGPSSGAPPPS-DY(SO₃)MGWMDF-NH₂ |
| 21 | HGEGTFTSDLSKQMEEEAVRLFIEWLKN-DY(SO₃)MGWMDF-NH₂ |
| 22 | HGEGTFTSDLSKQMEEEAVRLFIEWLKN-DF(CH₂SO₃)MGWMDF-NH₂ |
| 23 | HGEGTFTSDLSKQMEEEAVRLFIEWLKN-[8-amino- 3,6- dioxaoctanoyl]-DY(SO₃)MGWMDF-NH₂ |
| 24 | HGEGTFTSDLSKQMEEEAVRLFIEWLKN-[8-amino-3,6-dioxactoanoyl]- DF(CH₂SO₃)MGWMDF-NH |
| 25 | HGEGTFTSDLSKQMEEEAVRLFIEWLKKCNTATCVLGRLSQELHRLQTYPRTNTGSNTY-NH₂ |
| 26 | HGEGTFTSDLSKQMEEEAVRLFIEWLKKANTATAVLG-NH₂ |
| 27 | HGEGTFTSDLSKQMEEEAVRLFIEWLKN-12-Ado-KCNTATCVLGRLHRLQTYPRTNTGSNTY-NH₂ |
| 28 | HGEGTFTSDLSKQMEEEAVRLFIEWLKN-12-Ado-CNTATCVLGRLHRLQTYPRTNTGSNTY-NH₂ |
| 29 | HGEGTFTSDLSKQMEEEAVRLFIEWLKN-3,6-dioxaoctanoyl-KCNTATCVLGRLHRLQTYPRTNTGSNTY-NH₂ |
| 30 | HGEGTFTSDLSKQMEEEAVRLFIEWLKN-3,6-dioxaoctanoyl-CNTATCVLGRLHRLQTYPRTNTGSNTY-NH₂ |
| 31 | HGEGTFTSDLSKQMEEEAVRLFIEWLKN-5-Apa-KCNTATCVLGRLHRLQTYPRTNTGSNTY-NH₂ |
| 32 | HGEGTFTSDLSKQMEEEAVRLFIEWLKN-5-Apa-CNTATCVLGRLHRLQTYPRTNTGSNTY-NH₂ |
| 33 | HGEGTFTSDLSKQMEEEAVRLFIEWLKN-βAla-βAla-KCNTATCVLGRLHRLQTYPRTNTGSNTY-NH₂ |
| 34 | HGEGTFTSDLSKQMEEEAVRLFIEWLKN-βAla-βAla-CNTATCVLGRLHRLQTYPRTNTGSNTY-NH₂ |
| 35 | HGEGTFTSDLSKQMEEEAVRLFIEWLKN-4,7,10-trioxa-13-tridecanamine succinimidyl- KCNTATCVLGRLHRLQTYPRTNTGSNTY-NH2 |
| 36 | HGEGTFTSDLSKQMEEEAVRLFIEWLKN-4,7,10-trioxa-13-tridecanamine succinimidyl- CNTATCVLGRLHRLQTYPRTNTGSNTY-NH2 |
| 37 | DF(CH2SO3)MGWMDF-*GKR*-KCNTATCATQRLANELVRLQTYPRTNVGSNTY-NH2 |
| 38 | KCNTATCATQRLANFLVR-RYYASLRHYLNLVTRQRY-NH2 |
| 39 | isocaproyl-STAVL-(Aib)-K(formyl)-LSQEL-(Aib)-K(formyl)-LQT-NRYYASLRHYLNLVTRQRY-NH₂ |
| 40 | isocaproyl-STAVL-(Aib)-K(formyl)-LSQEL-(Aib)-K(formyl)-L-ELNRYYASLRHYLNLVTRQRY-NH₂ |
| 41 | 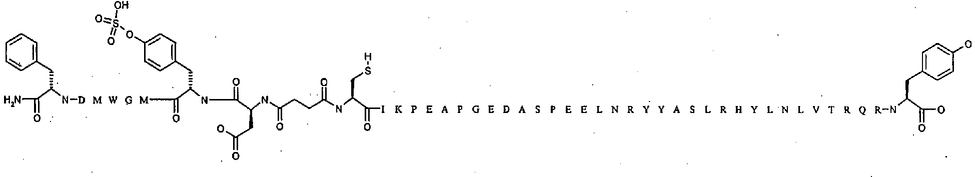 |
| 42 | 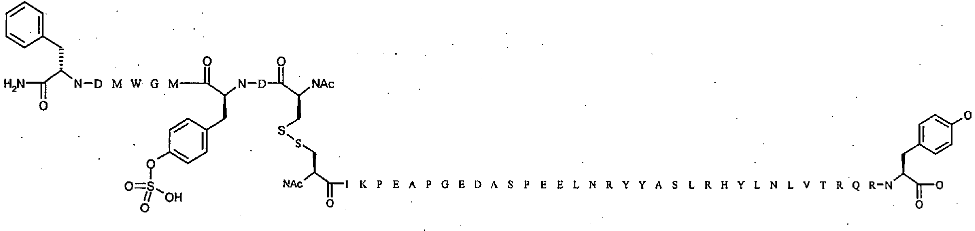 |
| 43 | 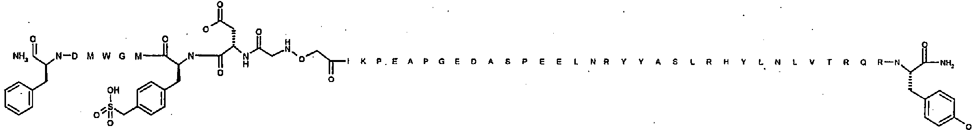 |

### Example 2. Binding Assays

The hybrid polypeptides of the invention may be tested in a variety of receptor binding assays using binding assay methodologies generally known to those skilled in the art. Such assays include those described below.

Amylin binding assay: Evaluation of the binding of some exemplary compounds of the invention to amylin receptors may be carried out as follows in nuclueus accumbens membranes prepared from rat brain. Male Sprague-Dawley® rats (200-250) grams are sacrificed by decapitation. Brains are removed and place in cold phosphate-buffered saline (PBS). From the ventral surface, cuts are made rostral to the hypothalamus, bounded laterally by the olfactory tracts and extending at a 45° angle medially from these tracts. This basal forebrain tissue, containing the nucleus accumbens and surrounding regions, is weighed and homogenized in ice-cold 20 mM HEPES buffer (20 mM HEPES acid, pH adjusted to 7.4 with NaOH at 23°C). Membranes are washed three times in fresh buffer by centrifugation for 15 minutes at 48,000 x g. The final membrane pellet is resuspended in 20 mM HEPES buffer containing 0.2 mM phenylmethylsulfonyl fluoride (PMSF).

To measure ¹²⁵I_amylin binding (see, Beaumont K et al. Can J Physiol Pharmacol. 1995 Jul; 73(7):1025-9), membranes from 4 mg original wet weight of tissue are incubated with ¹²⁵I -amylin at 12-16 pM in 20 mM HEPES buffer containing 0.5 mg/ml bacitracin, 0.5 mg/ml bovine serum albumin, and 0.2 mM PMSF. Solutions are incubated for 60 minutes at 2°C. Incubations are terminated by filtration through GF/B glass fiber filters (Whatman Inc., Clifton, N.J.) that are presoaked for 4 hours in 0.3% poylethyleneimine in order to reduce nonspecific binding of radiolabeled peptides. Filters are washed immediately before filtration with 5 ml cold PBS, and immediately after filtration with 15 ml cold PBS. Filters are removed and radioactivity assessed in a gamma-counter at a counting efficiency of 77%. Competition curves are generated by measuring binding in the presence of 10⁻¹² to 10⁻⁶ M unlabeled test compound and are analyzed by nonlinear regression using a 4-parameter logistic equation (Inplot program; GraphPAD Software, San Diego).

CGRP receptor binding assay: Evaluation of the binding of compounds of the disclosure, to CGRP receptors are essentially as described for amylin except using membranes prepared from SK-N-MC cells, known to express CGRP receptors (Muff, R. et.al., Ann NY Acad. Sci. 1992: 657, 106-16). Binding assays are performed as described for amylin except using 13,500 cpm 125I-hCGRP /well or 21.7 pM/well (Amersham).

Adrenomedullin binding assay: Binding to the adrenomedullin receptor may be investigated using HUVECs that contain the adrenomedullin receptor (Kato J et.al., Eur J Pharmacol. 1995, 289:383-5) using the Perkin Elmer AlphaScreen^{™} assay for cyclic AMP using an optimum of 25-30,000 cells per well. Elevation of cAMP levels is not large for HUVEC compared to CHO cells. As such, CHO cells may be chosen as a negative control since they do not express the adrenomedullin receptor if desired. Calcitonin receptor binding assay: Binding to the calcitonin receptor may be investigated using CHO cells or T47D cells, which also express the calcitonin receptor (Muff R. et.al, Ann N Y Acad Sci. 1992, 657:106-16 and Kuestner R.E. et. al. Mol Pharmacol. 1994, 46:246-55), as known in the art

Leptin binding assay: Two *in vitro* bioassays are routinely used to assess leptin binding and receptor activation (see *e.g.,* White, et al., 1997. Proc.Natl. acid. Sci. U. S. A. 94: 10657-10662). An alkaline phosphatase("AP")-leptin ("OB") fusion protein ("AP-OB") may be used to measure inhibition of leptin binding in the absence or presence of recombinant mouse leptin (positive control) or peptide, by COS-7 cells transfected with the long (signaling) form of the mouse OB receptor ("OB-RL"). Signal transduction assays may be done in GT1-7 cells cotransfected with AP reporter and OB-RL constructs. Secreted alkaline phosphatase("SEAP") activity in response to stimulation with mouse leptin or peptide may be measured by chemiluminescence.

Y1 receptor binding assay: Membranes are prepared from confluent cultures of SK-N-MC cells that endogenously expresses the neuropeptide Y1 receptors. Membranes are incubated with 60 pM [¹²⁵I]- human Peptide YY (2200 Ci/mmol, PerkinElmer Life Sciences), and with unlabeled PPF polypeptide for 60 minutes at ambient temperature in a 96 well polystyrene plate. Then well contents are harvested onto a 96 well glass fiber plate using a Perkin Elmer plate harvestor. Dried glass fiber plates are combined with scintillant and counted on a Perkin Elmer scintillation counter.

Y2 receptor binding assay: Membranes are prepared from confluent cultures of SK-N-BE cells that endogenously expresses the neuropeptide Y2 receptors. Membranes are incubated with 30 pM [¹²⁵I]- human Peptide YY (2200 Ci/mmol, PerkinElmer Life Sciences), and with unlabeled PPF polypeptide for 60 minutes at ambient temperature in a 96 well polystyrene plate. Then well contents are harvested onto a 96 well glass fiber plate using a Perkin Elmer plate harvestor. Dried glass fiber plates are combined with scintillant and counted on a Perkin Elmer scintillation counter.

Y4 receptor binding assay: CHO-K1 cells are transiently transfected with cDNA encoding neuropeptide Y4 gene, and then forty-eight hours later membranes are prepared from confluent cell cultures. Membranes are incubated with 18 pM [¹²⁵I] human Pancreatic Polypeptide (2200 Ci/mmol, PerkinElmer Life Sciences), and with unlabeled PPF polypeptide for 60 minutes at ambient temperature in a 96 well polystyrene plate. Then well contents are harvested onto a 96 well glass fiber plate using a Perkin Elmer plate harvestor. Dried glass fiber plates are combined with scintillant and counted on a Perkin Elmer scintillation counter.

Y5 receptor binding assay: CHO-K1 cells are transiently transfected with cDNA encoding neuropeptide Y5 gene, and then forty-eight hours later membranes are prepared from confluent cell cultures. Membranes are incubated with 44 pM [¹²⁵I]- human Peptide YY (2200 Ci/mmol, PerkinElmer Life Sciences), and with unlabeled PPF polypeptide for 60 minutes at ambient temperature in a 96 well polystyrene plate. Then well contents are harvested onto a 96 well glass fiber plate using a Perkin Elmer plate harvestor. Dried glass fiber plates are combined with scintillant and counted on a Perkin Elmer scintillation counter.

GLP-1 receptor binding assay: GLP-1 receptor binding activity and affinity may be measured using a binding displacement assay in which the receptor source is RINm5F cell membranes, and the ligand is [¹²⁵I]GLP-1. Homogenized RINm5F cell membranes are incubated in 20 mM HEPES buffer with 40,000 cpm [¹²⁵I]GLP-1 tracer, and varying concentrations of test compound for 2 hours at 23° C with constant mixing. Reaction mixtures are filtered through glass filter pads presoaked with 0.3% PEI solution and rinsed with ice-cold phosphate buffered saline. Bound counts are determined using a scintillation counter. Binding affinities are calculated using GraphPad Prism software (GraphPad Software, Inc., San Diego, CA).

### Example 3: Mouse Food Intake Assay

The hybrid polypeptides of the invention may be tested for appetite suppression in the mouse food intake assay and for their effect on body weight gain in diet-induced obesity (DIO) mice. The experimental protocols for the screens are described below.

Female NIH/Swiss mice (8-24 weeks old) are group housed with a 12:12 hour light:dark cycle with lights on at 0600. Water and a standard pelleted mouse chow diet are available ad libitum, except as noted. Animals are fasted starting at approximately 1500 hrs, 1 day prior to experiment. The morning of the experiment, animals are divided into experimental groups. In a typical study, n=4 cages with 3 mice/cage.

At time=0 min, all animals are given an intraperitoneal injection of vehicle or compound in an amount ranging from about 10 nmol/kg to 75 nmol/kg, and immediately given a pre-weighed amount (10-15g) of the standard chow. Food is removed and weighed at 30, 60, and 120 min to determine the amount of food consumed (Morley, Flood et al., Am. J. Physiol. 267: R178-R184, 1994). Food intake is calculated by subtracting the weight of the food remaining after the e.g., 30, 60, 120, 180 and/or 240 minute time point from the weight of the food provided initially at time=0. Significant treatment effects were identified by ANOVA (p<0.05). Where a significant difference exists, test means are compared to the control mean using Dunnett's test (Prism v. 2.01, GraphPad Software Inc., San Diego, California).

### Example 4: Body Weight Gain in Fattened C57B1/6 (Diet-induced-obesity, or DIO) Mice

Male C57BL/6 mice (4 weeks old at start of study) are fed high fat (HF, 58% of dietary kcal as fat) or low fat (LF, 11% of dietary kcal as fat) chow. After 4 weeks on chow, each mouse is implanted with an osmotic pump (Alzet # 2002) that subcutaneously delivers a predetermined dose of hybrid polypeptide continuously for two weeks. Body weight and food intake are measured weekly (Surwit et al., Metabolism-Clinical and Experimental, 44: (645-51, 1995). Effects of the test compound are expressed as the mean +/- sd of % body weight change (*i.e*., % change from starting weight) of at least 14 mice per treatment group (p<0.05 ANOVA, Dunnett's test, Prism v. 2.01, GraphPad Software Inc., San Diego, California).

### Exendin/PYY Hybrids;

Exemplary hybrid polypeptides of the disclosure were synthesized using a C-terminally truncated exendins (e.g., exendin-4(1-28) or ⁵Ala,¹⁴Leu,²⁵Phe-exendin-4(1-28)) and an N-terminally truncated PYY spanning the 18-36 to 31-36 regions. As such, the exemplary hybrid polypeptides generally comprise two modules, wherein the first module is a fragment of an exendin-4 analog and the second module is a peptidic enhancer selected from PYY truncations. For comparison, a β-alanine dipeptide spacers were also incorporated between the peptide building blocks in several variants (see Table 4-1).

**Table 4-1; Exendin/PYY Hybrids and Their Effects in the Food Intake Assay**

| | *Mouse Food Intake % basal* | | | |
|---|---|---|---|---|
| *Description* | *30min* | *60min* | *120min* | *Dose* |
| ⁵Ala, ¹⁴Leu, ²⁵Phe-exendin-4(1-17)-PYY(18-36) | -4 | -11 | -10 | 10 mmol/Kg |
| ⁵Ala, ¹⁴Leu, ²⁵Phe-exendin-4(1-28)-PYY(22-36) | 21 | 9 | -4 | 10 nmol/Kg |
| ⁵Ala,¹⁴Leu,²⁵Phe-exendin-4(1-28)-βAla-βAla-PYY(22-36) | -3 | -5 | -22 | 10 nmol/Kg |
| ⁵Ala,¹⁴Leu,²⁵Phe-exendin-4(1-28)-PYY(25-36) | 8 | -13 | -30 | 10 nmol/Kg |
| ⁵Ala,¹⁴Leu,²⁵Phe-exendin-4(1-28)-βAla-βAla-PYY(25-36) | -9 | -25 | -42 | 10 nmol/Kg |
| ⁵Ala,¹⁴Leu,²⁵Phe-exendin-4(1-28)-βAla-βAla-PYY(31-36) | -14 | -36 | -52 | 10 nmol/Kg |
| exendin-4(1-28)-PYY(25-36) | -30 | -37 | -45 | 10 nmol/Kg |
| exendin-4(1-28)- βAla-βAla-PYY(25-36) | -24 | -40 | -52 | 10 nmol/Kg |
| exendin-4(1-28)- βAla-βAla-PYY(31-36) | -49 | -56 | -61 | 10 nmol/Kg |

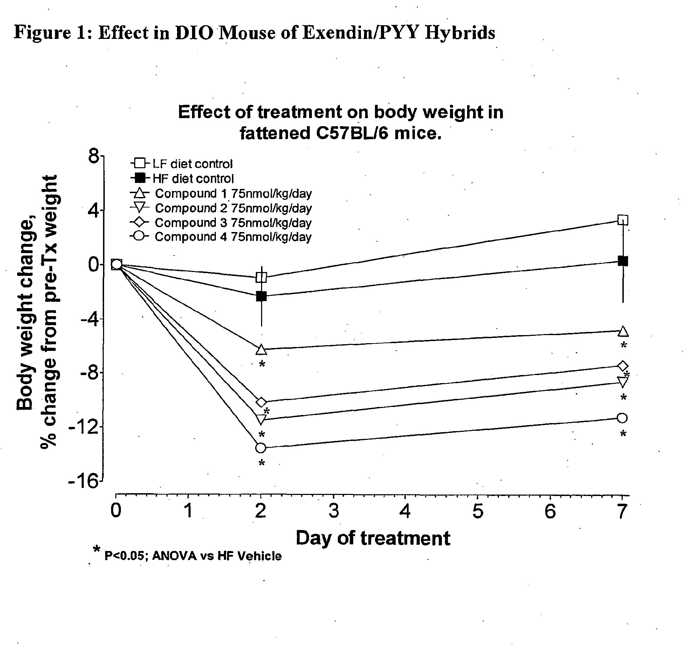

As shown in Table 4-1, certain exemplary compounds of the disclosure showed efficacy in the food intake assay. Certain peptides were also tested at 75 nmol/kg in the DIO assay and proved to be more efficacious than PYY (Figure 1).

### Exendin/Amylin Hybrids:

Exemplary hybrid polypeptides of the invention were prepared from C-terminally truncated exendin (1-27), C-terminally truncated amylin peptides (e.g., amylin(1-7), ^{2,7}Ala-Amylin(1-7), and Amylin(33-27)), and optional sCT fragments (e.g., sCT(8-10) and ¹⁴Gln,^{11,18}Arg-sCT(8-27)). Whereas both hybrid polypeptides were very active in appetite suppression (see Table 4-2), the onset of action differed from the activity profiles of the parent molecules (data not shown).

**Stable 4-2: Exendin/Amylin Hybrids and Their Effect in the FI Assay**

| | *Mouse Food Intake % basal* | | | |
|---|---|---|---|---|
| *Description* | *30min* | *60min* | *120min* | *Dose* |
| Exendin-4(1-27)-Amylin(1-7)-¹⁴Gln,^{11,18} Arg-sCT(8-27)-Amylin(33-37) | -24 | -40 | -48 | 25 nmol/Kg |
| Exendin-4(1-27)-^{2,7}Ala-Amylin(1-7)-sCT(8-10) | -40 | -59 | -66 | 25 nmol/Kg |

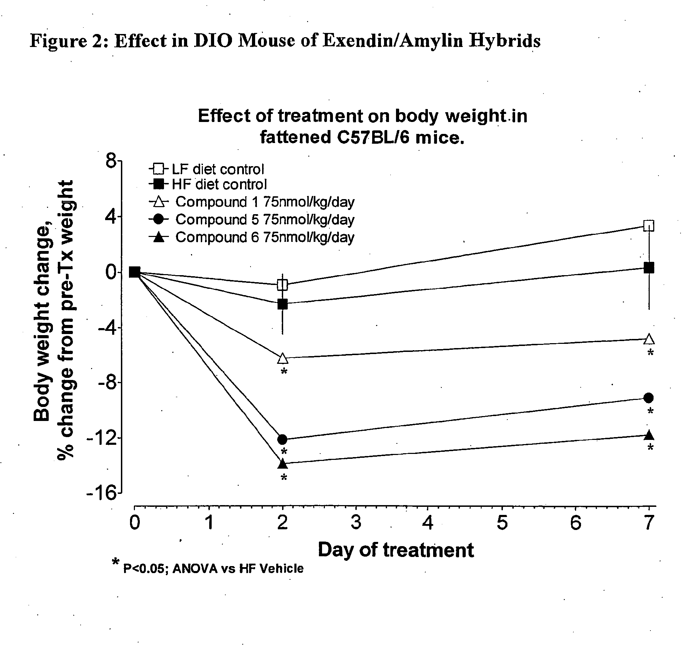

Both compounds also showed excellent efficacy when screened in the DIO assay (Figure 2).

### Exendin/CCK-8 Hybrids:

Exemplary hybrid polypeptides of the disclosure were prepared from full length or C-terminally truncated exendin-4 attached to the N-terminus of CCK-8 either directly or *via* a linker, preserving the N-terminal amide of the CCK-8. (Table 4-3). Further, certain hybrids were prepared incorporating the naturally occurring Tyr(SO₃), while another hybrid incorporating the more stable Phe(CH₂SO₃) group was prepared. All the prepared hybrid polypeptides were active in inhibiting food intake (Table 4-3).

**Table 4-3: Exendin/CCK-8 Hybrids and Their Effect in the Food Intake Assay**

| | *Mouse Food Intake % basal* | | | |
|---|---|---|---|---|
| *Description* | *30min* | *60min* | *120min* | *Dose* |
| Exendin-4-CCK-8 | -12 | -28 | -28 | 10 nmol/ Kg |
| Exendin-4(1-28)-CCK-8 | -20 | -36 | -45 | 10 nmol/ Kg |
| Exendin-4(1-28)-CCK-8 [Phe(CH₂SO₃)] | -24 | -47 | -66 | 10 nmol/ Kg |
| Exendin-4(1-28)- [8-amino- 3,6- dioxaoctanoyl]-CCK-8 | -12 | -28 | -40 | 10 nmol/Kg |

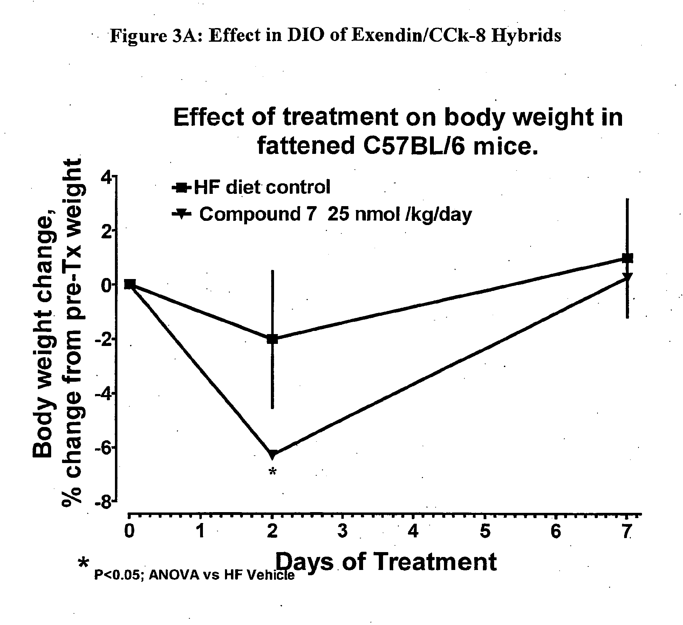

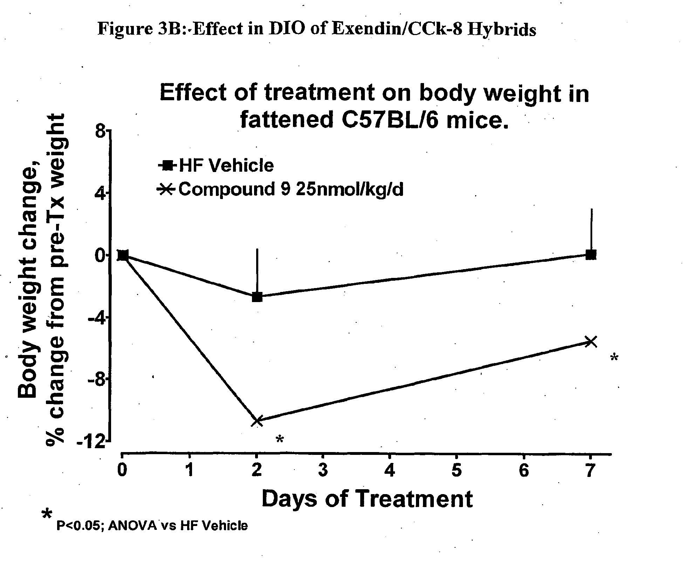

Exemplary exendin/CCK-8 hybrid polypeptides were tested in the DIO assay at 25 nmol/kg (Figures 3A and 3B). The data shows an initial weight loss, followed by a rebound effect in all compounds. Interestingly, the rebound effect appears to be diminished in hybrids incorporating the more hydrolytically stable Phe(CH₂SO₃) residue, as well as hybrids incorporating the linker 8-amino-3,6-dioxaoctanoyl between the exendin and the CCK residues.

### Amylin/PYY Hybrid:

An Amylin/PYY hybrid polypeptide was synthesized that contained truncated segments of each peptide. *In-vivo* activity in the food intake assay is shown in Table 4-4.

**Table 4-4: Amylin/PYY Phybrid**

| | Mouse Food Intake % Basal | | | |
|---|---|---|---|---|
| Description | 30min | 60min | 120min | Dose |
| Amylin(1-18)-PYY(19-36) | -13 | -14 | -13 | 25 nmol/Kg |

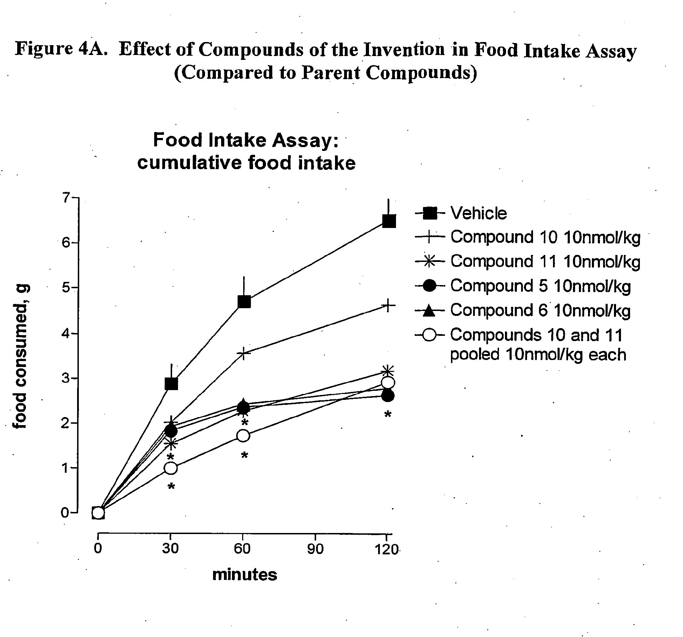

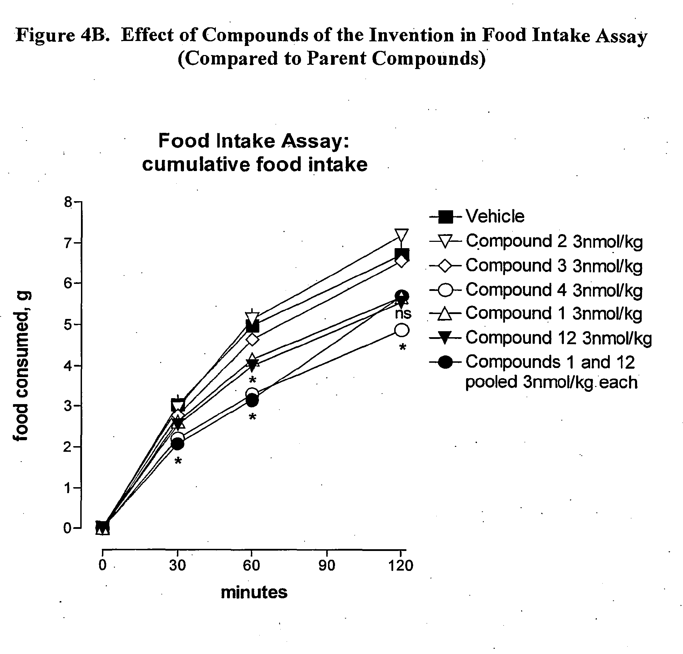

To ascertain if exemplary hybrid polypeptides of the disclosure are more potent than their parent component peptide hormones, exemplary compounds were tested in the food intake assay at the minimum efficacious dose of the more active parent molecule. The results are shown below in Figures 4A and 4B, which also compares the effects of pooled parent peptides (Compounds 1, 11, and 12 are component peptide hormones, analogs or fragments thereof). The data indicate that several peptides are at least as equipotent as the pooled parent peptides. In parallel with the *in vivo* studies, *in vitro* receptor binding and functional assays (cyclase activity) have been performed for all the compounds (data not shown).

While the present invention has been described in terms of preferred examples and embodiments, it is understood that variations and modifications will occur to those skilled in the art. Therefore, it is intended that the appended claims cover all such equivalent variations which come within the scope of the invention as claimed.

## Claims

1. A hybrid polypeptide exhibiting at least two hormonal-activities, said hybrid polypeptide comprising a first bio-active peptide hormone module covalently linked to at least one additional bio-active peptide hormone module; wherein:
the first bio-active peptide hormone is selected from exendin-4, an agonist having one or more insertions, deletions, or substitutions to the exendin-4, a pegylated or fatty acylated derivative of exendin-4 or the agonist, or fragment thereof, wherein the first bio-active peptide hormone is an agonist of the GLP-1 receptor and exhibits the insulin secretion activity of exendin-4; and
the at least one additional bio-active peptide hormone is selected from amylin, an agonist having one or more insertions, deletions, or substitutions to the amylin, a pegylated or fatty acylated derivative of amylin or the agonist, or fragment thereof, wherein the at least one additional bio-active peptide hormone is an agonist of the calcitonin receptor (CTR) and exhibits the food intake reduction activity of amylin.

2. The hybrid polypeptide of claim 1 or 2, wherein the agonist has at least 50% sequence identity to the exendin-4 or amylin.

3. The hybrid polypeptide of claim 1, wherein the amylin agonist has at least 50 % sequence identity to amylin and the exendin-4 agonist has at least 70% sequence identity to exendin-4.

4. The hybrid polypeptide of claim 1, wherein the first bio-active peptide hormone module is the agonist of exendin-4 and the at least one additional bio-active peptide hormone module is the agonist of amylin.

5. The hybrid polypeptide of claim 1, wherein the first bio-active peptide hormone module is exendin-4 and the at least one additional bio-active peptide hormone module is amylin.

6. The hybrid polypeptide of claim 1, wherein the first bio-active peptide hormone module is the agonist of exendin-4 and the at least one additional bio-active peptide hormone module is amylin.

7. The hybrid polypeptide of claim 1, wherein the first bio-active peptide hormone module is exendin-4 and the at least one additional bio-active peptide hormone module is the analog of amylin.

8. The hybrid polypeptide of claim 1, wherein the first bio-active peptide hormone module that exhibits the activity is located at the N-terminal portion of the hybrid polypeptide.

9. The hybrid polypeptide of claim 1, wherein the C-terminal end of the at least one additional peptide hormone module is amidated.

10. The hybrid polypeptide of claim 1, wherein the C-terminal end of the first bioactive peptide hormone module is directly attached to the N-terminal end of the at least one additional bio-active peptide hormone module to form the covalent attachment.

11. The hybrid polypeptide of claim 1, wherein the bio-active peptide hormone modules are covalently attached using one or more linking groups independently selected from the group consisting of: alkyls; dicarboxylic acids PEGS; amino acids; polyaminoacids; bifunctional linkers; aminocaproyl (Aca); β-alanyl; 8-amino-3,6-dioxaoctanoyl; and Gly-Lys-Arg (GKR).

12. The hybrid polypeptide of claim 1, 2 or 3, wherein the at least one additional bio-active peptide hormone module is independently selected from the group consisting of: human amylin, rat amylin, ^{25,28,29}Pro-h-amylin (pramlintide), des-¹Lys-h-amylin, ²⁵Pro,²⁶Val,^{28,29}Pro-h-amylin ¹⁸Arg,^{25,28}Pro-h-amyllin des-¹Lys,¹⁸Arg,^{25,28}Pro-h-amylin ¹⁸Arg,^{25,28,29}Pro-h-amylin, des-¹Lys,¹⁸Arg,^{25,28,29}Pro-h-amylin, des-¹,Lys^{25,28,29}Pro-h-amylin, ²⁵Pro,²⁶Val,^{28,29}Pro-h-amylin, ²⁸Pro-h-amylin, 2,7-cyclo-[²Asp,⁷Lys]-h-amylin, ²⁻³⁷h-amylin, ¹Ala-h-amylin, ²Ala-h-amylin, ^{2,7}Ala-h-amylin, ¹Ser-h-amylin, ²⁹Pro-h-amylin, ^{25,28}Pro-h-amylin, des-¹Lys,^{25,28} Pro-h-amylin, ²³Leu,²⁵Pro,²⁶Val,^{28,29}Pro-h-amylin, ²³Leu²⁵Pro²⁶Val²⁸Pro-h-amylin, des-¹Lys,²³Leu, ²⁵Pro,²⁶Val,²⁸Pro-h-amylin, ¹⁸Arg,²³Leu,²⁵Pro,²⁶Val,²⁸Pro-h-amylin, ¹⁸Arg,²³Leu,^{25,28,29}Pro-h-amylin, ¹⁸Arg²³Leu,^{25,28}Pro-h-amylin, ¹⁷Ile, ²³Leu,^{25,28,29}Pro-h-amylin, ¹⁷Ile, ^{25,28,29}Pro-h-amylin, des-¹Lys,¹⁷Ile,²³Leu,^{25,28,29}Pro-h-amylin, ¹⁷Ile,¹⁸Arg,²³Leu-h-amylin, ¹⁷Ile,¹⁸Arg,²³Leu,²⁶Val,²⁹pro-h-amylin, ¹⁷Ile,¹⁸Arg,²³Leu,²⁵Pro,²⁶Val,^{28,29}Pro-h-amylin, ¹³Thr,²¹His,²³Leu,²⁶Ala,²⁸Leu,²⁹Pro,³¹Asp-h-amylin, ¹³Thr,²¹His,²³Leu,²⁶Ala,²⁹Pro,³¹Asp-h-amylin, des-¹Lys,¹³Thr,²¹His,²³Leu,²⁶Ala,²⁸Pro,³¹Asp-h-amylin, ¹³Thr,¹⁸Arg,²¹His,²³Leu,²⁶Ala,²⁹Pro,³¹Asp-h-amylin, ¹³Thr,¹⁸Arg,²¹His,²³Leu,^{28,29}Pro,³¹Asp-h-amylin, ¹³Thr,¹⁸Arg,²¹His,²³Leu,²⁵Pro,²⁶Ala,^{28,29}Pro,³¹Asp-h-amylin, hAmylin(1-7)-¹⁴Glu,11,18Arg-sCT(8-27)-Amylin(33-37), hAmylin(1-7)-^{11,18}Arg-sCT(8-27)-Amylin(33-37), ^{2,7}Ala-hAmylin(1-7)-sCT(8-10), hAmylin(1-7)-^{11,18 18}Arg-sCt(8-27)-hAmylin(33-37), ¹des-Lys-hAmylin(1-7)-^{11,18}Arg-sCt(8-27)-hAmylin(33-37), ¹⁵Glu-hAmylin(1-17)-¹⁸Arg-sCT(18-26)-Amylin(32-37), ¹des-Lys-hAmylin(1-7)-¹⁴Glu,^{11,18}Arg-sCt(8-27)-hAmylin(33-37), and h-amylin(1-7)-¹⁴Gln,^{11,18}Arg-sCT(8-27)-Amylin(33-37) and agonists having at least 90% sequence identity thereto that exhibit the food intake reduction activity of amylin.

13. The hybrid polypeptide of claim 1 or 2, wherein the first bio-active peptide hormone module is selected from the group consisting of: exendin-4, exendin-4(1-27), exendin-4(1-28), ¹⁴Leu,²⁵Phe-exendin-4(1-28); ⁵Ala,¹⁴Leu,²⁵Phe-exendin-4(1-28), exendin-3, exendin-4(1-29), exendin-4(1-30), ¹⁴Leu,²⁵Phe-exendin-4(1-27) and ¹⁴Leu-exendin-4(1-28) and agonist analogs having at least 90% sequence identity thereto that exhibit the insulin secretion activity of exendin-4.

14. The hybrid polypeptide of claim 1, wherein the first bio-active peptide hormone module is located at the N-terminal end of the hybrid polypeptide and the at least one additional bio-active peptide hormone module is located at the C-terminal end of the hybrid polypeptide.

15. The hybrid polypeptide of claim 1, wherein the agonist has at least 80% sequence identity to said exendin-4 or amylin.

16. The hybrid polypeptide of claim 15, wherein the agonist analog has at least 90% sequence identity to said exendin-4 or amylin.

17. A pharmaceutical composition comprising the hybrid polypeptide of any of claims 1 to 16.

18. The hybrid polypeptide of any one of claims 1 to 16 or pharmaceutically acceptable salt thereof for use as a medicament.

19. The hybrid polypeptide of any one of claims 1 to 16 for use in the treatment of diabetes or diabetic neuropathy.

20. The hybrid polypeptide for the use according to claim 19, wherein the diabetes is Type II diabetes or gestational diabetes.

## Patentansprüche

1. Hybridpolypeptid, das zumindest zwei Hormonaktivitäten zeigt, wobei das Hybridpolypeptid ein erstes bioaktives Peptidhormonmodul umfasst, das kovalent mit zumindest einem zusätzlichen bioaktiven Peptidhormonmodul verbunden ist; wobei:
das erste bioaktive Peptidhormon aus Exendin-4, einem Agonisten mit einer oder mehreren Einfügungen, Löschungen oder Ersetzungen an dem Exendin-4, einem pegylierten oder fettacyliertem Derivat von Exendin-4 oder dem Agonisten oder einem Fragment desselben ausgewählt ist, wobei das erste bioaktive Peptidhormon ein Agonist des GLP-1-Rezeptors ist und die Insulinsekretionsaktivität von Exendin-4 zeigt; und
das zumindest eine zusätzliche bioaktive Peptidhormon aus Amylin, einem Agonisten mit einer oder mehreren Einfügungen, Löschungen oder Ersetzungen an dem Amylin, einem pegylierten oder fettacylierten Derivat von Amylin oder dem Agonisten oder einem Fragment desselben ausgewählt ist, wobei das zumindest eine zusätzliche bioaktive Peptidhormon ein Agonist des Calcitoninrezeptors (CTR) ist und die Nahrungsaufnahmereduktionsaktivität von Amylin zeigt.

2. Hybridpolypeptid nach Anspruch 1 oder 2, wobei der Agonist eine zumindest 50%-ige Sequenzidentität zu dem Exendin-4 oder Amylin aufweist.

3. Hybridpolypeptid nach Anspruch 1, wobei der Amylinagonist eine zumindest 50 %-ige Sequenzidentität zu Amylin aufweist und der Exendin-4-Agonist eine zumindest 70%-ige Sequenzidentität zu Exendin-4 aufweist.

4. Hybridpolypeptid nach Anspruch 1, wobei das erste bioaktive Peptidhormonmodul der Agonist von Exendin-4 und das zumindest eine zusätzliche bioaktive Peptidhormonmodul der Agonist von Amylin ist.

5. Hybridpolypeptid nach Anspruch 1, wobei das erste bioaktive Peptidhormonmodul Exendin-4 und das zumindest eine zusätzliche bioaktive Peptidhormonmodul Amylin ist.

6. Hybridpolypeptid nach Anspruch 1, wobei das erste bioaktive Peptidhormonmodul der Agonist von Exendin-4 und das zumindest eine zusätzliche bioaktive Peptidhormonmodul Amylin ist.

7. Hybridpolypeptid nach Anspruch 1, wobei das erste bioaktive Peptidhormonmodul Exendin-4 und das zumindest eine zusätzliche bioaktive Peptidhormonmodul das Analogon von Amylin ist.

8. Hybridpolypeptid nach Anspruch 1, wobei das erste bioaktive Peptidhormonmodul, das die Aktivität zeigt, am N-terminalen Teil des Hybridpolypeptids positioniert ist.

9. Hybridpolypeptid nach Anspruch 1, wobei das C-terminale Ende des zumindest einen zusätzlichen Peptidhormonmoduls amidiert ist.

10. Hybridpolypeptid nach Anspruch 1, wobei das C-terminale Ende des ersten bioaktiven Peptidhormonmoduls direkt an dem N-terminalen Ende des zumindest einen zusätzlichen bioaktiven Peptidhormonmoduls angelagert ist, um die kovalente Anlagerung zu bilden.

11. Hybridpolypeptid nach Anspruch 1, wobei die bioaktiven Peptidhormonmodule kovalent unter Verwendung von einer oder mehreren Verbindungsgruppen angelagert sind, die unabhängig aus der Gruppe ausgewählt sind, die aus: Alkylen; Dicarboxylsäuren-PEGS; Aminosäuren; Polyaminosäuren; bifunktionalen Linkern; Aminocaproyl (Aca); β-Alanyl; 8-Amino-3,6-dioxaoctanoyl; und Gly-Lys-Arg (GKR) besteht.

12. Hybridpolypeptid nach Anspruch 1, 2 oder 3, wobei das zumindest eine zusätzliche bioaktive Peptidhormonmodul unabhängig aus der Gruppe ausgewählt ist, die aus: menschlichem Amylin, Rattenamylin, ^{25,28,29}Pro-h-Amylin (Pramlintid), des-¹Lys-h-Amylin, ²⁵Pro,²⁶Val, ^{28,29}Pro-h-Amylin ¹⁸Arg ^{25,28}Pro-h-Amylin des-¹Lys,¹⁸Arg ^{25,28}Pro-h-Amylin ¹⁸Arg, ^{25,28,29}Pro-h-Amylin, des-¹ Lys, ¹⁸Arg, ^{25,28,29}Pro-h-Amylin, des-¹ Lys^{25,28,29}Pro-h-Amylin, ²⁵Pro,²⁶Val,^{28,29}Pro-h-Amylin,²⁸Pro-h-Amylin, 2,7-Cyclo-[²Asp,⁷Lys]-h-Amylin, ²⁻³⁷h-Amylin, ¹Ala-h-Amylin, ²Ala-h-Amylin, ^{2,7}Ala-h-Amylin, ¹Ser-h-Amylin, ²⁹Pro-h-Amylin, ^{11,28}Pro-h-Amylin, des-¹Lys,^{25,28}Pro-h-Amylin ²³Leu,²⁵Pro,²⁶Val,^{28,29}Pro-h-Amylin, ²³Leu²⁵Pro²⁶Val²⁸Pro-h-Amylin, des-¹Lys,²³Leu,²⁵Pro,²⁶Val,²⁸Pro-h-Amylin, ¹⁸Arg, ²³Leu,²⁵Pro, ²⁶Val,²⁸Pro-h-Amylin, ¹⁸Arg²³Leu^{25,28,29}Pro-h-Amylin ¹⁸Arg²³Leu^{25,28}Pro-h-Amylin ¹⁷Ile, ²³Leu,^{25,28,29}Pro-h-Amylin, ¹⁷Ile,^{25,28,29}Pro-h-Amylin des-¹Lys, ¹⁷Ile,²³Leu,^{25,28,29}Pro-h-Amylin, ¹⁷Ile,¹⁸Arg, ²³Leu-h-Amylin, ¹⁷Ile,¹⁸Arg,²³Leu,²⁶Val,²⁹Pro-h-Amylin, ¹⁷Ile,¹⁸Arg,²³Leu,²⁵Pro,²⁶Val,^{28,29}Pro-h-Amylin, ¹³Thr,²¹His, ²³Leu, ²⁶Ala, ²⁸Leu,²⁹Pro,³¹Asp-h-Amylin, ¹³Thr,²¹His,²³Leu,²⁶Ala,²⁹Pro,³¹Asp-h-Amylin,des-¹Lys,¹³Thr,²¹His,²³Leu,²⁶Ala,²⁸Pro,³¹Asp-h-Amylin, ¹³Thr,¹⁸Arg,²¹His,²³Leu,²⁶Ala,²⁹Pro,³¹Asp-h-Amylin, ¹³Thr,¹⁸Arg,²¹His,²³Leu,^{28,29}Pro,³¹Asp-h-Amylin, hAmylin(1-7)-14Glu, 11, 18Arg-sCT(8-27)-Amylin(33-37), hAmylin(1-7)-^{11,18}Arg-sCT(8-27)-Amylin(33-37), ^{2,7}Ala-hAmylin(1-7)-sCT(8-10), hAmylin(1-7)-^{11,18}Arg-sCt(8-27)-hAmylin(33-37), ¹des-Lys-hAmylin(1-7)-^{11,18}Arg-sCt(8-27)-hAmylin(33-37), ¹⁵Glu-hAmylin(1-17)- ¹⁸Arg-sCT(18-26)-Amylin(32-37), ¹des-Lys-hAmylin(1-7)-¹⁴Glu,^{11,18}Arg-sCt(8-27)-hAmylin(33-37) und h-Amylin(1-7)-¹⁴Gln,^{11,18}Arg-sCT(8-27)-Amylin(33-37) und Agonisten besteht, die eine zumindest 90%-ige Sequenzidentität mit denselben aufweisen, die die Nahrungsaufnahmereduktionsaktivität von Amylin zeigen.

13. Hybridpolypeptid nach Anspruch 1 oder 2, wobei das erste bioaktive Peptidhormonmodul aus der Gruppe ausgewählt ist, die aus: Exendin-4, Exendin-4(1-27), Exendin-4(1-28), ¹⁴Leu, ²⁵Phe-Exendin-4(1-28); ⁵Ala,¹⁴Leu,²⁵Phe-Exendin-4(1-28), Exendin-3, Exendin-4(1-29), Exendin-4(1-30), ¹⁴Leu,²⁵Phe-Exendin-4(1-27) und ¹⁴Leu-Exendin-4(1-28) und Agonistanaloga besteht, die eine zumindest 90%-ige Sequenzidentität mit denselben aufweisen, die die Insulinsekretionsaktivät von Exendin-4 zeigen.

14. Hybridpolypeptid nach Anspruch 1, wobei das erste bioaktive Peptidhormonmodul am N-terminalen Ende des Hybridpolypeptids positioniert und das zumindest eine zusätzliche bioaktive Peptidhormonmodul am C-terminalen Ende des Hybridpolypeptids positioniert ist.

15. Hybridpolypeptid nach Anspruch 1, wobei der Agonist eine zumindest 80%-ige Sequenzidentität mit dem Exendin-4 oder Amylin aufweist.

16. Hybridpolypeptid nach Anspruch 15, wobei das Agonistanalogon eine zumindest 90%-ige Sequenzidentität mit dem Exendin-4 oder Amylin aufweist.

17. Pharmazeutische Zusammensetzung mit dem Hybridpolypeptid nach irgendeinem der Ansprüche 1 bis 16.

18. Hybridpolypeptid nach irgendeinem der Ansprüche 1 bis 16 oder pharmazeutisch annehmbares Salz desselben zur Verwendung als Arzneimittel.

19. Hybridpolypeptid nach irgendeinem der Ansprüche 1 bis 16 zur Verwendung bei der Behandlung von Diabetes oder diabetischer Neuropathie.

20. Hybridpolypeptid zur Verwendung nach Anspruch 19, wobei der Diabetes Typ-II-Diabetes oder Schwangerschaftsdiabetes ist.

## Revendications

1. Un polypeptide hybride présentant au moins deux activités hormonales, le polypeptide hybride comprenant un premier module d'hormones peptidiques bioactives lié par covalence à au moins un module d'hormones peptidiques bioactives supplémentaire ; dans lequel :
la première hormone peptidique bioactive est sélectionnée parmi l'exendine-4, un agoniste ayant une ou plusieurs insertions, suppressions ou substitutions de l'exendine-4, un dérivé pégylé ou acylé gras de l'exendine-4 ou de l'agoniste, ou un fragment de ce dernier, la première hormone peptidique bioactive étant un agoniste du récepteur GLP-1 et présentant l'activité de sécrétion d'insuline de l'exendine-4 ; et
la ou les hormones peptidiques bioactives supplémentaires sont sélectionnées parmi l'amyline, un agoniste ayant une ou plusieurs insertions, suppressions ou substitutions de l'amyline, un dérivé pégylé ou acylé gras de l'amyline ou de l'agoniste, ou un fragment de ce dernier, la ou les hormones peptidiques bioactives supplémentaires étant un agoniste du récepteur de la calcitonine (CTR) et présentant l'activité de réduction de l'apport alimentaire de l'amyline.

2. Le polypeptide hybride des revendications 1 ou 2, dans lequel l'agoniste possède une identité de séquence d'au moins 50 % avec l'exendine-4 ou l'amyline.

3. Le polypeptide hybride de la revendication 1, dans lequel l'agoniste de l'amyline possède une identité de séquence d'au moins 50 % avec l'amyline et l'agoniste de l'exendine-4 possède une identité de séquence d'au moins 70 % avec l'exendine-4.

4. Le polypeptide hybride de la revendication 1, dans lequel le premier module d'hormones peptidiques bioactives est l'agoniste de l'exendine-4 et le ou les modules d'hormones peptidiques bioactives supplémentaires sont l'agoniste de l'amyline.

5. Le polypeptide hybride de la revendication 1, dans lequel le premier module d'hormones peptidiques bioactives est l'exendine-4 et le ou les modules d'hormones peptidiques bioactives supplémentaires sont l'amyline.

6. Le polypeptide hybride de la revendication 1, dans lequel le premier module d'hormones peptidiques bioactives est l'agoniste de l'exendine-4 et le ou les modules d'hormones peptidiques bioactives supplémentaires sont l'amyline.

7. Le polypeptide hybride de la revendication 1, dans lequel le premier module d'hormones peptidiques bioactives est l'exendine-4 et le ou les modules d'hormones peptidiques bioactives supplémentaires sont l'analogue de l'amyline.

8. Le polypeptide hybride de la revendication 1, dans lequel le premier module d'hormones peptidiques bioactives qui présente l'activité se trouve dans la partie de terminaison N du polypeptide hybride.

9. Le polypeptide hybride de la revendication 1, dans lequel le côté de terminaison C du ou des modules d'hormones peptidiques bioactives supplémentaires est amidaté.

10. Le polypeptide hybride de la revendication 1, dans lequel le côté de terminaison C du premier module d'hormones peptidiques bioactives est directement fixé au côté de terminaison N du ou des modules d'hormones peptidiques bioactives supplémentaires pour former la liaison par covalence.

11. Le polypeptide hybride de la revendication 1, dans lequel les modules d'hormones peptidiques bioactives sont liés par covalence à l'aide d'un ou plusieurs groupes de liaison indépendamment sélectionnés parmi ce qui suit : alkyles ; PEGS d'acides dicarboxyliques ; acides aminés ; polyaminoacides ; lieurs bifonctionnels ; aminocaproyl (Aca) ; β-alanyle ; 8-amino-3,6-dioxaoctanoyl ; et Gly-Lys-Arg (GKR).

12. Le polypeptide hybride des revendications 1, 2 ou 3, dans lequel le ou les modules d'hormones peptidiques bioactives supplémentaires sont indépendamment sélectionnés parmi ce qui suit : amyline humaine, amyline de rat, ^{25,28,29}Pro-h-amyline (pramlintide), des-¹Lys-h-amyline, ²⁵Pro, ²⁶Val, ^{28,29}Pro-h-amyline, ¹⁸Arg, ^{25,28}Pro-h-amyline, des-¹Lys-¹⁸Arg, ^{25,28}Pro-h-amyline, ¹⁸Arg ^{25,28,29}Pro-h-amyline, des-¹Lys, ¹⁸Arg, ^{25,28,29}Pro-h-amyline, des-¹ Lys^{25,28,29}Pro-h-amyline, ²⁵Pro, ²⁶Val, ^{28,29}Pro-h-amyline, ²⁸Pro-h-amyline, 2,7-cyclo-[²Asp,⁷Lys]-h-amyline, ²⁻³⁷h-amyline, ¹Ala-h-amyline, ²Ala-h-amyline, ^{2,7}Ala-h-amyline, ¹Ser-h-amyline, ²⁹Pro-h-amyline, ^{25,28}Pro-h-amyline, des-¹Lys, ^{25,28}Pro-h-amyline, ²³Leu, ²⁵Pro, ²⁶Val, ^{28,29}Pro-h-amyline, ²³Leu²⁵Pro²⁶Val²⁸Pro-h-amyline, des-¹Lys, ²³Leu, ²⁵Pro, ²⁶Val, ²⁸Pro-h-amyline,¹⁸Arg, ²³Leu, ²⁵Pro, ²⁶Val, ²⁸Pro-h-amyline, ¹⁸Arg, ²³Leu, ^{25,28,29}Pro-h-amyline, ¹⁸Arg²³Leu, ^{25,28}Pro-h-amyline, ¹⁷Ile, ²³Leu, ^{25,28,29}Pro-h-amyline, ¹⁷Ile, ^{25,28,29}Pro₋h-amyline, des-¹Lys, ¹⁷Ile, ²³Leu, ^{25,28,29}Pro-h-amyline, ¹⁷Ile, ¹⁸Arg, ²³Leu-h-amyline, ¹⁷Ile, ¹⁸Arg, ²³Leu, ²⁶Val, ²⁹Pro-h-amyline, ¹⁷Ile, ¹⁸Arg, ²³Leu, ²⁵Pro, ²⁶Val, ^{28,29}Pro-h-amyline ¹³Thr ²¹His ²³Leu, ²⁶Ala, ²⁸Leu, ²⁹Pro, ³¹Asp-h-amyline ¹³Thr, ²¹His ²³Leu, ²⁶Ala, ²⁹Pro, ³¹Asp-h-amyline, des-¹Lys, ¹³Thr, ²¹His, ²³Leu, ²⁶Ala, ²⁸Pro, ³¹Asp-h-amyline, ¹³Thr, ¹⁸Arg, ²¹His, ²³Leu, ²⁶Ala, ²⁹Pro, ³¹Asp-h-amyline, ¹³Thr, ¹⁸Arg, ²¹His, ²³Leu, ^{28,29}Pro, ³¹Asp-h-amyline, hAmyline(1-7)-14Glu, 11, 18Arg-sCT(8-27)-Amyline(33-37), hAmyline(I-7)-^{11,18}Arg-sCT(8-27)-Amyline(33-37), ^{2,7}Ala-hAmyline(I-7)-sCT(8-10), hAmyline(I-7)-^{11,18}Arg-sCt(8-27)-hAmyline(33-37), ¹des-Lys-hAmyline(I-7)-^{11,18}Arg-sCt(8-27)-hAmyline(33-37), ¹⁵Glu-hAmyline(1-17)-¹⁸Arg-sCT(f18-26)-Amyline(32-37), ¹des-Lys-hAmyline(1-7)-¹⁴Glu,^{11,18}Arg-sCt(8-27)-hAmyline(33-37) et h-amyline(I-7)-¹⁴Gln,^{11,18}Arg-sCT(8-27)-Amyline(33-37) et des agonistes ayant au moins 90 % d'identité de séquence avec ceux qui présentent l'activité de réduction de l'apport alimentaire de l'amyline.

13. Le polypeptide hybride des revendications 1 ou 2, dans lequel le premier module d'hormones peptidiques bioactives est sélectionné parmi ce qui suit : exendine-4, exendine-4(I-27), exendine-4(I-28), ¹⁴Leu, ²⁵Phe-exendine-4(I-28); ⁵Ala, ¹⁴Leu, ²⁵Phe-exendine-4(I-28), exendine-3, exendine-4(I-29), exendine-4(1-30), ¹⁴Leu, ²⁵Phe-exendine-4(I-27) et ¹⁴Leu-exendine-4(I-28) et les analogues agonistes possèdent une identité de séquence d'au moins 90 % avec ceux qui présentent l'activité de sécrétion d'insuline de l'exendine-4.

14. Le polypeptide hybride de la revendication 1, dans lequel le premier module d'hormones peptidiques bioactives se trouve du côté de terminaison N du polypeptide hybride et le ou les modules d'hormones peptidiques bioactives supplémentaires se trouvent du côté de terminaison C du polypeptide hybride.

15. Le polypeptide hybride de la revendication 1, dans lequel l'agoniste possède une identité de séquence d'au moins 80 % avec l'exendine-4 ou l'amyline.

16. Le polypeptide hybride de la revendication 15, dans lequel l'analogue agoniste possède une identité de séquence d'au moins 90 % avec l'exendine-4 ou l'amyline.

17. Une composition pharmaceutique comprenant le polypeptide hybride de n'importe laquelle des revendications 1 à 16.

18. Le polypeptide hybride de n'importe laquelle des revendications 1 à 16 ou un sel pharmaceutiquement acceptable en provenant destiné à être utilisé comme médicament.

19. Le polypeptide hybride de n'importe laquelle des revendications 1 à 16, destiné au traitement du diabète ou de la neuropathie diabétique.

20. Le polypeptide hybride destiné à l'utilisation prévue à la revendication 19, dans lequel le diabète est un diabète du Type Il ou un diabète gestationnel.
